(19)

Europäisches
Patentamt
European
Patent Office
Office européen
des brevets

(11) **EP 4 357 361 A1**

(12) **EUROPEAN PATENT APPLICATION**
published in accordance with Art. 153(4) EPC

(43) Date of publication:
**24.04.2024 Bulletin 2024/17**

(21) Application number: **22824301.0**

(22) Date of filing: **17.06.2022**

(51) International Patent Classification (IPC):
**C07K 16/24** (2006.01)    **C07K 16/28** (2006.01)
**G01N 33/564** (2006.01)    **A61P 17/06** (2006.01)
**A61P 37/06** (2006.01)

(52) Cooperative Patent Classification (CPC):
**A61P 17/06; A61P 37/06; C07K 16/24;
C07K 16/28; G01N 33/564**

(86) International application number:
**PCT/CN2022/099294**

(87) International publication number:
**WO 2022/262828 (22.12.2022 Gazette 2022/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.06.2021   CN 202110675925**

(71) Applicant: CHIA TAI TIANQING
**PHARMACEUTICAL GROUP CO., LTD.
Lianyungang,
Jiangsu 222062 (CN)**

(72) Inventors:
• **LONG, Xi
Nanjing, Jiangsu 211100 (CN)**

• **XU, Hongjiang
Nanjing, Jiangsu 211100 (CN)**
• **SHI, Wei
Nanjing, Jiangsu 211100 (CN)**
• **WANG, Liangliang
Nanjing, Jiangsu 211100 (CN)**
• **LU, Zhenzhen
Nanjing, Jiangsu 211100 (CN)**

(74) Representative: **Grünecker Patent- und
Rechtsanwälte
PartG mbB
Leopoldstraße 4
80802 München (DE)**

Remarks:
The complete document including Reference
Table(s) and the Sequence Listing(s) can be
downloaded from the EPO website

(54) **ANTI-IL-36R ANTIBODY AND USE THEREOF**

(57)    Provided are an anti-IL-36R antibody and a use thereof. In particular, a mouse, chimeric or humanized antibody or an antigen-binding fragment thereof binding to IL-36R is provided; further provided are a nucleic acid molecule encoding the antibody or the antigen-binding fragment thereof, as well as an expression vector and host cell used to express the antibody or the antigen-binding fragment thereof. In addition, a preparation method and a use of the antibody or the antigen-binding fragment thereof are provided, comprising treatment and prevention of IL-36/IL-36R-mediated diseases and disorders.

| | BI 655130 | xi72C1 | xi84C4 |
|---|---|---|---|
| EC50 | 7.611e-010 | 2.624e-009 | 9.698e-010 |

FIG. 1

EP 4 357 361 A1

**Description**

**CROSS-REFERENCE TO RELATED APPLICATIONS**

The present disclosure claims priority to the Chinese Patent Application No. 202110675925.6 filed on June 18, 2021, the content of which is incorporated herein by reference in its entirety and for all purposes.

**TECHNICAL FIELD**

The present disclosure relates to an isolated antibody. In particular, the present disclosure relates to an isolated antibody specifically binding to IL-36R or an antigen-binding fragment thereof, and methods for preparing and using the antibody or the antigen-binding fragment thereof.

**BACKGROUND**

Interleukin 36 (IL-36) belongs to a member of the interleukin 1 (IL-1) family, including 3 agonistic cytokines IL-36α (IL-1F6), IL-36β (IL-1F8) and IL-36y (IL-1F9) and 1 inhibitory cytokine IL-36Ra. IL-36 receptor (IL-36R) is a co-receptor for members of the IL-36 family, also known as interleukin-1 receptor-like 2 (IL1RL2) or IL-1 receptor-associated protein 2 (IL-1Rrp2). IL-36α, IL-36P and IL-36y are involved in effector cell activation, promotion of inflammatory cytokine expression and inflammation occurrence by binding to IL-36R and IL-1 receptor auxiliary protein (IL-1RAcP), activating mitogen-activated protein kinase (MAPKs) and nuclear factor kappa B (NF-kB) signaling pathways.

IL-36α, IL-36P and IL-36y are mainly distributed in tissues such as skin, lung, joint, and intestinal tract, and can be generated by a plurality of cells such as monocytes, macrophages, T lymphocytes, B lymphocytes, and keratinocytes; IL-36R is mainly distributed in tissues such as lungs, and epididymides. Researchers discovered IL-36 for the first time in generalized pustular psoriasis (GPP) and plaque psoriasis, and observed over-expression of IL-36Rα and IL-36y mRNAs in GPP and plaque psoriasis lesions. Multiple studies have shown a certain correlation between IL-36 and psoriasis. For example, it was found in psoriasis that IL-36y inhibits differentiation of keratinocytes and induces inflammatory responses of keratinocytes through Wnt signaling pathway; IL-36y also can promote production of pro-inflammatory factors such as IFN-γ, IL-1β and IL-6 (Wang et al. (2017), Int J Med Sci, 14: 1002-1007); IL-36R-deficient mice do not develop imiquimod-induced psoriasis-like dermatitis (Tortola et al. (2012), J Clin Invest, 122: 3965-3976). Another research group has demonstrated that the expression levels of IL-36α and IL-36y is up-regulated in the intestinal mucosa of IBD patients and can induce expression of chemokines by intestinal epithelial cells, initiate downstream signaling pathways, and induce inflammatory responses (Nishida et al. (2016), Inflamm Bowel Dis, 22: 303-314).

Therefore, blocking binding of IL-36 to IL-36R and inhibiting IL-36/IL-36R signaling pathway plays an important role in treating various autoimmune diseases and inflammatory diseases. Spesolimab (BI655130) is an anti-IL-36R monoclonal antibody developed by Boehringer Ingelheim for use in treating generalized pustular psoriasis (GPP), palm plant pustulosis (PPP), Crohn's disease (CD), and atopic dermatitis (AD), and it has been demonstrated that it can significantly improve symptoms of GPP patients in clinical phase I (Bachelez Hervé et al. (2019), NEJM, 380: 981-983). However, there is also a need in the art for more antibodies with better medicinal properties, e.g., an antibody that binds to IL-36R with high affinity and can effectively neutralize IL-36R activity.

**BRIEF SUMMARY**

The present disclosure provides an isolated antibody, e.g., a mouse, chimeric, humanized or human monoclonal antibody or an antigen-binding fragment thereof that binds to IL-36R (e.g., human and monkey IL-36R). In particular, the antibody or the antigen-binding fragment thereof of the present disclosure is capable of binding to IL-36R (e.g., human and monkey IL-36R) with high affinity and blocking the IL-36/IL-36R interaction.

The antibody or the antigen-binding fragment thereof of the present disclosure has a variety of uses, including detecting IL-36R protein, and treating or preventing relevant IL-36/IL-36R-mediated diseases and disorders, e.g., autoimmune diseases, inflammatory diseases, respiratory diseases, metabolic disorders, cancer, or the like.

In one aspect, the present disclosure provides an isolated anti-IL-36R antibody or an antigen-binding fragment thereof, comprising:

i) heavy chain CDR1, heavy chain CDR2 and heavy chain CDR3, wherein

(1) heavy chain CDR1 comprises an amino acid sequence set forth in SEQ ID NO: 1 or 85 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 1 or 85, heavy chain CDR2 comprises an amino acid sequence set forth in SEQ ID NO: 2 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 2, and heavy chain CDR3 comprises an amino acid sequence set forth in SEQ ID NO: 3 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 3,

(2) heavy chain CDR1 comprises an amino acid sequence set forth in SEQ ID NO: 21 or 86 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 21 or 86, heavy chain CDR2 comprises an amino acid sequence set forth in SEQ ID NO: 22 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 22, and heavy chain CDR3 comprises an amino acid sequence set forth in SEQ ID NO: 23 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 23,

(3) heavy chain CDR1 comprises an amino acid sequence set forth in SEQ ID NO: 45 or 88 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 45 or 88, heavy chain CDR2 comprises an amino acid sequence set forth in SEQ ID NO: 46 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 46, and heavy chain CDR3 comprises an amino acid sequence set forth in SEQ ID NO: 47 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 47,

(4) heavy chain CDR1 comprises an amino acid sequence set forth in SEQ ID NO: 53 or 89 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 53 or 89, heavy chain CDR2 comprises an amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 54, and heavy chain CDR3 comprises an amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 55, or

(5) heavy chain CDR1 comprises an amino acid sequence set forth in SEQ ID NO: 61 or 90 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 61 or 90, heavy chain CDR2 comprises an amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 62, and heavy chain CDR3 comprises an amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 63; and/or

ii) light chain CDR1, light chain CDR2 and light chain CDR3, wherein

(1) light chain CDR1 comprises an amino acid sequence set forth in SEQ ID NO: 4 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 4, light chain CDR2 comprises an amino acid sequence set forth in SEQ ID NO: 5 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 5, and light chain CDR3 comprises an amino acid sequence set forth in SEQ ID NO: 6 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 6,

(2) light chain CDR1 comprises an amino acid sequence set forth in SEQ ID NO: 24 or 87 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 24 or 87, light chain CDR2 comprises an amino acid sequence set forth in SEQ ID NO: 25 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 25, and light chain CDR3 comprises an amino acid sequence set forth in SEQ ID NO: 26 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 26,

(3) light chain CDR1 comprises an amino acid sequence set forth in SEQ ID NO: 48 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 48, light chain CDR2 comprises an amino acid sequence set forth in SEQ ID NO: 49 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 49, and light chain CDR3 comprises an amino acid sequence set forth in SEQ ID NO: 50 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 50,

(4) light chain CDR1 comprises an amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 56, light chain CDR2 comprises an amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 57, and light chain CDR3 comprises an amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 58, or

(5) light chain CDR1 comprises an amino acid sequence set forth in SEQ ID NO: 64 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 64, light chain CDR2 comprises an amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 65, and light chain CDR3 comprises an amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 66.

In some embodiments, the anti-IL-36R antibody or the antigen-binding fragment thereof comprises heavy chain CDR1, heavy chain CDR2, heavy chain CDR3, light chain CDR1, light chain CDR2 and light chain CDR3, wherein

(1) heavy chain CDR1 comprises an amino acid sequence set forth in SEQ ID NO: 1 or 85 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 1 or 85, heavy chain CDR2 comprises an amino acid sequence set forth in SEQ ID NO: 2 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 2, heavy chain CDR3 comprises an amino acid sequence set forth in SEQ ID NO: 3 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 3, light chain CDR1 comprises an amino acid sequence set forth in SEQ ID NO: 4 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 4, light chain CDR2 comprises an amino acid sequence set forth in SEQ ID NO: 5 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 5, and light chain CDR3 comprises an amino acid sequence set forth in SEQ ID NO: 6 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 6,

(2) heavy chain CDR1 comprises an amino acid sequence set forth in SEQ ID NO: 21 or 86 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 21 or 86, heavy chain CDR2 comprises an amino acid sequence set forth in SEQ ID NO: 22 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 22, heavy chain CDR3 comprises an amino acid sequence set forth in SEQ ID NO: 23 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 23, light chain CDR1 comprises an amino acid sequence set forth in SEQ ID NO: 24 or 87 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 24 or 87, light chain CDR2 comprises an amino acid sequence set forth in SEQ ID NO: 25 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 25, and light chain CDR3 comprises an amino acid sequence set forth in SEQ ID NO: 26 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 26,

(3) heavy chain CDR1 comprises an amino acid sequence set forth in SEQ ID NO: 45 or 88 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 45 or 88, heavy chain CDR2 comprises an amino acid sequence set forth in SEQ ID NO: 46 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 46, heavy chain CDR3 comprises an amino acid sequence set forth in SEQ ID NO: 47 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 47, light chain CDR1 comprises an amino acid sequence set forth in SEQ ID NO: 48 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 48, light chain CDR2 comprises an amino acid sequence set forth in SEQ ID NO: 49 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 49, and light chain CDR3 comprises an amino acid sequence set forth in SEQ ID NO: 50 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 50,

(4) heavy chain CDR1 comprises an amino acid sequence set forth in SEQ ID NO: 53 or 89 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 53 or 89, heavy chain CDR2 comprises an amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 54, heavy chain CDR3 comprises an amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 55, light chain CDR1 comprises an amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 56, light chain CDR2 comprises an amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 57, and light chain CDR3 comprises an amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 58, or

(5) heavy chain CDR1 comprises an amino acid sequence set forth in SEQ ID NO: 61 or 90 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 61 or 90, heavy chain CDR2 comprises an amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 62, heavy chain CDR3 comprises an amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 63, light chain CDR1 comprises an amino acid sequence set forth in SEQ ID NO: 64 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 64, light chain CDR2 comprises an amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 65, and light chain CDR3 comprises an amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 66.

In some specific embodiments, the amino acid sequence of the aforementioned SEQ ID NO: 2 is YIX$_1$YSGYTYYNPSLKS, and the amino acid sequence of the aforementioned SEQ ID NO: 6 is QQX$_2$TTSPYT, wherein

$$X_1 = S \text{ or } Y, \text{ and } X_2 = F \text{ or } Y;$$

and the amino acid sequence of the aforementioned SEQ ID NO: 21 is GYX$_3$FTSYWMN, and the amino acid sequence of the aforementioned SEQ ID NO: 22 is X$_4$IX$_5$PYDSETRX$_6$X$_7$QKFX$_8$G, wherein $X_3$ = F or Y, $X_4$ = M or Y, $X_5$ = H or Y, $X_6$ = L or Y, $X_7$ = N or A, and $X_8$ = K or Q.

In some specific embodiments, the amino acid sequence of the aforementioned SEQ ID NO: 2 is YIX$_1$YSGYTYYNPSLKS, and the amino acid sequence of the aforementioned SEQ ID NO: 6 is QQX$_2$TTSPYT, wherein

i) $X_1$ = S or Y, $X_2$ = F, or
ii) $X_1$ = S or Y, $X_2$ = Y;

and the amino acid sequence of the aforementioned SEQ ID NO: 21 is $GYX_3FTSYWMN$, and the amino acid sequence of the aforementioned SEQ ID NO: 22 is $X_4IX_5PYDSETRX_6X_7QKFX_8G$, wherein

i) $X_3$ = F or Y, $X_4$ = M or Y, $X_5$ = H or Y, $X_6$ = L, $X_7$ = N or A, and $X_8$ = K, or
ii) $X_3$ = F or Y, $X_4$ = M or Y, $X_5$ = H or Y, $X_6$ = Y, $X_7$ = N or A, and $X_8$ = Q.

In some specific embodiments, the amino acid sequence of the aforementioned SEQ ID NO: 2 is $YIX_1YSGYTYYNPSLKS$, and the amino acid sequence of the aforementioned SEQ ID NO: 6 is $QQX_2TTSPYT$, wherein

i) $X_1$ = S or Y, $X_2$ = F, or
ii) $X_1$ = S or Y, $X_2$ = Y;

and the amino acid sequence of the aforementioned SEQ ID NO: 21 is $GYX_3FTSYWMN$, and the amino acid sequence of the aforementioned SEQ ID NO: 22 is $X_4IX_5PYDSETRX_6X_7QKFX_8G$, wherein

i) $X_3$ = F or Y, $X_4$ = M, $X_5$ = H, $X_6$ = L, $X_7$ = N, and $X_8$ = K,
ii) $X_3$ = F or Y, $X_4$ = M, $X_5$ = H, $X_6$ = Y, $X_7$ = A, and $X_8$ = Q,
iii) $X_3$ = F or Y, $X_4$ = Y, $X_5$ = H, $X_6$ = Y, $X_7$ = A, and $X_8$ = Q,
iv) $X_3$ = F or Y, $X_4$ = M, $X_5$ = Y, $X_6$ = Y, $X_7$ = A, and $X_8$ = Q, or
v) $X_3$ = F or Y, $X_4$ = M, $X_5$ = H, $X_6$ = L, $X_7$ = A, and $X_8$ = K.

In one aspect, the present disclosure provides an isolated anti-IL-36R antibody or an antigen-binding fragment thereof, comprising: heavy chain CDR1, heavy chain CDR2 and heavy chain CDR3 in a heavy chain variable region set forth in SEQ ID NO: 7, 9, 11, 13, 27, 29, 31, 33, 35, 37, 51, 59 or 67, and/or light chain CDR1, light chain CDR2 and light chain CDR3 in a light chain variable region set forth in SEQ ID NO: 15, 17, 19, 39, 41, 43, 52, 60 or 68.

In some embodiments, the anti-IL-36R antibody or the antigen-binding fragment thereof comprises:

(1) heavy chain CDR1, heavy chain CDR2 and heavy chain CDR3 in a heavy chain variable region set forth in SEQ ID NO: 7, and light chain CDR1, light chain CDR2 and light chain CDR3 in a light chain variable region set forth in SEQ ID NO: 15;

(2) heavy chain CDR1, heavy chain CDR2 and heavy chain CDR3 in a heavy chain variable region set forth in SEQ ID NO: 9, and light chain CDR1, light chain CDR2 and light chain CDR3 in a light chain variable region set forth in SEQ ID NO: 17;

(3) heavy chain CDR1, heavy chain CDR2 and heavy chain CDR3 in a heavy chain variable region set forth in SEQ ID NO: 11, and light chain CDR1, light chain CDR2 and light chain CDR3 in a light chain variable region set forth in SEQ ID NO: 17;

(4) heavy chain CDR1, heavy chain CDR2 and heavy chain CDR3 in a heavy chain variable region set forth in SEQ ID NO: 13, and light chain CDR1, light chain CDR2 and light chain CDR3 in a light chain variable region set forth in SEQ ID NO: 17;

(5) heavy chain CDR1, heavy chain CDR2 and heavy chain CDR3 in a heavy chain variable region set forth in SEQ ID NO: 9, and light chain CDR1, light chain CDR2 and light chain CDR3 in a light chain variable region set forth in SEQ ID NO: 19;

(6) heavy chain CDR1, heavy chain CDR2 and heavy chain CDR3 in a heavy chain variable region set forth in SEQ ID NO: 11, and light chain CDR1, light chain CDR2 and light chain CDR3 in a light chain variable region set forth in SEQ ID NO: 19;

(7) heavy chain CDR1, heavy chain CDR2 and heavy chain CDR3 in a heavy chain variable region set forth in SEQ ID NO: 13, and light chain CDR1, light chain CDR2 and light chain CDR3 in a light chain variable region set forth in SEQ ID NO: 19;

(8) heavy chain CDR1, heavy chain CDR2 and heavy chain CDR3 in a heavy chain variable region set forth in SEQ ID NO: 27, and light chain CDR1, light chain CDR2 and light chain CDR3 in a light chain variable region set forth in SEQ ID NO: 39;

(9) heavy chain CDR1, heavy chain CDR2 and heavy chain CDR3 in a heavy chain variable region set forth in SEQ ID NO: 29, and light chain CDR1, light chain CDR2 and light chain CDR3 in a light chain variable region set forth in SEQ ID NO: 41;

(10) heavy chain CDR1, heavy chain CDR2 and heavy chain CDR3 in a heavy chain variable region set forth in SEQ ID NO: 31, and light chain CDR1, light chain CDR2 and light chain CDR3 in a light chain variable region set forth in SEQ ID NO: 41;

(11) heavy chain CDR1, heavy chain CDR2 and heavy chain CDR3 in a heavy chain variable region set forth in SEQ ID NO: 33, and light chain CDR1, light chain CDR2 and light chain CDR3 in a light chain variable region set forth in SEQ ID NO: 41;

(12) heavy chain CDR1, heavy chain CDR2 and heavy chain CDR3 in a heavy chain variable region set forth in SEQ ID NO: 35, and light chain CDR1, light chain CDR2 and light chain CDR3 in a light chain variable region set forth in SEQ ID NO: 41;

(13) heavy chain CDR1, heavy chain CDR2 and heavy chain CDR3 in a heavy chain variable region set forth in SEQ ID NO: 37, and light chain CDR1, light chain CDR2 and light chain CDR3 in a light chain variable region set forth in SEQ ID NO: 41;

(14) heavy chain CDR1, heavy chain CDR2 and heavy chain CDR3 in a heavy chain variable region set forth in SEQ ID NO: 29, and light chain CDR1, light chain CDR2 and light chain CDR3 in a light chain variable region set forth in SEQ ID NO: 43;

(15) heavy chain CDR1, heavy chain CDR2 and heavy chain CDR3 in a heavy chain variable region set forth in SEQ ID NO: 31, and light chain CDR1, light chain CDR2 and light chain CDR3 in a light chain variable region set forth in SEQ ID NO: 43;

(16) heavy chain CDR1, heavy chain CDR2 and heavy chain CDR3 in a heavy chain variable region set forth in SEQ ID NO: 33, and light chain CDR1, light chain CDR2 and light chain CDR3 in a light chain variable region set forth in SEQ ID NO: 43;

(17) heavy chain CDR1, heavy chain CDR2 and heavy chain CDR3 in a heavy chain variable region set forth in SEQ ID NO: 35, and light chain CDR1, light chain CDR2 and light chain CDR3 in a light chain variable region set forth in SEQ ID NO: 43;

(18) heavy chain CDR1, heavy chain CDR2 and heavy chain CDR3 in a heavy chain variable region set forth in SEQ ID NO: 37, and light chain CDR1, light chain CDR2 and light chain CDR3 in a light chain variable region set forth in SEQ ID NO: 43;

(19) heavy chain CDR1, heavy chain CDR2 and heavy chain CDR3 in a heavy chain variable region set forth in SEQ ID NO: 51, and light chain CDR1, light chain CDR2 and light chain CDR3 in a light chain variable region set forth in SEQ ID NO: 52;

(20) heavy chain CDR1, heavy chain CDR2 and heavy chain CDR3 in a heavy chain variable region set forth in SEQ ID NO: 59, and light chain CDR1, light chain CDR2 and light chain CDR3 in a light chain variable region set forth in SEQ ID NO: 60; or

(21) heavy chain CDR1, heavy chain CDR2 and heavy chain CDR3 in a heavy chain variable region set forth in SEQ ID NO: 67, and light chain CDR1, light chain CDR2 and light chain CDR3 in a light chain variable region set forth in SEQ ID NO: 68.

In some embodiments, the anti-IL-36R antibody or the antigen-binding fragment thereof comprises a heavy chain variable region comprising an amino acid sequence set forth in SEQ ID NO: 7, 9, 11, 13, 27, 29, 31, 33, 35, 37, 51, 59 or 67, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 7, 9, 11, 13, 27, 29, 31, 33, 35, 37, 51, 59 or 67.

In some embodiments, the anti-IL-36R antibody or the antigen-binding fragment thereof comprises a light chain variable region comprising an amino acid sequence set forth in SEQ ID NO: 15, 17, 19, 39, 41, 43, 52, 60 or 68, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 15, 17, 19, 39, 41, 43, 52, 60 or 68.

In some embodiments, the anti-IL-36R antibody or the antigen-binding fragment thereof comprises a heavy chain variable region and a light chain variable region, wherein

(1) the heavy chain variable region comprises an amino acid sequence set forth in SEQ ID NO: 7 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 7, and the light chain variable region comprises an amino acid sequence set forth in SEQ ID NO: 15 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 15,

(2) the heavy chain variable region comprises an amino acid sequence set forth in SEQ ID NO: 9 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 9, and the light chain variable region comprises an amino acid sequence set forth in SEQ ID NO: 17 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 17,

(3) the heavy chain variable region comprises an amino acid sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 11, and the light chain variable region comprises an amino acid sequence set forth in SEQ ID NO: 17 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 17,

(4) the heavy chain variable region comprises an amino acid sequence set forth in SEQ ID NO: 13 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 13, and the light chain variable region comprises an amino acid sequence set forth in SEQ ID NO: 17 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 17,

(5) the heavy chain variable region comprises an amino acid sequence set forth in SEQ ID NO: 9 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 9, and the light chain variable region comprises an amino acid sequence set forth in SEQ ID NO: 19 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 19,

(6) the heavy chain variable region comprises an amino acid sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 11, and the light chain variable region comprises an amino acid sequence set forth in SEQ ID NO: 19 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 19,

(7) the heavy chain variable region comprises an amino acid sequence set forth in SEQ ID NO: 13 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 13, and the light chain variable region comprises an amino acid sequence set forth in SEQ ID NO: 19 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 19,

(8) the heavy chain variable region comprises an amino acid sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 27, and the light chain variable region comprises an amino acid sequence set forth in SEQ ID NO: 39 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 39,

(9) the heavy chain variable region comprises an amino acid sequence set forth in SEQ ID NO: 29 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 29, and the light chain variable region comprises an amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 41,

(10) the heavy chain variable region comprises an amino acid sequence set forth in SEQ ID NO: 31 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 31, and the light chain variable region comprises an amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 41,

(11) the heavy chain variable region comprises an amino acid sequence set forth in SEQ ID NO: 33 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 33, and the light chain variable region comprises an amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 41,

(12) the heavy chain variable region comprises an amino acid sequence set forth in SEQ ID NO: 35 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 35, and the light chain variable region comprises an amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 41,

(13) the heavy chain variable region comprises an amino acid sequence set forth in SEQ ID NO: 37 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 37, and the light chain variable region comprises an amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 41,

(14) the heavy chain variable region comprises an amino acid sequence set forth in SEQ ID NO: 29 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 29, and the light chain variable region comprises an amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 43,

(15) the heavy chain variable region comprises an amino acid sequence set forth in SEQ ID NO: 31 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 31, and the light chain variable region comprises an amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 43,

(16) the heavy chain variable region comprises an amino acid sequence set forth in SEQ ID NO: 33 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 33, and the light chain variable region comprises an amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 43,

(17) the heavy chain variable region comprises an amino acid sequence set forth in SEQ ID NO: 35 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 35, and the light chain variable region comprises an amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 43,

(18) the heavy chain variable region comprises an amino acid sequence set forth in SEQ ID NO: 37 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 37, and the light chain variable region comprises an amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 43,

(19) the heavy chain variable region comprises an amino acid sequence set forth in SEQ ID NO: 51 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 51, and the light chain variable region comprises an amino acid sequence set forth in SEQ ID NO: 52 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 52,

(20) the heavy chain variable region comprises an amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 59, and the light chain variable region comprises an amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 60, or

(21) the heavy chain variable region comprises an amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 67, and the light chain variable region comprises an amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 68.

In some specific embodiments, amino acid sequences set forth in SEQ ID NOs: 7, 9, 11, 13, 27, 29, 31, 33, 35 and 37 may be encoded by nucleic acids set forth in SEQ ID NOs: 8, 10, 12, 14, 28, 30, 32, 34, 36 and 38, respectively, and amino acid sequences set forth in SEQ ID NOs: 15, 17, 19, 39, 41 and 43 may be encoded by nucleic acids set forth in SEQ ID NOs: 16, 18, 20, 40, 42 and 44, respectively.

In some embodiments, the anti-IL-36R antibody or the antigen-binding fragment thereof comprises a heavy chain comprising a heavy chain variable region and a heavy chain constant region, and a light chain comprising a light chain variable region and a light chain constant region, wherein the C-terminus of the heavy chain variable region is linked to the N-terminus of the heavy chain constant region, and the C-terminus of the light chain variable region is linked to the N-terminus of the light chain constant region. The heavy chain constant region may be a human IgG1 or human IgG4 heavy chain constant region or a fragment thereof or a variant thereof, for example, a human IgG1 heavy chain constant region comprising an amino acid sequence set forth in SEQ ID NO: 69 or a fragment thereof, or a human IgG4 heavy chain constant region comprising an amino acid sequence set forth in SEQ ID NO: 71 or a fragment thereof, or a variant having 1, 2, 3, 4 or 5 amino acid substitutions, deletions and additions compared to the amino acid sequence set forth in SEQ ID NO: 69 or 71. The light chain constant region may be a human κ light chain constant region or a fragment thereof, for example, a human κ light chain constant region comprising an amino acid sequence set forth in SEQ ID NO: 73 or a fragment thereof, or a variant having 1, 2, 3, 4 or 5 amino acid substitutions, deletions and additions compared to the amino acid sequence set forth in SEQ ID NO: 73. In some embodiments, the heavy chain constant region may also comprise a mouse IgG1 or a mouse IgG4 heavy chain constant region or a fragment thereof, and the light chain constant region may also comprise a mouse κ light chain constant region or a fragment thereof.

In some embodiments, the anti-IL-36R antibody or the antigen-binding fragment thereof comprises a heavy chain comprising a heavy chain variable region and a heavy chain constant region, wherein the heavy chain variable region comprises an amino acid sequence set forth in SEQ ID NO: 7, 9, 11, 13, 27, 29, 31, 33, 35, 37, 51, 59 or 67, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 7, 9, 11, 13, 27, 29, 31, 33, 35, 37, 51, 59 or 67; the amino acid sequence of the heavy chain constant region is set forth in SEQ ID NO: 69 or 71, or has 1, 2, 3, 4 or 5 amino acid substitutions, deletions and additions compared to the amino acid sequence set forth in SEQ ID NO: 69 or 71.

In some embodiments, the anti-IL-36R antibody or the antigen-binding fragment thereof comprises a light chain comprising a light chain variable region and a light chain constant region, wherein the light chain variable region comprises an amino acid sequence set forth in SEQ ID NO: 15, 17, 19, 39, 41, 43, 52, 60 or 68, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 15, 17, 19, 39, 41, 43, 52, 60 or 68; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73.

In some embodiments, the anti-IL-36R antibody or the antigen-binding fragment thereof comprises a heavy chain and a light chain, wherein

(1) the heavy chain comprises a heavy chain variable region and a heavy chain constant region, wherein the heavy chain variable region comprises an amino acid sequence set forth in SEQ ID NO: 7, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 7; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71; the light chain comprises a light chain variable region and a light chain constant region, wherein the light chain variable region comprises an amino acid sequence set forth in SEQ ID NO: 15, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 15; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(2) the heavy chain comprises a heavy chain variable region and a heavy chain constant region, wherein the heavy chain variable region comprises an amino acid sequence set forth in SEQ ID NO: 9, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 9; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71; the light chain comprises a light chain variable region and a light chain constant region, wherein the light chain variable region comprises an amino acid sequence set forth in SEQ ID NO: 17, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 17; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(3) the heavy chain comprises a heavy chain variable region and a heavy chain constant region, wherein the heavy chain variable region comprises an amino acid sequence set forth in SEQ ID NO: 11, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 11; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 17, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 17; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(4) the heavy chain comprises a heavy chain variable region and a heavy chain constant region, wherein the heavy chain variable region comprises an amino acid sequence set forth in SEQ ID NO: 13, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 13; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 17, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 17; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(5) the heavy chain comprises a heavy chain variable region and a heavy chain constant region, wherein the heavy chain variable region comprises an amino acid sequence set forth in SEQ ID NO: 9, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 9; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 19, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 19; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(6) the heavy chain comprises a heavy chain variable region and a heavy chain constant region, wherein the heavy chain variable region comprises an amino acid sequence set forth in SEQ ID NO: 11, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 11; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 19, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 19; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(7) the heavy chain comprises a heavy chain variable region and a heavy chain constant region, wherein the heavy chain variable region comprises an amino acid sequence set forth in SEQ ID NO: 13, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 13; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 19, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 19; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(8) the heavy chain comprises a heavy chain variable region and a heavy chain constant region, wherein the heavy chain variable region comprises an amino acid sequence set forth in SEQ ID NO: 27, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 27; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 39, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 39; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(9) the heavy chain comprises a heavy chain variable region and a heavy chain constant region, wherein the heavy chain variable region comprises an amino acid sequence set forth in SEQ ID NO: 29, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 29; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 41, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 41; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(10) the heavy chain comprises a heavy chain variable region and a heavy chain constant region, wherein the heavy chain variable region comprises an amino acid sequence set forth in SEQ ID NO: 31, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 31; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 41, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 41; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(11) the heavy chain comprises a heavy chain variable region and a heavy chain constant region, wherein the heavy chain variable region comprises an amino acid sequence set forth in SEQ ID NO: 33, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 33; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 41, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 41; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(12) the heavy chain comprises a heavy chain variable region and a heavy chain constant region, wherein the heavy chain variable region comprises an amino acid sequence set forth in SEQ ID NO: 35, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 35; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 41, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 41; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(13) the heavy chain comprises a heavy chain variable region and a heavy chain constant region, wherein the heavy chain variable region comprises an amino acid sequence set forth in SEQ ID NO: 37, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 37; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 41, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 41; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(14) the heavy chain comprises a heavy chain variable region and a heavy chain constant region, wherein the heavy chain variable region comprises an amino acid sequence set forth in SEQ ID NO: 29, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 29; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 43, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 43; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(15) the heavy chain comprises a heavy chain variable region and a heavy chain constant region, wherein the heavy chain variable region comprises an amino acid sequence set forth in SEQ ID NO: 31, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 31; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 43, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 43; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(16) the heavy chain comprises a heavy chain variable region and a heavy chain constant region, wherein the heavy chain variable region comprises an amino acid sequence set forth in SEQ ID NO: 33, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 33; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 43, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 43; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(17) the heavy chain comprises a heavy chain variable region and a heavy chain constant region, wherein the heavy chain variable region comprises an amino acid sequence set forth in SEQ ID NO: 35, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 35; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 43, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 43; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(18) the heavy chain comprises a heavy chain variable region and a heavy chain constant region, wherein the heavy chain variable region comprises an amino acid sequence set forth in SEQ ID NO: 37, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 37; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 43, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 43; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(19) the heavy chain comprises a heavy chain variable region and a heavy chain constant region, wherein the heavy chain variable region comprises an amino acid sequence set forth in SEQ ID NO: 51, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 51; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 52, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 52; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73;

(20) the heavy chain comprises a heavy chain variable region and a heavy chain constant region, wherein the heavy chain variable region comprises an amino acid sequence set forth in SEQ ID NO: 59, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 59; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 60, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 60; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73; or

(21) the heavy chain comprises a heavy chain variable region and a heavy chain constant region, wherein the heavy chain variable region comprises an amino acid sequence set forth in SEQ ID NO: 67, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 67; the amino acid sequence of the heavy chain constant region is set forth in SEQ ID NO: 71, or has 1, 2, 3, 4 or 5 amino acid substitutions, deletions and additions compared to the amino acid sequence set forth in SEQ ID NO: 71;

the light chain comprises a light chain variable region and a light chain constant region, wherein the light chain variable region comprises an amino acid sequence set forth in SEQ ID NO: 68, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to the amino acid sequence set forth in SEQ ID NO: 68; the amino acid sequence of the light chain constant region is set forth in SEQ ID NO: 73, or has 1, 2, 3, 4 or 5 amino acid substitutions, deletions and additions compared to the amino acid sequence set forth in SEQ ID NO: 73.

In some specific embodiments, amino acid sequences set forth in SEQ ID NOs: 69, 71 and 73 may be encoded by nucleic acids set forth in SEQ ID NOs: 70, 72 and 74, respectively.

In some embodiments, the anti-IL-36R antibody of the present disclosure comprises or consists of two heavy chains and two light chains, the heavy chains and the light chains being linked to each other by disulfide bonds, wherein each of the heavy chain comprises the heavy chain constant region, the heavy chain variable region or CDR described above, and each of the light chain comprises the light chain constant region, the light chain variable region or CDR described above, wherein the C-terminus of the heavy chain variable region is linked to the N-terminus of the heavy chain constant region, and the C-terminus of the light chain variable region is linked to the N-terminus of the light chain constant region. The anti-IL-36R antibody of the present disclosure may be a full length antibody, e.g., a full length antibody of IgG1, IgG2 or IgG4 isotype, preferably IgG1 or IgG4.

In some embodiments, the anti-IL-36R antibody or the antigen-binding fragment thereof of the present disclosure may be an IgG4 isotype antibody. In other embodiments, the anti-IL-36R antibody or the antigen-binding fragment thereof of the present disclosure may be a single-chain variable fragment (scFv) antibody, or an antibody fragment, e.g., an Fab, an F(ab')$_2$ fragment, an Fd fragment, an Fv fragment, a dAb, or an isolated CDR region.

In one aspect, the present disclosure provides a multispecific molecule comprising the anti-IL-36R antibody or the antigen-binding fragment thereof of the present disclosure, wherein the anti-IL-36R antibody or the antigen-binding fragment thereof of the present disclosure is linked to at least one other functional portion with a different specificity thereto, and the other functional portion comprises another peptide or protein (e.g., another antibody or an antigen-binding fragment thereof, a cytokine, and/or a receptor ligand) to produce a multispecific molecule that binds to at least two different binding sites or targets. The "multispecific molecule" described in the present disclosure encompasses a molecule with two types of specificity (i.e., a bispecific molecule), three types of specificity (i.e., a trispecific molecule), four types of specificity (i.e., a tetraspecific molecule), or more specificities. In some specific embodiments, the "multispecific molecule" described in the present disclosure encompasses a multispecific antibody, e.g., a bispecific antibody, a trispecific antibody, or a tetraspecific antibody. These bispecific molecules can be prepared by genetic engineering, chemical methods, etc.

The present disclosure also provides an immunoconjugate comprising the anti-IL-36R antibody or the antigen-binding fragment thereof of the present disclosure, wherein the anti-IL-36R antibody or the antigen-binding fragment thereof of the present disclosure is linked to a therapeutic agent (e.g., a cytotoxin, a cytotoxic drug, etc.), e.g., an antibody-drug conjugate (ADC). In some embodiments, the linkage is a covalent linkage. In some embodiments, the linkage is a non-covalent linkage. In some embodiments, the linkage is a direct linkage. In some embodiments, the linkage is via a linker. A suitable linker includes, but is not limited to, an amino acid or a polypeptide linker.

The anti-IL-36R antibody or the antigen-binding fragment thereof of the present disclosure may also be part of a chimeric antigen receptor (CAR). The present disclosure also provides immune cells comprising the chimeric antigen receptor, e.g., T cells (i.e., CAR-T cells).

In addition, the present disclosure also provides a gene vector. The gene vector comprises a gene encoding the anti-IL-36R antibody or the antigen-binding fragment thereof of the present disclosure, and enables the gene to enter mammalian cells (preferably human cells) and to express therein. Such genetic vectors include, but are not limited to, naked plasmid vectors, yeast plasmid vectors, adenoviral vectors, adeno-associated viral vectors, retroviral vectors, poxvirus vectors, rhabdovirus vectors, or baculovirus vectors. Techniques for inserting DNA into these gene vectors are well known to those of ordinary skill in the art.

In one aspect, the present disclosure also provides a nucleic acid molecule encoding the anti-IL-36R antibody or the antigen-binding fragment thereof of the present disclosure, an expression vector comprising the nucleic acid molecule, and a host cell comprising the nucleic acid molecule or the expression vector.

In one aspect, the present disclosure also provides a pharmaceutical composition comprising the anti-IL-36R antibody, the antigen-binding fragment thereof, the multispecific molecule, the immunoconjugate, the CAR-T cell, the gene vector or the nucleic acid molecule of the present disclosure, and a pharmaceutically acceptable excipient, a diluent, or a carrier.

In one aspect, the present disclosure also provides a method for preparing an anti-IL-36R antibody or an antigen-binding fragment thereof, which comprises the following steps: (i) expressing the anti-IL-36R antibody or the antigen-binding fragment thereof in a host cell, and (ii) isolating the anti-IL-36R antibody or the antigen-binding fragment thereof from a host cell or a cell culture thereof.

In another aspect, the present disclosure provides a method for inhibiting signal transduction of IL-36/IL-36R in a subject, comprising administering to the subject a therapeutically effective amount of the anti-IL-36R antibody or the antigen-binding fragment thereof, the pharmaceutical composition, the multispecific molecule, the immunoconjugate, the CAR-T cell, the gene vector, or the nucleic acid molecule of the present disclosure. In some embodiments, the subject is a human. In some embodiments, the subject has an autoimmune disease, an inflammatory disease, a respiratory disease, a metabolic disorder, or cancer.

In another aspect, the present disclosure provides a method for treating or preventing relevant IL-36/IL-36R-mediated diseases and disorders in a subject, comprising administering to the subject a therapeutically effective amount of the anti-IL-36R antibody or the antigen-binding fragment thereof, the pharmaceutical composition, the multispecific molecule, the immunoconjugate, the CAR-T cell, the gene vector, or the nucleic acid molecule of the present disclosure. The present disclosure also provides use of the anti-IL-36R antibody or the antigen-binding fragment thereof, the pharmaceutical composition, the multispecific molecule, the immunoconjugate, the CAR-T cell, the gene vector, or the nucleic acid molecule of the present disclosure in preparing a medicament for treating or preventing relevant IL-36/IL-36R-mediated diseases and disorders. In some embodiments, in the method and the use, the subject is a human.

In some embodiments, in the method and the use, the relevant IL-36/IL-36R-mediated diseases and disorders include autoimmune diseases, inflammatory diseases, respiratory diseases, metabolic disorders, or cancer. In some embodiments, in the method and the use, the relevant IL-36/IL-36R-mediated diseases and disorders include multiple sclerosis, scleroderma, asthma, chronic obstructive pulmonary disease, hidradenitis suppurativa, arthritis, ankylosing spondylitis, psoriasis, palmoplantar pustulosis, systemic lupus erythematosus, atopic dermatitis, acne vulgaris, skin rash, allergic rhinitis, nephritis, inflammatory bowel disease, and/or food allergy. In some specific embodiments, the arthritis includes rheumatoid arthritis, and psoriatic arthritis. In some specific embodiments, the psoriasis includes psoriasis vulgaris, pustular psoriasis, local pustular psoriasis, and generalized pustular psoriasis. In some specific embodiments, the inflammatory bowel disease includes Crohn's disease and ulcerative colitis. In some embodiments, at least one other antibody, e.g., an anti-IgE antibody, an anti-IL4 antibody, an anti-IL13 antibody and/or an anti-IL5 antibody, may be administered together with the anti-IL-36R antibody or the antigen-binding fragment thereof of the present disclosure. In some embodiments, at least one other drug, e.g., an anti-allergic agent, may be administered together with the anti-IL-36R antibody or the antigen-binding fragment thereof of the present disclosure. In some embodiments, in the method and the use, the relevant IL-36/IL-36R-mediated disease and disorder is a solid tumor or a non-solid tumor. In some embodiments, at least one other antibody, e.g., an anti-PD-1 antibody, an anti-PD-L1 antibody and/or an anti-CTLA-4 antibody, can be administered together with the anti-IL-36R antibody or the antigen-binding fragment thereof of the present disclosure. In some embodiments, at least one other drug, e.g., a small-molecule anti-cancer agent, may be administered together with the anti-IL-36R antibody or the antigen-binding fragment thereof of the present disclosure.

Other features and advantages of the present disclosure will become clearer based on the following detailed description and examples, which are not to be construed as limiting. The content of all of the documents, Genbank records, patents and published patent applications cited in the present disclosure is expressly incorporated into the present disclosure by reference.

## BRIEF DESCRIPTION OF THE DRAWINGS

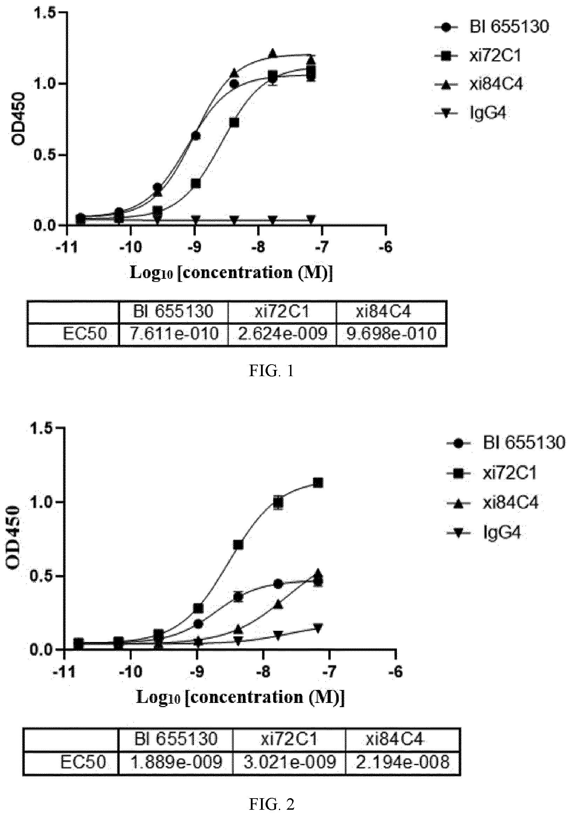

FIG. 1: the binding of a chimeric anti-IL-36R antibody to hIL-36R detected by ELISA. FIG. 1 shows the binding of chimeric antibodies xi72C1 and xi84C4 to hIL-36R. The binding of BI655130 is shown as a control, and the binding of IgG4 is shown as a negative control.

FIG. 2: the binding of a chimeric anti-IL-36R antibody to CynoIL-36R detected by ELISA. FIG. 2 shows the binding of chimeric antibodies xi72C1 and xi84C4 to CynoIL-36R. The binding of BI655130 is shown as a control, and the binding of IgG4 is shown as a negative control.

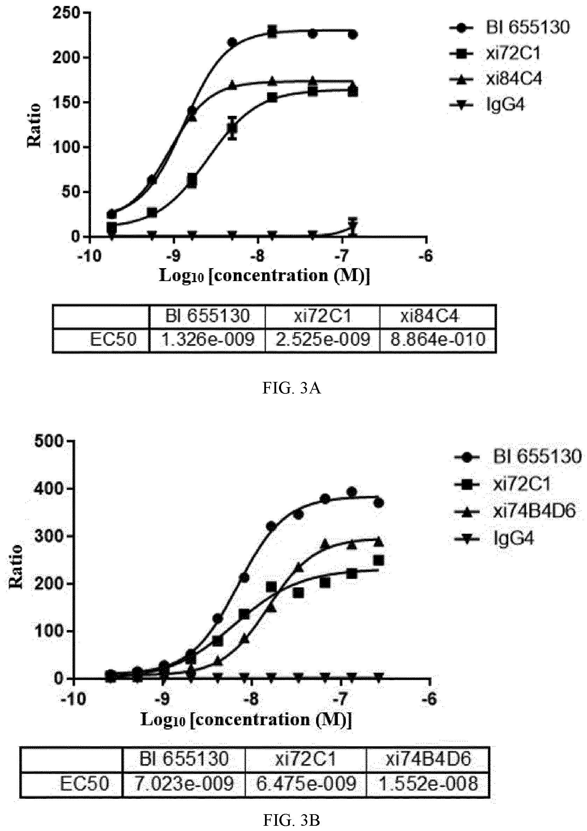

FIGs. 3A-3B: the binding of a chimeric anti-IL-36R antibody to hIL-36R-overexpressing cells detected by FACS. FIG. 3A shows the binding of chimeric antibodies xi72C1 and xi84C4 to 293T-hIL36R-NF$\kappa$B cells and FIG. 3B shows the binding of chimeric antibodies xi72C1 and xi74B4D6 to 293T-hIL36R-NF$\kappa$B cells. The binding of BI655130 is shown as a control, and the binding of IgG4 is shown as a negative control.

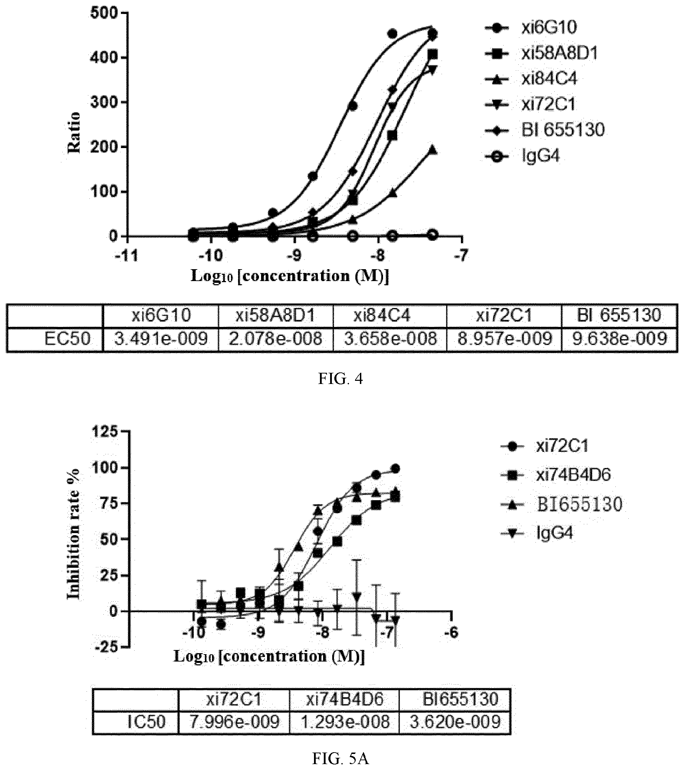

FIG. 4: the binding of a chimeric anti-IL-36R antibody to CynoIL-36R-overexpressing cells detected by FACS. FIG. 4 shows the binding of chimeric antibodies xi72C1, xi84C4, xi58A8D1 and xi6G10 to 293T-CynoIL36R-NF$\kappa$B cells. The binding of BI655130 is shown as a control, and the binding of IgG4 is shown as a negative control.

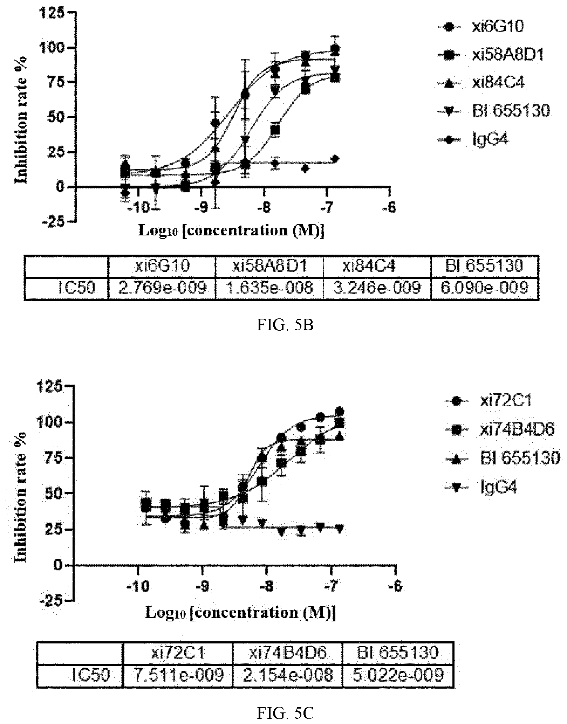

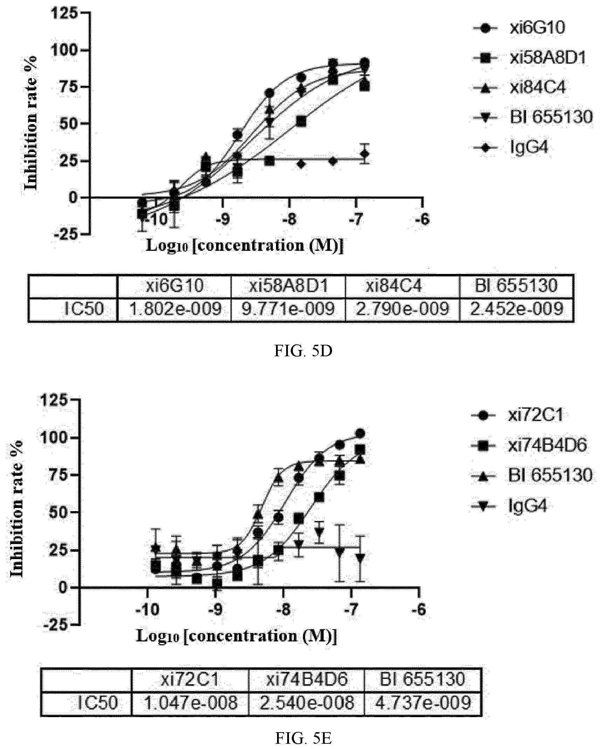

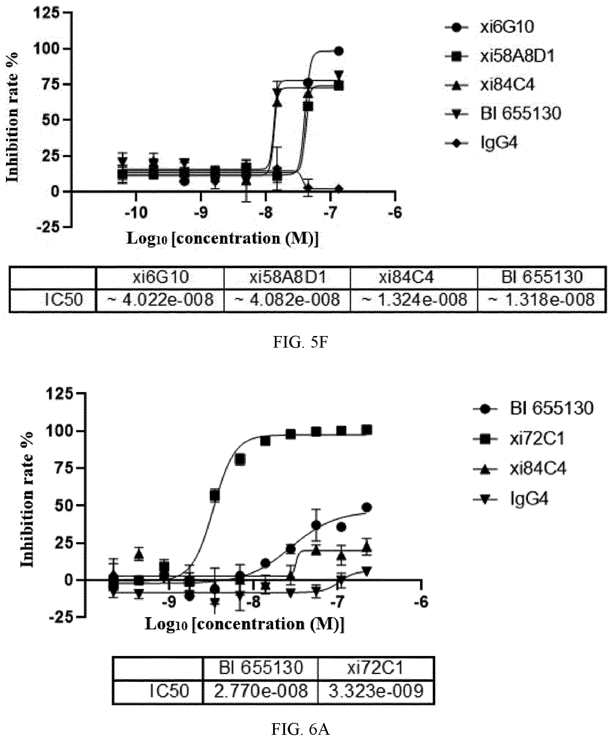

FIGs. 5A-5F: the function of a chimeric anti-IL-36R antibody blocking the interaction between hIL36$\alpha$, hIL36$\beta$ and hIL36$\gamma$ proteins, and hIL-36R-overexpressing cells. FIGs. 5A-5B show the results of chimeric antibodies xi72C1, xi74B4D6, xi84C4, xi58A8D1 and xi6G10 blocking the interaction between hIL36$\alpha$ and 293T-hIL36R-NF$\kappa$B cells; FIGs. 5C-5D show the results of chimeric antibodies xi72C1, xi74B4D6, xi84C4, xi58A8D1 and xi6G10 blocking the interaction between hIL36$\beta$ and 293T-hIL36R-NF$\kappa$B cells; FIGs. 5E-5F show the results of chimeric antibodies xi72C1, xi74B4D6, xi84C4, xi58A8D1 and xi6G10 blocking the interaction between hIL36$\gamma$ and 293T-hIL36R-NF$\kappa$B cells. BI655130 blocking is shown as a control, and IgG4 blocking is shown as a negative control.

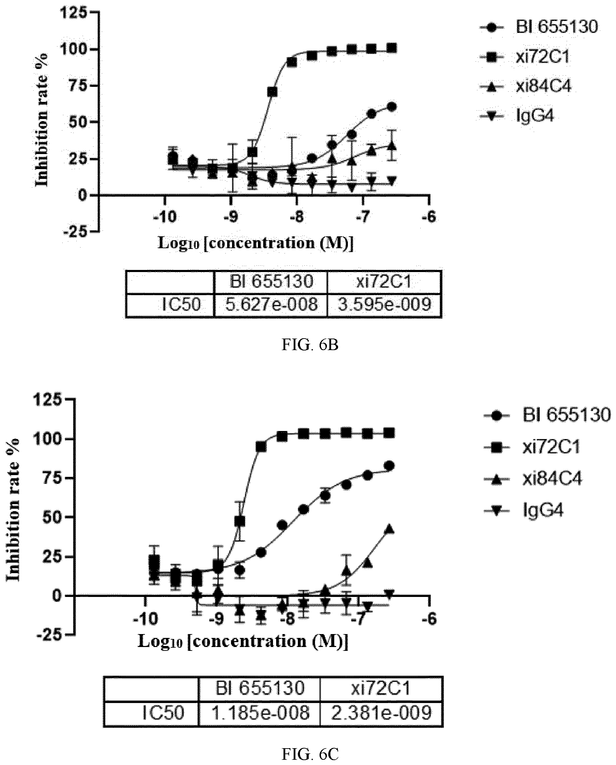

FIGs. 6A-6C: the function of a chimeric anti-IL-36R antibody blocking the interaction between hIL36$\alpha$, hIL36$\beta$ and hIL36$\gamma$ proteins, and CynoIL-36R-overexpressing cells. FIG. 6A shows the results of chimeric antibodies xi72C1 and xi84C4 blocking the interaction between hIL36$\alpha$ and 293T-CynoIL36R-NF$\kappa$B cells; FIG. 6B shows the results of chimeric antibodies xi72C1 and xi84C4 blocking the interaction between hIL36$\beta$ and 293T-CynoIL36R-NF$\kappa$B

cells; FIG. 6C shows the results of chimeric antibodies xi72C1 and xi84C4 blocking the interaction between hIL36γ and 293T-CynoIL36R-NFκB cells. BI655130 blocking is shown as a control, and IgG4 blocking is shown as a negative control.

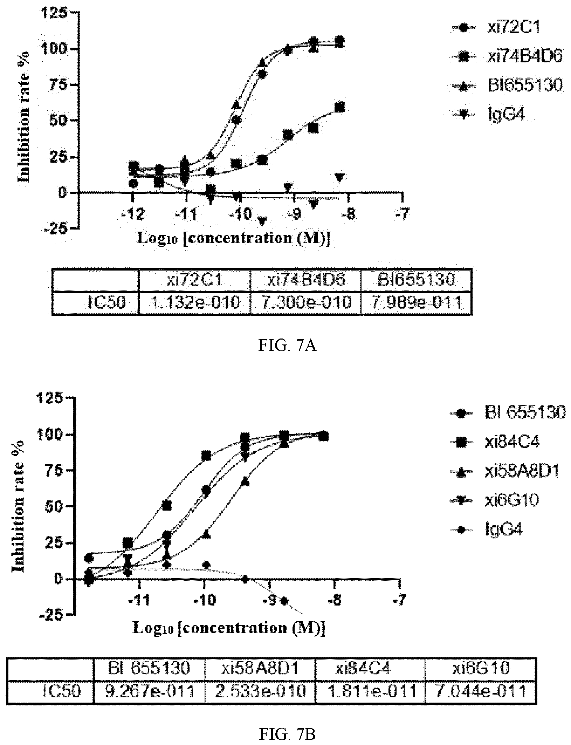

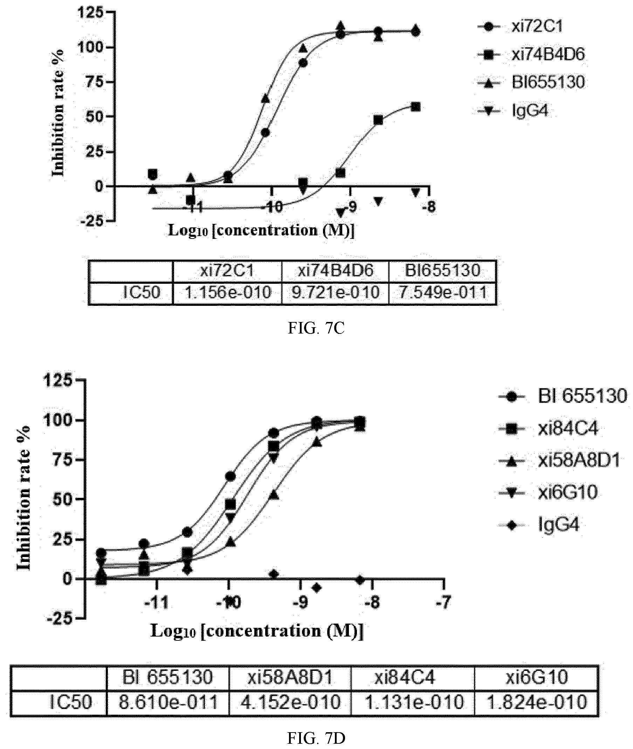

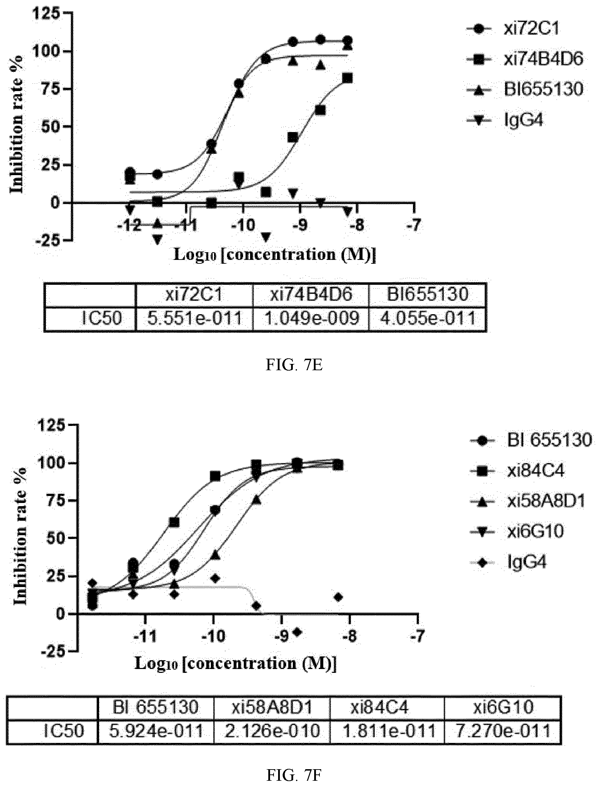

FIGs. 7A-7F: the function of a chimeric anti-IL-36R antibody blocking the interaction between hIL36α, hIL36β and hIL36γ proteins, and OVCAR8 cells. FIGs. 7A-7B show the results of chimeric antibodies xi72C1, xi74B4D6, xi84C4, xi58A8D1 and xi6G10 blocking the interaction between hIL36α and OVCAR8 cells; FIGs. 7C-7D show the results of chimeric antibodies xi72Cl, xi74B4D6, xi84C4, xi58A8D1 and xi6G10 blocking the interaction between hIL36β and OVCAR8 cells; FIGs. 7E-7F show the results of chimeric antibodies xi72C1, xi74B4D6, xi84C4, xi58A8D1 and xi6G10 blocking the interaction between hIL36γ and OVCAR8 cells. BI655130 blocking is shown as a control, and IgG4 blocking is shown as a negative control.

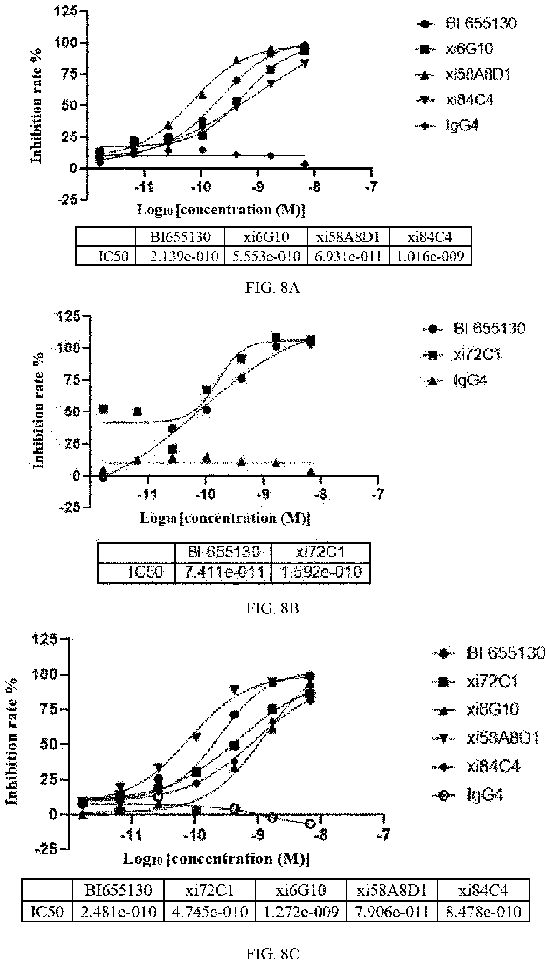

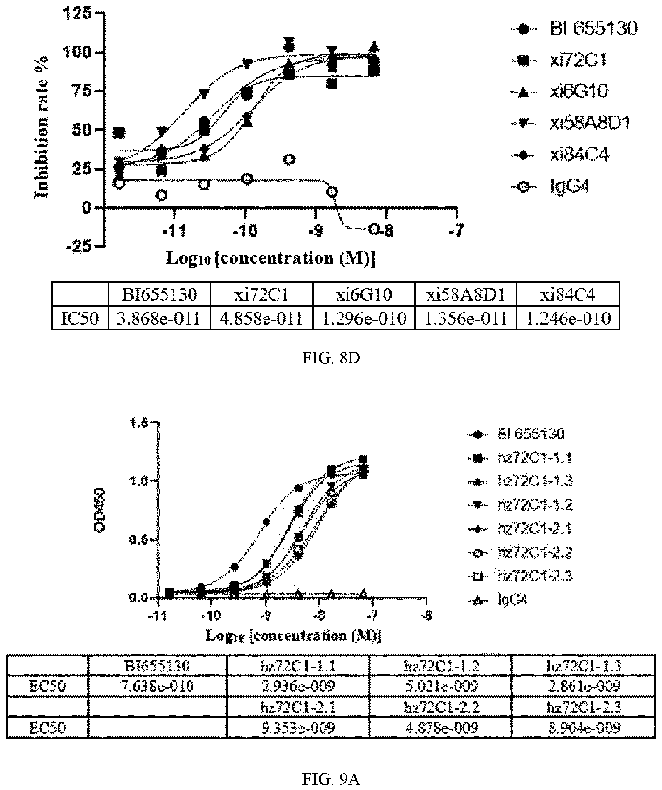

FIGs. 8A-8D: the function of a chimeric anti-IL-36R antibody blocking the interaction between hIL36α, hIL36β and hIL36γ proteins, and keratinocytes (NHK) cells. FIGs. 8A-8B show the results of chimeric antibodies xi84C4, xi58A8D1, xi6G10 and xi72C1 blocking the interaction between hIL36α and NHK cells; FIG. 8C shows the results of chimeric antibodies xi72C1, xi84C4, xi58A8D1 and xi6G10 blocking the interaction between hIL36β and NHK cells; FIG. 8D shows the results of chimeric antibodies xi72C1, xi84C4, xi58A8D1 and xi6G10 blocking the interaction between hIL36γ and NHK cells. BI655130 blocking is shown as a control, and IgG4 blocking is shown as a negative control.

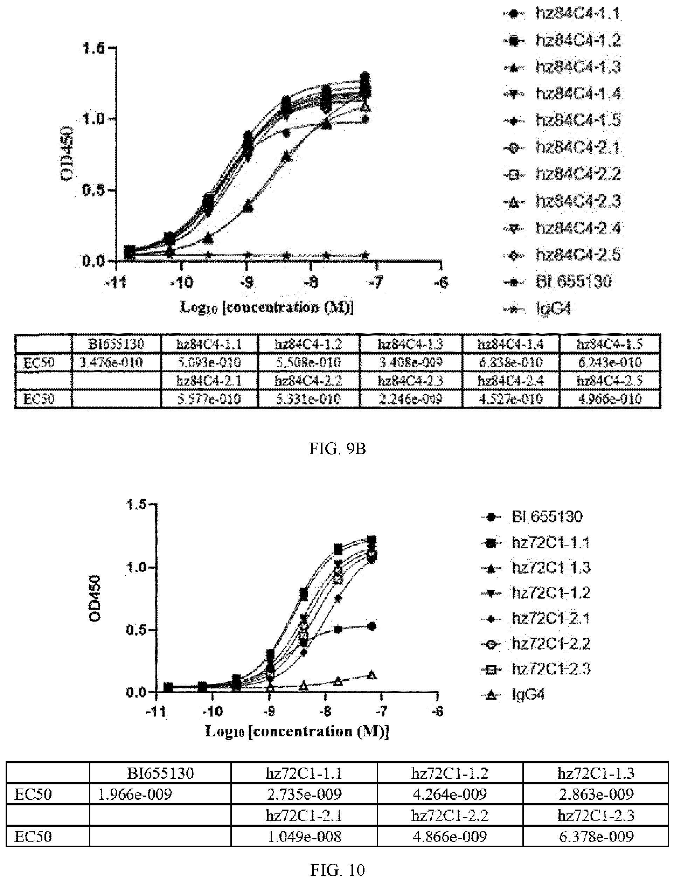

FIGs. 9A-9B: the binding of a humanized anti-IL-36R antibody to hIL-36R detected by ELISA. FIG. 9A shows the binding of humanized antibodies hz72C1-1.1, hz72C1-1.2, hz72C1-1.3, hz72C1-2.1, hz72C1-2.2 and hz72C1-2.3 to hIL-36R; FIG. 9B shows the binding of humanized antibodies hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 to hIL-36R. The binding of BI655130 is shown as a control, and the binding of IgG4 is shown as a negative control.

FIG. 10: the binding of a humanized anti-IL-36R antibody to CynoIL-36R detected by ELISA. FIG. 10 shows the binding of humanized antibodies hz72C1-1.1, hz72C1-1.2, hz72C1-1.3, hz72C1-2.1, hz72C1-2.2 and hz72C1-2.3 to CynoIL-36R. The binding of BI655130 is shown as a control, and the binding of IgG4 is shown as a negative control.

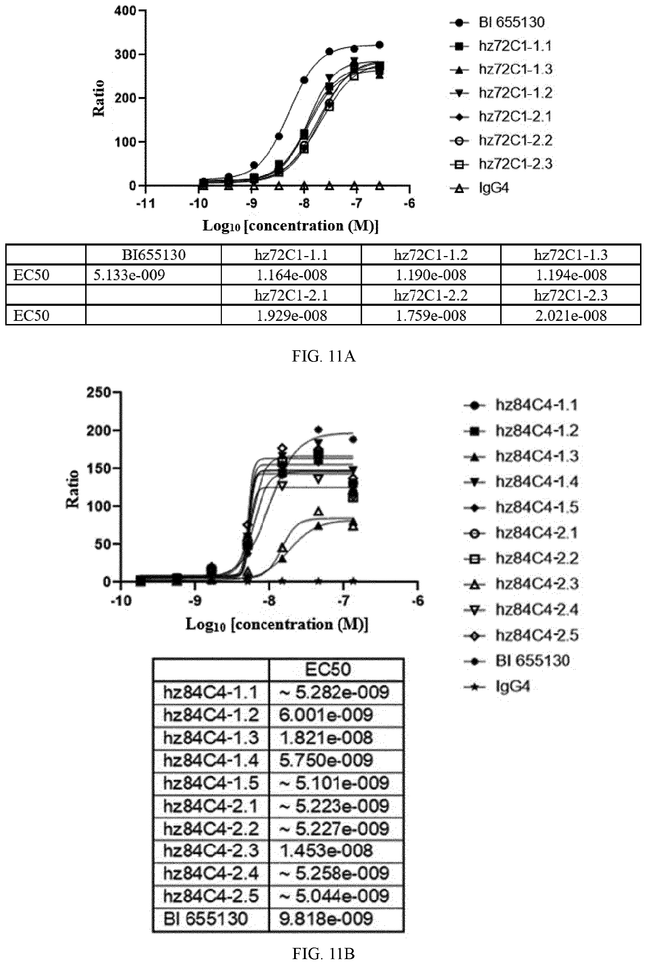

FIGs. 11A-11B: the binding of a humanized anti-IL-36R antibody to hIL-36R-overexpressing cells detected by FACS. FIG. 11A shows the binding of humanized antibodies hz72C1-1.1, hz72C1-1.2, hz72C1-1.3, hz72C1-2.1, hz72C1-2.2 and hz72C1-2.3 to 293T-hIL36R-NFκB cells; FIG. 11B shows the binding of humanized antibodies hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 to 293T-hIL36R-NFxB cells. The binding of BI655130 is shown as a control, and the binding of IgG4 is shown as a negative control.

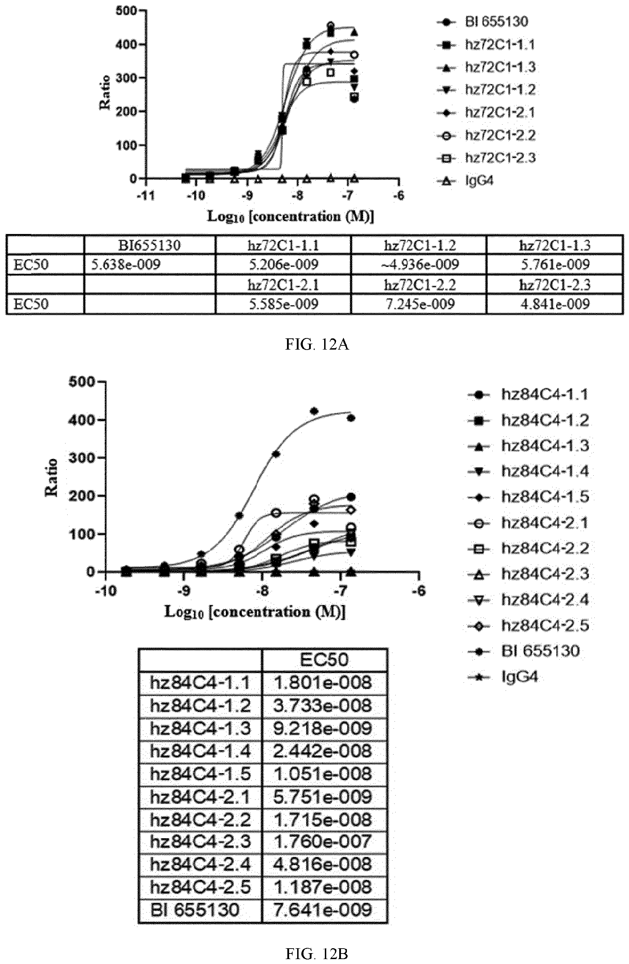

FIGs. 12A-12B: the binding of a humanized anti-IL-36R antibody to CynoIL-36R-overexpressing cells detected by FACS. FIG. 12A shows the binding of humanized antibodies hz72C1-1.1, hz72C1-1.2, hz72C1-1.3, hz72C1-2.1, hz72C1-2.2 and hz72C1-2.3 to 293T-CynoIL36R-NFκB cells; FIG. 12B shows the binding of humanized antibodies hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 to 293T-CynoIL36R-NFκB cells. The binding of BI655130 is shown as a control, and the binding of IgG4 is shown as a negative control.

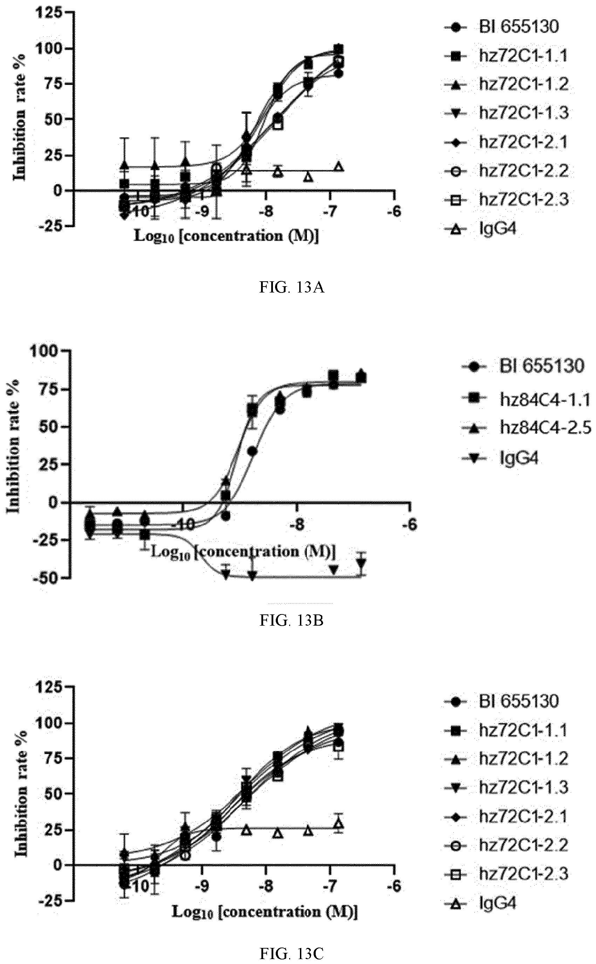

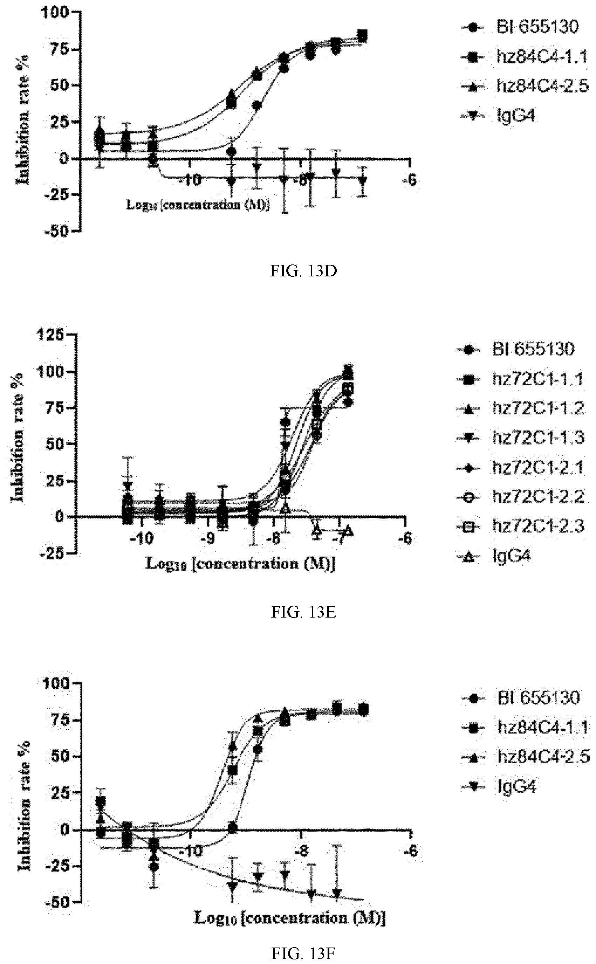

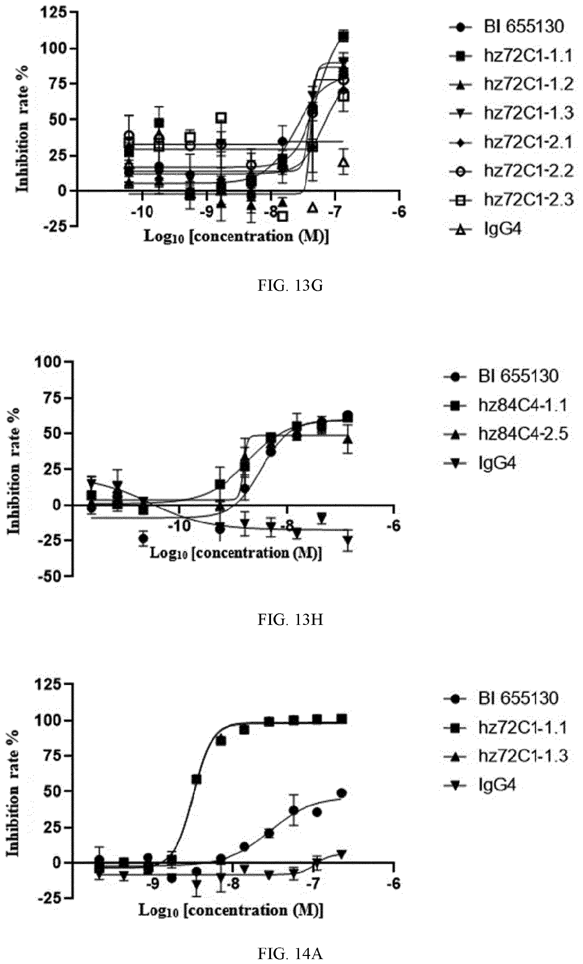

FIGs. 13A-13H: the function of a humanized anti-IL-36R antibody blocking the interaction between hIL36α, hIL36β, hIL36γ and hIL36αβγ proteins, and hIL-36R-overexpressing cells. FIGs. 13A-13B show the results of humanized antibodies hz72C1-1.1, hz72C1-1.2, hz72C1-1.3, hz72C1-2.1, hz72C1-2.2, hz72C1-2.3, hz84C4-1.1 and hz84C4-2.5 blocking the interaction between hIL36α and 293T-hIL36R-NFκB cells; FIGs. 13C-13D show the results of humanized antibodies hz72C1-1.1, hz72C1-1.2, hz72C1-1.3, hz72C1-2.1, hz72C1-2.2, hz72C1-2.3, hz84C4-1.1 and hz84C4-2.5 blocking the interaction between hIL36β and 293T-hIL36R-NFκB cells; FIGs. 13E-13F show the results of humanized antibodies hz72C1-1.1, hz72C1-1.2, hz72C1-1.3, hz72C1-2.1, hz72C1-2.2, hz72C1-2.3, hz84C4-1.1 and hz84C4-2.5 blocking the interaction between hIL36γ and 293T-hIL36R-NFκB cells; FIGs. 13G-13H show the results of humanized antibodies hz72C1-1.1, hz72C1-1.2, hz72C1-1.3, hz72C1-2.1, hz72C1-2.2, hz72C1-2.3, hz84C4-1.1 and hz84C4-2.5 blocking the interaction between hIL36αβγ and 293T-hIL36R-NFκB cells. BI655130 blocking is shown as a control, and IgG4 blocking is shown as a negative control.

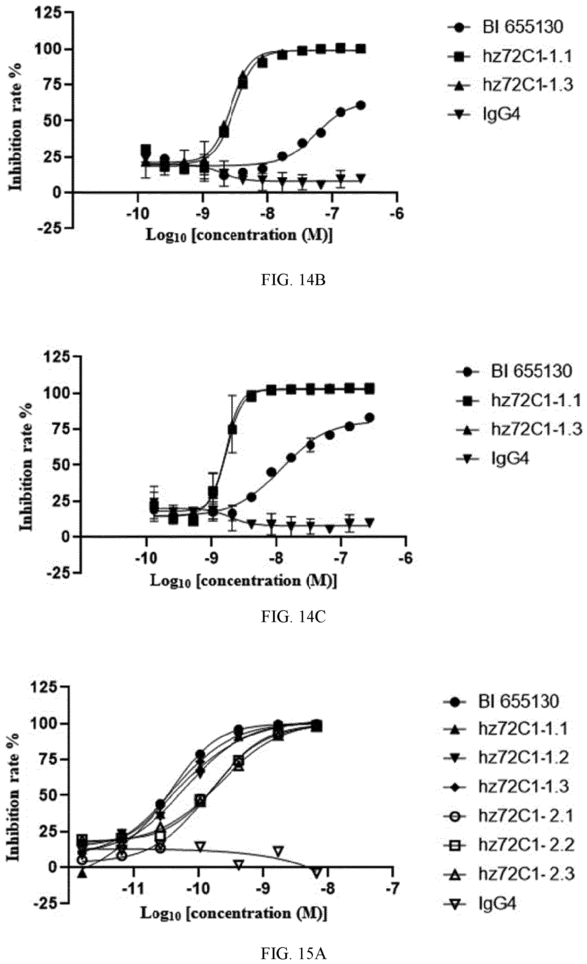

FIGs. 14A-14C: the function of a humanized anti-IL-36R antibody blocking the interaction between hIL36α, hIL36β and hIL36γ proteins, and CynoIL-36R-overexpressing cells. FIG 14A shows the results of humanized antibodies hz72C1-1.1 and hz72C1-1.3 blocking the interaction between hIL36α and 293T-CynoIL36R-NFκB cells; FIG 14B shows the results of humanized antibodies hz72C1-1.1 and hz72C1-1.3 blocking the interaction between hIL36β and 293T-CynoIL36R-NFκB cells; FIG 14C shows the results of humanized antibodies hz72C1-1.1 and hz72C1-1.3 blocking the interaction between hIL36γ and 293T-CynoIL36R-NFκB cells. BI655130 blocking is shown as a control, and IgG4 blocking is shown as a negative control.

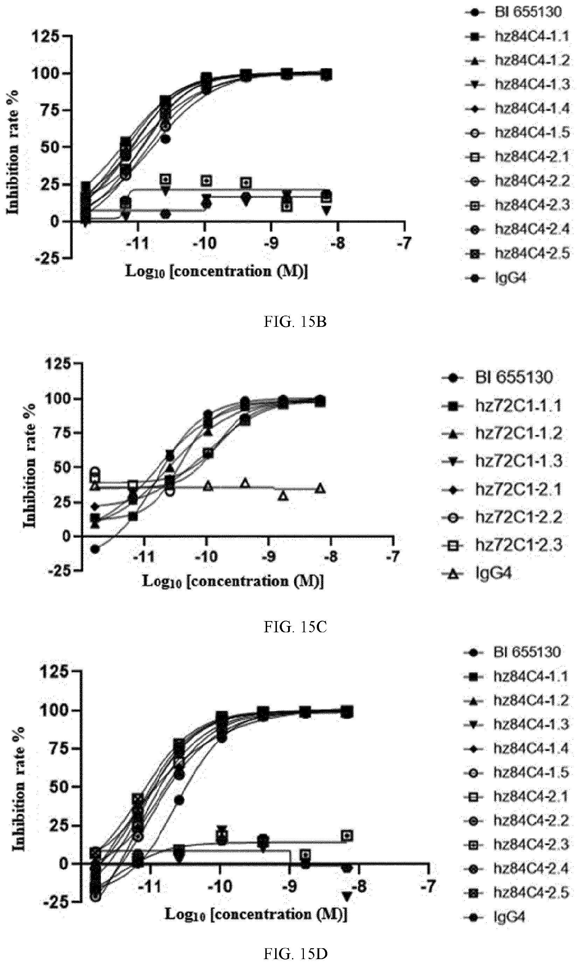

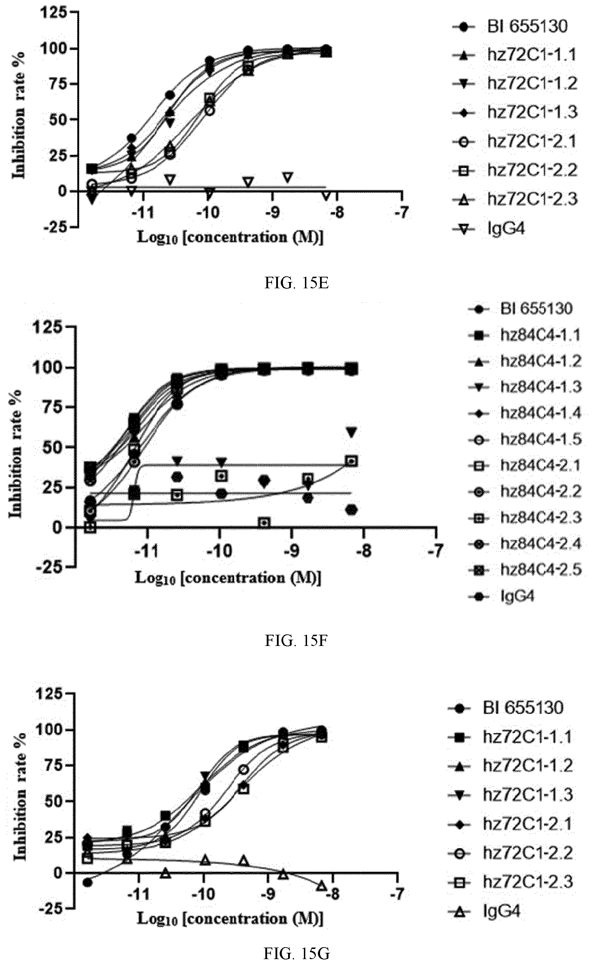

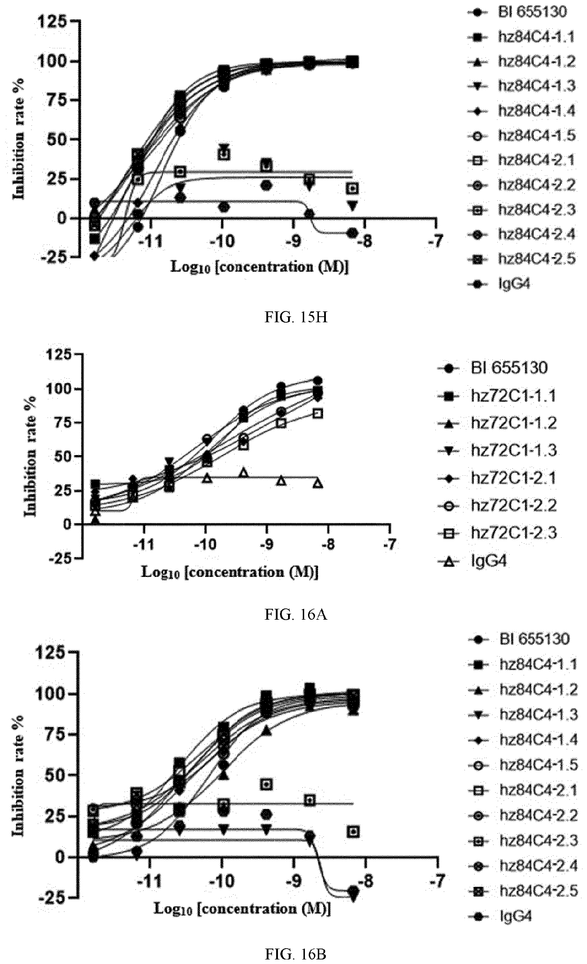

FIGs. 15A-15H: the function of a humanized anti-IL-36R antibody blocking the interaction between hIL36α, hIL36β, hIL36γ and hIL36αβγ proteins, and OVCAR8 cells. FIGs. 15A-15B show the results of humanized antibodies hz72C1-1.1, hz72C1-1.2, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\alpha$ and OVCAR8 cells; FIGs. 15C-15D show the results of humanized antibodies hz72C1-1.1, hz72C1-1.2, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\beta$ and OVCAR8 cells; FIGs. 15E-15F show the results of humanized antibodies hz72C1-1.1, hz72C1-1.2, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\gamma$ and OVCAR8 cells; FIGs. 15G-15H show the results of humanized antibodies hz72C1-1.1, hz72C1-1.2, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\alpha\beta\gamma$ and OVCAR8 cells. BI655130 blocking is shown as a control, and IgG4 blocking is shown as a negative control.

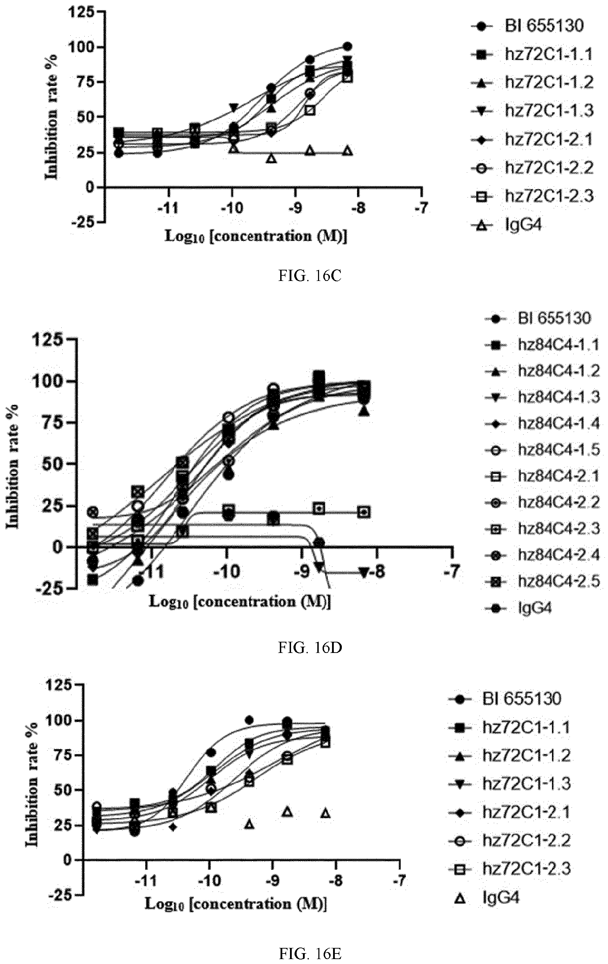

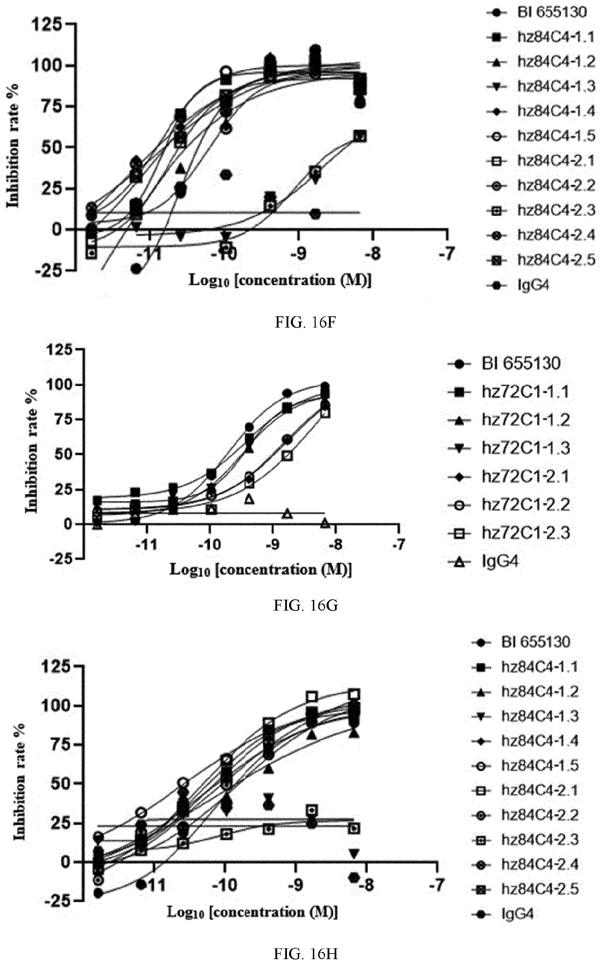

FIGs. 16A-16H: the function of a humanized anti-IL-36R antibody blocking the interaction between hIL36$\alpha$, hIL36$\beta$, hIL36$\gamma$ and hIL36$\alpha\beta\gamma$ proteins, and keratinocytes (NHK) cells. FIGs. 16A-16B show the results of humanized antibodies hz72C1-1.1, hz72C1-12, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\alpha$ and NHK cells; FIGs. 16C-16D show the results of humanized antibodies hz72C1-1.1, hz72C1-12, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\beta$ and NHK cells; FIGs. 16E-16F show the results of humanized antibodies hz72C1-1.1, hz72C1-12, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\gamma$ and NHK cells; FIGs. 16G-16H show the results of humanized antibodies hz72C1-1.1, hz72C1-12, hz72C1-1.3, hz72C1-2.1, hz72C1-2.2, hz72C1-2.3, hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 blocking the interaction between hIL36$\alpha\beta\gamma$ and NHK cells. BI655130 blocking is shown as a control, and IgG4 blocking is shown as a negative control.

## DETAILED SUMMARY

It should be understood that the terminology used in the present disclosure is for the purpose of describing specific embodiments only and is not intended to be limiting. Unless otherwise defined, all of the technical and scientific terms used herein have the same meaning as commonly understood by those of ordinary skill in the art to which the present disclosure belongs.

"IL-36R" refers to a IL-36 receptor, also known as interleukin-1 receptor-like 2 (IL1RL2) or IL-1 receptor-associated protein 2 (IL-1Rrp2), including variants, isoforms, homologs, orthologs and paralogs of IL-36R. For example, in some embodiments, an antibody specific for the human IL-36R protein may cross-react with IL-36R proteins of another species (e.g., monkey) in certain circumstances. In other embodiments, an antibody specific for the human IL-36R protein may be completely specific for the human IL-36R protein and not cross-react with proteins of other species or other types, or may only cross-react with IL-36R proteins of some other species rather than all the other species.

The terms "human IL-36R" and "hIL-36R" and the like are interchangeable herein and refer to a protein with the amino acid sequence of human IL-36R, e.g. the human IL-36R amino acid sequence set forth in SEQ ID NO: 75. The terms "monkey IL-36R", "cynomolgus monkey IL-36R" and "CynoIL-36R" and the like are interchangeable herein and refer to a protein with the amino acid sequence of monkey IL-36R, e.g. the monkey IL-36R amino acid sequence set forth in SEQ ID NO: 78.

"Antibody" refers to a binding protein having at least one antigen (e.g., IL-36R) binding domain, and thus, the antibody of the present disclosure encompasses a polyclonal antibody, a monoclonal antibody or a fragment thereof and a monoclonal antibody variant or a fragment thereof, a multispecific antibody, a monospecific antibody, a monovalent antibody or a single chain antibody. The antibody of the present disclosure can be derived from any species including, but not limited to, mice, rats, rabbits, primates, llamas, and humans. The antibody of the present disclosure includes, but is not limited to, a mouse antibody, a chimeric antibody, a humanized antibody and a human antibody. Unless otherwise stated, the "antibody" of the present disclosure includes a full-length antibody and any antigen-binding portion (i.e., "antigen-binding fragment") or a single chain thereof. A conventional full-length antibody is a glycoprotein comprising two heavy (H) chains and two light (L) chains, wherein the heavy chains and the light chains are linked by disulfide bonds. Each of the heavy chains consists of a heavy chain variable region (VH) and a heavy chain constant region. The heavy chain constant region consists of three domains: CH1, CH2 and CH3. Each of the light chains consists of a light chain variable region (VL) and a light chain constant region. The light chain constant region consists of one domain CL. The VH and VL regions can further be divided into hypervariable regions, i.e., complementarity determining regions (CDRs), and framework regions (FRs) with relatively conserved sequences. Each VH and VL consists of three CDRs and four FRs, arranged from the amino-terminus to the carboxyl-terminus in the following order: FR1, CDR1, FR2, CDR2, FR3, CDR3, FR4. The variable regions of the antibody comprise binding domains that interact with antigens. The constant regions of the antibody can mediate the binding of immunoglobulins to host tissues or factors, including various cells of the immune system (e.g., effector cells) and the first component (C1q) of the classical complement system. Meanwhile, as is understood by those skilled in the art, a particular "full-length antibody", e.g., a nanobody, has only heavy (H) chains and no light (L) chains.

The "antigen-binding fragment" or "antigen-binding portion" of the antibody refers to one or more fragments of the antibody that retain the ability to specifically bind to an antigen (e.g., IL-36R). It has been demonstrated that the antigen-binding function of an antibody can be performed by fragments of a full-length antibody. Examples encompassed within the term "antigen-binding portion/fragment" of the antibody include: (i) Fab fragments: monovalent fragments consisting of the VL,VH, CL and CH1 domains; (ii) F(ab')$_2$ fragments, bivalent fragments comprising two Fab fragments linked by a disulfide bond in the hinge region; (iii) Fd fragments consisting of the VH and CH1 domains; (iv) Fv fragments consisting of the VL and VH domains of a single arm of the antibody; (v) dAb fragments consisting of the VH domain (see Ward et al., Nature. 341:544-546 (1989)); (vi) isolated complementarity determining regions (CDRs); and (vii) nanobodies, heavy chain variable regions comprising a single variable domain and two constant domains. Furthermore, although the two domains-VL and VH-of the Fv fragment are encoded by different genes, they can be recombinantly linked by a synthetic linker to form a single protein chain in which VL pairs with VH to form a monovalent molecule (referred to as single-chain Fv (scFv), see, e.g., Bird et al., Science. 242:423-426 (1988); Huston et al., Proc. Natl. Acad. Sci. 85:5879-5883 (1988)). These single-chain antibodies are also encompassed within the term "antigen-binding portion/fragment" of the antibody. Furthermore, a recombinant polypeptide and a fusion protein comprising the antigen-binding portion/fragment described above are also encompassed within the term antigen-binding portion/fragment. These antibody fragments can be obtained using conventional techniques known to those skilled in the art, and can be subjected to functional screening using the same method as full-length antibodies.

"Isolated antibody" refers to an antibody that is substantially free of other antibodies with different antigenic specificities (e.g., an isolated antibody that specifically binds to an IL-36R protein is substantially free of antibodies that specifically bind to antigens other than the IL-36R protein). However, an isolated antibody that specifically binds to human IL-36R protein may have cross-reactivity with other antigens (e.g., IL-36R proteins from other species). Furthermore, the isolated antibody is substantially free of other cellular components and/or chemical substances.

"Mouse antibody" or "murine antibody" refers to an antibody in which the framework regions and CDRs in the variable region are derived from mouse germline immunoglobulin sequences. Furthermore, if the antibody comprises a constant region, the constant region is also derived from mouse germline immunoglobulin sequences. The mouse antibody of the present disclosure may comprise amino acid residues not encoded by mouse germline immunoglobulin sequences (e.g., mutations introduced by *in vitro* random mutation or point mutation or by *in vivo* somatic mutation), but "mouse antibody" does not include antibodies in which CDR sequences derived from other mammalian species are inserted into a mouse framework sequence.

"Chimeric antibody" refers to an antibody formed by combining genetic materials of human origin and those of non-human origin. More generally, a chimeric antibody refers to an antibody comprising genetic materials from one species and those from another species. In the present disclosure, a chimeric antibody is also denoted as "xi". "Humanized antibody" refers to an antibody comprising complementarity determining regions (CDRs) derived from a non-human antibody, and framework and constant regions derived from a human antibody. For example, the humanized antibody that binds to IL-36R provided herein may comprise CDRs derived from one or more murine antibodies as well as human framework and constant regions. Thus, in some embodiments, the humanized antibody provided herein binds to the same epitope on IL-36R as the murine antibody from which the CDRs of the humanized antibody are derived. Exemplary humanized antibodies are provided herein. Additional humanized antibodies that bind to IL-36R or variants thereof comprising the heavy and light chain CDRs provided herein can be generated using any human framework sequences, and are also included in the present disclosure. In some embodiments, framework sequences suitable for use in the present disclosure include those similar in structure to the framework sequences provided herein. Additional modifications may be made in the framework regions to alter the properties of the antibodies provided herein. Such additional framework modifications may include chemical modifications, point mutations for reducing immunogenicity or removing T cell epitopes, or reversions to residues in original germline sequences. In some embodiments, such modifications include those corresponding to the mutations exemplified herein, including reversions in germline sequences. For example, in some embodiments, one or more amino acids in the human VH and/or VL framework regions of the humanized antibodies provided herein are reverted to the corresponding amino acids in the parent murine antibodies. In the present disclosure, humanized antibodies are also denoted as "hz".

"Isotype" refers to the class of antibodies encoded by the heavy chain constant region genes (e.g., IgM or IgG1). "...-recognizing antibody" and "...-specific antibody" are used interchangeably herein with the terms "antibody that specifically binds to ..." and "antibody that specifically binds ...".

An antibody that "specifically binds to human IL-36R" refers to one that binds to human IL-36R protein (or possibly other IL-36R proteins of non-human species) but does not substantially bind to non-IL-36R proteins. Preferably, the antibody binds to a human IL-36R with "high affinity", i.e., binds to a human IL-36R with a KD of $5.0 \times 10^{-8}$ M or less, preferably $1.0 \times 10^{-8}$ M or less, more preferably $5.0 \times 10^{-9}$ M or less.

The term "not substantially bind to" a protein or cell refers to not binding to the protein or cell, or not binding to it with high affinity, i.e., binding to the protein or cell with a KD of $1.0 \times 10^{-5}$ M or higher, preferably $1.0 \times 10^{-4}$ M or higher, more preferably $1.0 \times 10^{-3}$ M or higher, more preferably $1.0 \times 10^{-2}$ M or higher.

For IgG, the term "high affinity" refers to binding to an antigen with a KD of $1.0 \times 10^{-6}$ M or less, $1.0 \times 10^{-7}$ M or less, preferably $1.0 \times 10^{-8}$ M or less, more preferably $5.0 \times 10^{-9}$ M or less. However, for other antibody isotypes, "high affinity" binding may be different. For example, "high affinity" binding of IgM isotype refers to binding to an antigen with a KD of $1.0 \times 10^{-6}$ M or less, preferably $1.0 \times 10^{-7}$ M or less, more preferably $1.0 \times 10^{-8}$ M or less.

"Identity" refers to the similarity between two nucleic acid sequences or between two polypeptides. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. The sequence identity of the present disclosure is at least 80%, 85%, 90% or 95%, preferably at least 95%, and non-limiting examples include: 80%, 81%, 82%, 83%, 84%, 85%, 86%, 87%, 88%, 89%, 90%, 91%, 92%, 93%, 94%, 95%, 96%, 97%, 98%, 99%, and 100%. Alignment of amino acid sequences for identity can be performed in a variety of ways within the skill in the art, e.g., BLAST, BLAST-2, ALIGN or Megalign (DNASTAR) software. Those skilled in the art can determine suitable parameters for aligning sequences, including any algorithms required to obtain maximum alignment for the full length of sequences being compared.

The term "$EC_{50}$", also known as half maximal effective concentration, refers to the concentration of antibody that can cause 50% of the maximum effect after a specified exposure time.

The term "$IC_{50}$", also known as half maximal inhibitory concentration, refers to the concentration of an antibody that inhibits a specific biological or biochemical function by 50% relative to the case where the antibody is absent. The terms "inhibit" and "block" include partial and complete inhibition/blocking.

The term "subject" includes any human or non-human animal. The term "non-human animal" includes all vertebrates, e.g. mammals and non-mammals, preferably mammals, e.g., non-human primates, sheep, dogs, cats, cows and horses.

The term "therapeutically effective amount" refers to an amount sufficient to prevent or ameliorate symptoms associated with a disease or condition, preferably an amount that causes a reduction in the severity of the symptoms of the disease or an increase in the frequency and duration of asymptomatic phases, or prevents the damage or inability caused by the disease. The therapeutically effective amount is related to the disease to be treated, wherein the actual effective amount can be readily determined by those skilled in the art.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to $\pm$ 5%, for example, fluctuating within a particular numerical range given $\pm$ 2%, $\pm$ 1% or $\pm$ 0.5%. Where a particular value is given in the present disclosure or in the claims, unless otherwise stated, "about" should be considered to mean being within an acceptable error range for that particular value. In this context, unless otherwise indicated, the values of the parameters or conditions in a step are all modified by "about" by default.

The terms "$X_n$" and "Xaa" are equivalent and refer to an unspecified amino acid whose scope is specified by the subsequent definitions in the relevant description.

The term "include", "contain" or "comprise" and variants thereof should be understood as "including but not limited to", which means that other elements, components and steps that are not specified may also be encompassed in addition to the elements, components and steps that are listed.

The use of singular forms includes plural forms unless otherwise specified. Unless otherwise specified, the word "a" or "an" refers to "at least one". The phrase "at least one" means the same as "one or more", and "and/or" is used to mean "and" or "or", unless otherwise stated.

Aspects of the present disclosure are described in more detail below.

The anti-IL-36R antibody or the antigen-binding fragment thereof of the present disclosure binds to IL-36R (e.g., human IL-36R and monkey IL-36R) with high affinity. The anti-IL-36R antibody or the antigen-binding fragment thereof of the present disclosure: (a) binds to human IL-36R; (b) binds to monkey IL-36R; (c) blocks the binding of IL-36 to IL-36R; and/or (d) inhibits IL-36/IL-36R signaling (e.g., inhibits production of IL-8, IL-6, and/or GM-CSF), more specifically, inhibits IL-36 (hIL36$\alpha$, hIL36$\beta$, and hIL36$\gamma$)/IL-36R signal transduction and production of IL-8 in OVCAR8 cells and/or NHK cells.

Preferably, the anti-IL-36R antibody of the present disclosure is a humanized monoclonal antibody. Furthermore, the anti-IL-36R antibody of the present disclosure may be a mouse, human or chimeric monoclonal antibody. Exemplary antibodies or antigen-binding fragments thereof of the present disclosure are characterized structurally and chemically as described below and in the examples. The antibody or the antigen-binding fragment thereof comprises a heavy chain variable region (VH) comprising VH CDR1, VH CDR2 and VH CDR3 (i.e., heavy chain CDR1, heavy chain CDR2 and heavy chain CDR3), and a light chain variable region (VL) comprising VL CDR1, VL CDR2 and VL CDR3 (i.e., light chain CDR1, light chain CDR2 and light chain CDR3), wherein the amino acid sequence IDs (SEQ ID NOs.) of the exemplary heavy chain variable regions, light chain variable regions and CDRs are provided in Tables 1.1 and 1.2 below. Some antibodies have identical CDRs, and some antibodies have identical VHs or VLs. The heavy chain constant region of the antibody may be a human IgG1 heavy chain constant region comprising an amino acid sequence set forth in SEQ ID NO: 69, or a human IgG4 heavy chain constant region comprising an amino acid sequence set forth in SEQ ID NO: 71, or a variant having 1, 2, 3, 4, or 5 amino acid substitutions, deletions and additions compared to the amino acid sequence set forth in SEQ ID NO: 69 or 71. The light chain constant region of the antibody may be a human κ light chain constant region comprising an amino acid sequence set forth in SEQ ID NO: 73, or a variant having 1, 2, 3, 4, or 5 amino acid substitutions, deletions and additions compared to the amino acid sequence set forth in SEQ ID NO: 73. These antibodies may also comprise a mouse IgG1 or a mouse IgG4 heavy chain constant region and/or a mouse κ light chain constant region.

The amino acid sequences of the variable regions and CDRs of the exemplary antibodies or the antigen-binding fragments thereof of the present disclosure are shown in Table 1.1. However, as is well known in the art, CDRs or "complementarity determining regions" may also be determined by other numbering systems/methods, e.g., Kabat (see, Kabat et al., Sequences of Proteins of Immunological Interest, fifth edition, National Institutes of Health, Bethesda, Maryland (1991)), Chothia (see, Jmol Biol 273: 927-48, 1997), IMGT (Lefranc M.P., Immunologist, 7, 132-136 (1999); Lefranc, M.P. et al., Dev. Comp. Immunol., 27, 55-77 (2003)), AbM (defined by Oxford Molecular's AbM antibody modeling software 35), or Contact (based on analysis of available complex crystal structures) based on heavy/light chain variable region sequences, and CDRs may also be determined based on two or more combinations of Kabat, Chothia, IMGT, AbM and Contact definition rules. For example, CDRs shown in Table 1.2 are defined by the Kabat numbering system. It will be understood by those skilled in the art that, unless otherwise specified, the term "CDRs" of a given antibody or a region thereof (e.g., a variable region) will be understood to encompass CDRs defined by any one of the known schemes. Where reference is made to an antibody defined by a particular CDR sequence defined by certain divisions of the present disclosure, the scope of the antibody also encompasses antibodies defined by CDR sequences that are converted to other arbitrary numbering system definitions (e.g., a combination of one or more of Kabat, Chothia, IMGT, or Contact, etc. definitions well known in the art).

Table 1.1 Amino acid sequence IDs (SEQ ID NOs.) of variable regions and CDRs

| Antibody | VH CDR1 | VH CDR2 | VH CDR3 | VH | VL CDR1 | VL CDR2 | VL CDR3 | VL |
|---|---|---|---|---|---|---|---|---|
| Mouse/chimeric 72C1 | | 2, $X_1$=S | | 7 | | | 6, $X_2$=F | 15 |
| hz72C1-1.1 | | 2, $X_1$=S | | 9 | | | 6, $X_2$=F | |
| hz72C1-1.2 | | 2, $X_1$=S | | 11 | | | 6, $X_2$=F | 17 |
| hz72C1-1.3 | 1 | 2, $X_1$=Y | 3 | 13 | 4 | 5 | 6, $X_2$=F | |
| hz72C1-2.1 | | 2, $X_1$=S | | 9 | | | 6, $X_2$=Y | |
| hz72C1-2.2 | | 2, $X_1$=S | | 11 | | | 6, $X_2$=Y | 19 |
| hz72C1-2.3 | | 2, $X_1$=Y | | 13 | | | 6, $X_2$=Y | |
| Mouse/ chimeric 84C4 | 21, $X_3$=F | 22, $X_4$=M,$X_5$=H, $X_6$=L, $X_7$=N,$X_8$=K | | 27 | | | | 39 |
| hz84C4-1.1 | 21, $X_3$=F | 22, $X_4$=M,$X_5$=H, $X_6$=Y, $X_7$=A,$X_8$=Q | 23 | 29 | 24 | 25 | 26 | 41 |
| hz84C4-1.2 | 21, $X_3$=F | 22, $X_4$=Y,$X_5$=H, $X_6$=Y, $X_7$=A,$X_8$=Q | | 31 | | | | |

(continued)

| Antibody | VH CDR1 | VH CDR2 | VH CDR3 | VH | VL CDR1 | VL CDR2 | VL CDR3 | VL |
|---|---|---|---|---|---|---|---|---|
| hz84C4-1.3 | 21, $X_3$=F | 22, $X_4$=M,$X_5$=Y, $X_6$=Y, $X_7$=A,$X_8$=Q | | 33 | | | | |
| hz84C4-1.4 | 21, $X_3$=Y | 22, $X_4$=M,$X_5$=H, $X_6$=L, $X_7$=A,$X_8$=K | | 35 | | | | |
| hz84C4-1.5 | 21, $X_3$=F | 22, $X_4$=M,$X_5$=H, $X_6$=L, $X_7$=A,$X_8$=K | | 37 | | | | |
| hz84C4-2.1 | 21, $X_3$=F | 22, $X_4$=M,$X_5$=H, $X_6$=Y, $X_7$=A,$X_8$=Q | | 29 | | | | |
| hz84C4-2.2 | 21, $X_3$=F | 22, $X_4$=Y,$X_5$=H, $X_6$=Y, $X_7$=A,$X_8$=Q | | 31 | | | | |
| hz84C4-2.3 | 21, $X_3$=F | 22, $X_4$=M,$X_5$=Y, $X_6$=Y, $X_7$=A,$X_8$=Q | | 33 | | | | 43 |
| hz84C4-2.4 | 21, $X_3$=Y | 22, $X_4$=M,$X_5$=H, $X_6$=L, $X_7$=A,$X_8$=K | | 35 | | | | |
| hz84C4-2.5 | 21, $X_3$=F | 22, $X_4$=M,$X_5$=H, $X_6$=L, $X_7$=A,$X_8$=K | | 37 | | | | |
| Mouse/ chimeric 74B4D6 | 45 | 46 | 47 | 51 | 48 | 49 | 50 | 52 |
| Mouse/ chimeric 58A8D1 | 53 | 54 | 55 | 59 | 56 | 57 | 58 | 60 |
| Mouse/ chimeric 6G10 | 61 | 62 | 63 | 67 | 64 | 65 | 66 | 68 |

Table 1.2 Amino acid sequence IDs (SEQ ID NOs.) of CDRs defined by Kabat numbering system

| Antibody | VH CDR1 | VH CDR2 | VH CDR3 | VL CDR1 | VL CDR2 | VL CDR3 |
|---|---|---|---|---|---|---|
| Mouse/chimeric 72C1 | | 2, $X_1$=S | | | | 6, $X_2$=F |
| hz72C1-1.1 | | 2, $X_1$=S | | | | 6, $X_2$=F |
| hz72C1-1.2 | | 2, $X_1$=S | | | | 6, $X_2$=F |
| hz72C1-1.3 | 85 | 2, $X_1$=Y | 3 | 4 | 5 | 6, $X_2$=F |
| hz72C1-2.1 | | 2, $X_1$=S | | | | 6, $X_2$=Y |
| hz72C1-2.2 | | 2, $X_1$=S | | | | 6, $X_2$=Y |
| hz72C1-2.3 | | 2, $X_1$=Y | | | | 6, $X_2$=Y |

(continued)

| Antibody | VH CDR1 | VH CDR2 | VH CDR3 | VL CDR1 | VL CDR2 | VL CDR3 |
|---|---|---|---|---|---|---|
| Mouse/chimeric 84C4 | | 22, $X_4$=M,$X_5$=H, $X_6$=L, $X_7$=N,$X_8$=K | | | | |
| hz84C4-1.1 | | 22, $X_4$=M,$X_5$=H, $X_6$=Y, $X_7$=A,$X_8$=Q | | | | |
| hz84C4-1.2 | | 22, $X_4$=Y,$X_5$=H, $X_6$=Y, $X_7$=A,$X_8$=Q | | | | |
| hz84C4-1.3 | | 22, $X_4$=M,$X_5$=Y, $X_6$=Y, $X_7$=A,$X_8$=Q | | | | |
| hz84C4-1.4 | | 22, $X_4$=M,$X_5$=H, $X_6$=L, $X_7$=A,$X_8$=K | | | | |
| hz84C4-1.5 | 86 | 22, $X_4$=M,$X_5$=H, $X_6$=L, $X_7$=A,$X_8$=K | 23 | 87 | 25 | 26 |
| hz84C4-2.1 | | 22, $X_4$=M,$X_5$=H, $X_6$=Y, $X_7$=A,$X_8$=Q | | | | |
| hz84C4-2.2 | | 22, $X_4$=Y,$X_5$=H, $X_6$=Y, $X_7$=A,$X_8$=Q | | | | |
| hz84C4-2.3 | | 22, $X_4$=M,$X_5$=Y, $X_6$=Y, $X_7$=A,$X_8$=Q | | | | |
| hz84C4-2.4 | | 22, $X_4$=M,$X_5$=H, $X_6$=L, $X_7$=A,$X_8$=K | | | | |
| hz84C4-2.5 | | 22, $X_4$=M,$X_5$=H, $X_6$=L, $X_7$=A,$X_8$=K | | | | |
| Mouse/chimeric 74B4D6 | 88 | 46 | 47 | 48 | 49 | 50 |
| Mouse/chimeric 58A8D1 | 89 | 54 | 55 | 56 | 57 | 58 |
| Mouse/chimeric 6G10 | 90 | 62 | 63 | 64 | 65 | 66 |

The VH and/or VL sequences (or CDR sequences) of other anti-IL-36R antibodies that bind to human IL36R can be "mixed and paired" with the VH and/or VL sequences (or CDR sequences) of the anti-IL-36R antibody of the present disclosure. Preferably, when VH and VL chains (or CDRs thereof) are mixed and paired, the VH sequence in a particular VH/VL pair can be substituted with a structurally similar VH sequence. Likewise, preferably, the VL sequence in a particular VH/VL pair can be substituted with a structurally similar VL sequence.

Therefore, in one embodiment, the antibody or the antigen-binding fragment thereof disclosed herein comprises:

(a) a heavy chain variable region comprising an amino acid sequence listed in Table 1.1, and
(b) a light chain variable region comprising an amino acid sequence listed in Table 1.1, or the VL of another anti-IL-36R antibody; wherein the antibody or the antigen-binding fragment thereof specifically binds to human IL36R.

In another embodiment, the antibody or the antigen-binding fragment thereof of the present disclosure comprises:

(a) VH CDR1, VH CDR2 and VH CDR3 listed in Table 1.1 or Table 1.2, and
(b) VL CDR1, VL CDR2, and VL CDR3 listed in Table 1.1 or Table 1.2, or CDRs of a light chain variable region of another anti-IL-36R antibody, wherein the antibody or the antigen-binding fragment thereof specifically binds to human IL36R. In another embodiment, the antibody or the antigen-binding fragment thereof of the present disclosure comprises CDR2 of the heavy chain variable region of the anti-IL-36R antibody of the present disclosure as well as other CDRs that bind to a human IL-36R antibody, e.g., CDR1 and/or CDR3 of the heavy chain variable region of another anti-IL-36R antibody, and/or CDR1, CDR2 and/or CDR3 of the light chain variable region of another anti-IL-36R antibody, wherein the antibody or the antigen-binding fragment thereof specifically binds to human IL36R.

Furthermore, it is well known in the art that, independent of the CDR1 and/or CDR2 domains, the CDR3 domains can individually determine the binding specificity of an antibody to an alloantigen, and that multiple antibodies with the same binding specificity can be predictively generated based on a common CDR3 sequence. In one embodiment, the antibody or the antigen-binding fragment thereof of the present disclosure comprises CDR3 of the heavy and/or light chain variable region of the anti-IL-36R antibody of the present disclosure and other CDRs that bind to a human IL-36R antibody, e.g., CDR1 and/or CDR2 of the heavy chain variable region of another anti-IL-36R antibody, and/or CDR1 and/or CDR2 of the light chain variable region of another anti-IL-36R antibody, wherein the antibody or the antigen-binding fragment thereof specifically binds to human IL36R. In another embodiment, the antibody or the antigen-binding fragment thereof of the present disclosure comprises CDR2 of the heavy chain variable region of the anti-IL-36R antibody of the present disclosure, and CDR3 of the heavy and/or light chain variable region of the anti-IL-36R antibody of the present disclosure or CDR3 of the heavy and/or light chain variable region of another anti-IL-36R antibody, wherein the antibody or the antigen-binding fragment thereof specifically binds to human IL-36R. Preferably, these antibodies (a) compete with the anti-IL-36R antibody of the present disclosure for binding to IL36R; (b) and the anti-IL-36R antibody of the present disclosure retain functional features; (c) and the anti-IL-36R antibody of the present disclosure bind to the same epitope; and/or (d) and the anti-IL-36R antibody of the present disclosure have similar binding affinity. In another embodiment, the antibody or the antigen-binding fragment thereof of the present disclosure further comprises CDR2 of the light chain variable region of the anti-IL-36R antibody of the present disclosure or CDR2 of the light chain variable region of another anti-IL-36R antibody, wherein the antibody or the antigen-binding fragment thereof specifically binds to human IL36R. In another embodiment, the antibody or the antigen-binding fragment thereof of the present disclosure further comprises CDR1 of the heavy and/or light chain variable region of the anti-IL-36R antibody of the present disclosure or CDR1 of the heavy and/or light chain variable region of another anti-IL-36R antibody, wherein the antibody or the antigen-binding fragment thereof specifically binds to human IL36R.

In another embodiment, the antibody or the antigen-binding fragment thereof of the present disclosure comprises CDR1, CDR2, and CDR3 sequences that are different from the heavy and/or light chain variable region of the anti-IL-36R antibody of the present disclosure, the difference being derived from one or more conservative modifications. It is understood in the art that some conservative sequence modifications do not eliminate the antigen-binding ability.

Therefore, in one embodiment, the antibody or the antigen-binding fragment thereof of the present disclosure comprises a heavy chain variable region and/or a light chain variable region, the heavy chain variable region and the light chain variable region respectively comprising CDR1, CDR2 and CDR3, wherein:

(a) the CDR1 sequence of the heavy chain variable region comprises the sequences listed in Table 1.1 or Table 1.2, and/or conservative modifications thereof; and/or

(a) the CDR2 sequence of the heavy chain variable region comprises the sequences listed in Table 1.1 or Table 1.2, and/or conservative modifications thereof; and/or

(c) the CDR3 sequence of the heavy chain variable region comprises the sequences listed in Table 1.1 or Table 1.2, and/or conservative modifications thereof; and/or

(d) the CDR1 and/or CDR2 and/or CDR3 sequences of the light chain variable region comprise the sequences listed in Table 1.1 or Table 1.2; and/or conservative modifications thereof; and

(e) the antibody specifically binds to human IL36R.

The term "conservative sequence modification" refers to an amino acid modification that does not significantly affect or alter the binding properties of the antibody. Such conservative modifications include amino acid substitutions, additions and deletions. Modifications can be introduced into the antibody disclosed herein using standard techniques known in the art, e.g., point mutation and PCR-mediated mutation. Conservative amino acid substitutions are those in which an amino acid residue is replaced with an amino acid residue having a similar side chain. Amino acid residue groups having similar side chains are known in the art. These amino acid residue groups include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Therefore, one or more amino acid residues in the CDRs of the antibody of the present disclosure can be substituted with other amino acid residues of the same side chain group, and the resulting antibody can be tested for retained functions using the functional assays described herein.

The antibody of the present disclosure can be engineered to produce modified antibodies using antibodies having one or more VH/VL sequences of the anti-IL-36R antibody of the present disclosure as starting materials. One or more residues within one or both of the variable regions (i.e., VH and/or VL) (e.g., within one or more CDRs and/or one or more framework regions) of the antibody can be engineered and modified. Furthermore or alternatively, residues in the constant region can be modified, for example, to alter the effector function of the antibody.

In certain embodiments, CDR grafting can be used to modify the variable regions of the antibody. The antibody interacts with the target antigen mainly through amino acid residues in the six complementarity determining regions (CDRs) of the heavy chain and the light chain. Therefore, the amino acid sequences within the CDRs are more diverse than the sequences outside the CDRs among various antibodies. Since the CDR sequences are responsible for the major antibody-antigen interactions, such expression vectors in which the CDR sequences of particular naturally occurring antibodies are grafted into the framework sequences of another antibody with different properties can be constructed to express recombinant antibodies simulating the properties of the particular naturally occurring antibodies (see, e.g., Riechmann et al., (1998) Nature 332: 323-327; Jones et al., (1986) Nature 321: 522-525; Queen et al., (1989) Proc. Natl. Acad. Also see U.S.A. 86:10029-10033; U.S. Pat. 5,225,539; 5,530,101; 5,585,089; 5,693,762 and 6,180,370).

Accordingly, another embodiment of the present disclosure relates to an isolated monoclonal antibody or an antigen-binding fragment thereof, which comprises a heavy chain variable region comprising heavy chain CDR1, heavy chain CDR2 and heavy chain CDR3 described above in the present disclosure and/or a light chain variable region comprising light chain CDR1, light chain CDR2 and light chain CDR3 described above in the present disclosure. Although these antibodies comprise the CDR sequences of the VH and VL of the monoclonal antibody of the present disclosure, they may comprise different framework sequences.

Such framework sequences can be found in public DNA databases or references including antibody gene sequences. For example, DNA sequences for human heavy chain variable region and light chain variable region genes can be obtained from the Vbase human germline sequence database (www.mrc-cpe.cam.ac.uk/vbase) and documents such as Kabat et al, (1991), supra; Tomlinson et al, (1992) J. Mol. Biol. 227: 776-798; and Cox et al., (1994) Eur. J. Immunol. 24: 827-836, the content of which is expressly incorporated herein by reference. In another embodiment, DNA sequences for human heavy chain variable region and human light chain variable region genes can be obtained from the Genbank database. Antibody protein sequences were compared to a database of compiled protein sequences using the sequence similarity search methods of Gapped BLAST (Altschul et al, (1997), supra) that are well known to those skilled in the art.

The antibody framework sequences used in the present disclosure are preferably those that are structurally similar to the antibody framework sequences of the present disclosure. The VH CDR1, VH CDR2 and VH CDR3 sequences can be grafted into a framework region that comprises the same sequence as the germline immunoglobulin gene from which the framework sequences were derived, or the CDR sequences can be grafted into a framework region that comprises one or more mutations compared to the germline sequence. For example, in some cases, it may be beneficial to mutate residues in the framework region; such mutations can maintain or enhance the antigen-binding ability of the antibody (see, e.g., U.S. Pat. 5,530,101; 5,585,089; 5,693,762 and 6,180,370).

Another type of variable region modification is to mutate amino acid residues within CDR1, CDR2 and/or CDR3 of the VH and/or VL to improve one or more binding properties (e.g., affinity) of the target antibody. Mutations can be introduced by point mutations or PCR-mediated mutations, and the effect of the mutations on antibody binding or other functional properties can be assessed through *in vitro* or *in vivo* assays known in the art. Preferably, conservative modifications known in the art are introduced. Mutations may be amino acid substitutions, additions or deletions, preferably substitutions. Furthermore, typically no more than one, two, three, four or five residues within each CDR are altered.

Furthermore, in another embodiment, the present disclosure provides an isolated anti-IL-36R monoclonal antibody or an antigen-binding fragment thereof comprising a heavy chain variable region and a light chain variable region, wherein the heavy chain variable region and the light chain variable region comprise:

(a) a VH CDR1 comprising the VH CDR1 sequence of the present disclosure or an amino acid sequence having one, two, three, four or five amino acid substitutions, deletions or additions;
(b) a VH CDR2 comprising the VH CDR2 sequence of the present disclosure or an amino acid sequence having one, two, three, four or five amino acid substitutions, deletions or additions;
(c) a VH CDR3 comprising the VH CDR3 sequence of the present disclosure or an amino acid sequence having one, two, three, four or five amino acid substitutions, deletions or additions;
(d) a VL CDR1 comprising the VL CDR1 sequence of the present disclosure or an amino acid sequence having one, two, three, four or five amino acid substitutions, deletions or additions;
(e) a VL CDR2 comprising the VL CDR2 sequence of the present disclosure or an amino acid sequence having one, two, three, four or five amino acid substitutions, deletions or additions; and

(f) a VL CDR3 comprising the VL CDR3 sequence of the present disclosure or an amino acid sequence having one, two, three, four or five amino acid substitutions, deletions or additions.

The engineered antibodies of the present disclosure include those antibodies in which the framework region residues of the VH and/or the VL were modified, for example, to alter the properties of the antibody. In general, such framework region modifications can be used to reduce the immunogenicity of the antibody. For example, one or more framework region residues are "reverted" to the corresponding germline sequence. More specifically, antibodies undergoing somatic mutation may contain framework residues that differ from the resulting antibody germline sequence. These residues can be identified by comparing the antibody framework sequence to the germline sequence of the resulting antibody.

Another type of framework region modification includes mutating one or more residues of the framework region or even one or more CDRs to remove T cell epitopes, thereby reducing the immunogenicity that an antibody may produce. This method is also known as "deimmunization" and is described in more detail in U.S. patent publication 20030153043.

Furthermore, as an alternative to the framework region or CDR modifications, the Fc region of the antibody of the present disclosure may be modified, typically to alter one or more functional properties of the antibody, e.g., serum half-life, complement fixation, Fc receptor binding, and/or antibody-dependent cytotoxicity. Furthermore, the antibody of the present disclosure may also be chemically modified (e.g., one or more chemical functional groups may be linked to the antibody), or modified to alter its glycosylation, to alter one or more functional properties of the antibody.

In one embodiment, the hinge region is modified to alter (for example, increasing or decreasing) the number of cysteine residues in the hinge region. This method is described in detail in U.S. Pat. 5,677,425. Altering the number of cysteine residues in the hinge region can, for example, facilitate the assembly of the heavy chain and the light chain, or improve or lower the stability of the antibody.

In another embodiment, the Fc-hinge region of the antibody is mutated to increase or decrease the biological half-life of the antibody. More specifically, one or more amino acid mutations are introduced into the CH2-CH3 junction region of the Fc-hinge fragment such that the antibody has reduced staphylococcal protein A (SpA) binding force compared to the antibody with the natural Fc-hinge domain. This method is described in more detail in U.S. Pat. 6,165,745.

In another embodiment, the glycosylation of the antibody is altered. For example, deglycosylated antibodies (i.e., antibodies lack glycosylation) is prepared. Such glycosylation modifications can be achieved, for example, by altering one or more glycosylation sites within the antibody sequence. For example, one or more amino acid replacements can be made to eliminate glycosylation sites in framework regions of one or more variable regions and thus the glycosylation at those sites. Such deglycosylation can increase the affinity of the antibody for the antigen. See, e.g., U.S. Pat. 5,714,350 and 6,350,861.

Furthermore, antibodies with altered glycosylation, e.g., low-fucosylated antibodies with reduced fucose residues, or antibodies with increased bisecting GlcNac structures, can be prepared. It has been demonstrated that altered glycosylation can increase the ADCC activity of the antibody. Such sugar modification can be achieved, for example, by expressing the antibody in a host cell with altered glycosylation mechanism. Cells with altered glycosylation mechanism are known in the art and can be used as host cells for expressing the recombinant antibodies of the present disclosure to prepare antibodies with altered glycosylation.

Another type of modification for the antibody is PEGylation. PEGylation of an antibody can increase the biological (e.g., serum) half-life of the antibody. To obtain a PEGylated antibody, an antibody or a fragment thereof is reacted with polyethylene glycol (PEG), e.g., a reactive ester or aldehyde derivative of PEG, under such conditions that one or more PEG groups are attached to the antibody or the antibody fragment. Preferably, the PEGylation is performed by acylation or alkylation with a reactive PEG molecule (or a similar reactive water-soluble polymer). The term "polyethylene glycol" includes any form of PEG for derivatize other proteins, e.g., mono(C1-C10)alkoxy polyethylene glycol or aryloxy polyethylene glycol or polyethylene glycol-maleimide. Methods of PEGylation of proteins are known in the art and can be applied to the antibody of the present disclosure. See, e.g., EP0154316 and EP0401384.

Each antibody has a unique isoelectric point (pI), typically within the pH range of 6-9.5. The pI of IgG1 antibodies is typically within the pH range of 7-9.5, while that of IgG4 antibodies is substantially within the pH range of 6-8. It is speculated that antibodies with pI outside the normal range may undergo some unfolding and be unstable *in vivo*. Therefore, anti-IL-36R antibodies having a pI value within the normal range are preferred. This can be achieved by selecting antibodies with the pI within the normal range or by mutating charged surface residues.

In another aspect, the present disclosure provides a nucleic acid molecule encoding the heavy chain and/or light chain variable regions or CDRs of the antibody of the present disclosure. The nucleic acid molecule may be present in intact cells, in cell lysates, or in partially purified or substantially pure form. The nucleic acid molecule is "isolated" or "substantially pure" when purified from other cellular components or other contaminants, e.g., from other cellular nucleic acids or proteins, by using standard techniques. The nucleic acid molecule of the present disclosure may be DNA or RNA, and may or may not comprise intron sequences. In preferred embodiments, the nucleic acid molecule is a cDNA molecule.

The nucleic acid of the present disclosure can be obtained using standard molecular biology techniques. For antibodies expressed by hybridomas, light chain and heavy chain cDNAs encoding the antibodies prepared from the hybridomas can be obtained by standard PCR amplification or using cDNA cloning techniques. For antibodies obtained from immunoglobulin gene libraries (e.g., using phage display technology), nucleic acids encoding such antibodies can be recovered from the gene libraries.

Preferably, the nucleic acid molecule of the present disclosure includes those encoding the VH and VL sequences or CDRs of the anti-IL-36R monoclonal antibody of the present disclosure. Once the DNA fragments encoding the VH and the VL are obtained, further operations, e.g., converting the variable region genes into full-length antibody chain genes, Fab fragment genes or scFv genes, can be conducted on these DNA fragments using standard recombinant DNA techniques. In these operations, the DNA fragments encoding the VH and the VL are operably linked to DNA fragments encoding another protein (e.g., an antibody constant region or a flexible linker). The term "operably linked" as used in this case means that two DNA fragments are joined together such that the amino acid sequences encoded by the two DNA fragments are in the reading frame.

Isolated DNA encoding the VH can be converted into a full-length heavy chain gene by operably linking the DNA encoding the VH to another DNA molecule encoding the heavy chain constant region (CH1, CH2 and CH3). The sequences of the human heavy chain constant region genes are known in the art, and a DNA fragment comprising the human heavy chain constant region gene can be obtained by standard PCR amplification. The heavy chain constant region may be an IgG1, IgG2, IgG3, IgG4, IgA, IgE, IgM or IgD constant region, but is preferably an IgG1 or IgG4 constant region. For the Fab fragment heavy chain gene, the DNA encoding the VH can be operably linked to another DNA molecule encoding only the heavy chain CH1 constant region.

Isolated DNA encoding the VL can be converted into a full-length light chain gene by operably linking the DNA encoding the VL to another DNA molecule encoding the light chain constant region CL. The sequences of the human light chain constant region genes are known in the art, and a DNA fragment comprising the human light chain constant region gene can be obtained by standard PCR amplification. In preferred embodiments, the light chain constant region may be a κ or λ constant region.

To prepare an scFv gene, a DNA fragment encoding the VH and the VL is operably linked to another fragment encoding a flexible linker, for example, another fragment encoding the amino acid sequence (Gly4-Ser)3, such that the VH and VL sequences can be expressed as a continuous single-chain protein in which the VL and VH regions are linked by the flexible linker (see, e.g., Bird et al., (1988) Science 242:423-426; Huston et al., (1988) Proc. Natl. Acad. Sci. USA 85:5879-5883; McCafferty et al., (1990) Nature 348:552-554).

The monoclonal antibody (mAb) of the present disclosure can be prepared using the somatic hybridization (hybridoma) technique well known in the art in Kohler and Milstein (1975) Nature 256: 495. Other embodiments for preparing the monoclonal antibody include viral or oncogenic transformation of B lymphocytes and phage display techniques. Chimeric or humanized antibodies are also well known in the art. See, e.g., U.S. Pat. 4,816,567; 5,225,539; 5,530,101; 5,585,089; 5,693,762 and 6,180,370, the content of each of which is expressly incorporated herein by reference.

The antibody of the present disclosure may also be prepared in host cell transfectomas using, for example, recombinant DNA techniques in combination with gene transfection methods (e.g., Morrison, S. (1985) Science 229:1202). In one embodiment, DNA encoding partial or full-length light and heavy chains obtained using standard molecular biotechnology is inserted into one or more expression vectors such that the gene is operably linked to transcriptional and translational regulatory sequences. In this case, the term "operably linked" refers to linking the antibody gene into the vector such that the transcriptional and translational regulatory sequences in the vectors perform their intended functions of regulating the transcription and translation of the antibody gene.

The term "regulatory sequence" includes promoters, enhancers and other expression control elements (e.g., polyadenylation signals) that control the transcription or translation of the antibody gene. Such regulatory sequences are described, for example, in Goeddel (Gene Expression Technology. Methods in Enzymology 185, Academic Press, San Diego, Calif. (1990)). Preferably, regulatory sequences for expression in a mammalian host cell include viral elements that direct the high-level protein expression in mammalian cells, e.g., promoters and/or enhancers derived from cytomegalovirus (CMV), simian virus 40 (SV40), adenoviruses (e.g., the adenovirus major late promoter (AdMLP)) and polyomavirus. Alternatively, non-viral regulatory sequences such as ubiquitin promoters or β-globin promoters can be used. In addition, the regulatory elements are composed of sequences of different origins, such as the SRα promoter system, which comprises the sequence from the SV40 early promoter and the sequence of the long terminal repeat of the human T-cell leukemia type I virus (Takebe et al., (1988) Mol. Cell. Biol. 8:466-472). The expression vector and expression regulatory sequences compatible with the expression host cell used are selected.

The antibody light chain gene and the antibody heavy chain gene can be inserted into the same expression vector or different expression vectors. In preferred embodiments, variable regions are inserted into an expression vector that has encoded the heavy chain constant region and the light chain constant region of the desired isotype to construct a full-length antibody gene, such that the VH is operably linked to the CH in the vector, and the VL is operably linked to the CL in the vector. Furthermore, the recombinant expression vector can encode a signal peptide that facilitates the secretion of antibody chains from the host cell. The antibody chain gene can be cloned into a vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vector of the present disclosure may carry other sequences, e.g., a sequence that regulates replication of the vector in the host cell (e.g., an origin of replication) and a selectable marker gene.

To express the light chain and the heavy chain, host cells are transfected with expression vectors encoding the heavy chain and the light chain using standard techniques. Different forms of "transfect" encompasses a variety of techniques for introducing exogenous DNA into prokaryotic or eukaryotic host cells commonly, e.g., electroporation, calcium phosphate precipitation, DEAE-dextran transfection, etc. Although expressing the antibody of the present disclosure in prokaryotic or eukaryotic host cells is theoretically feasible, expressing the antibody in eukaryotic cells is preferred, and expressing the antibody in mammalian host cells is most preferred. This is because eukaryotic cells, particularly mammalian cells, are more likely to assemble and secrete properly folded and immunologically active antibodies than prokaryotic cells. Preferred mammalian host cells for expressing the recombinant antibody of the present disclosure include Chinese hamster ovary cells (CHO cells) (including dhfr-CHO cells used with a DHFR selectable marker, as described in Urlaub and Chasin, (1980) Proc. Natl. Acad. Sci. USA 77:4216-4220, the DHFR selectable marker is described, for example, in R. J. Kaufman and P. A. Sharp (1982) J. Mol. Biol. 159:601-621), NSO myeloma cells, COS cells and SP2 cells. Another preferred expression system, particularly when NSO myeloma cells are used, is the GS gene expression system recited in WO87/04462, WO89/01036 and EP338,841. When a recombinant expression vector encoding an antibody gene is introduced into a mammalian host cell, the antibody is prepared by culturing the host cell for a period of time sufficient to allow the expression of the antibody in the host cell, or preferably, sufficient to allow the secretion of the antibody into the medium in which the host cell grows. The antibody can be recovered from the culture medium using standard protein purification methods.

In another aspect, the present disclosure provides a pharmaceutical composition comprising one or more of the anti-IL-36R antibody or the antigen-binding fragment thereof, the multispecific molecule, the immunoconjugate, the CAR-T cell, the gene vector or the nucleic acid molecule of the present disclosure, and a pharmaceutically acceptable excipient, diluent, or carrier. In some embodiments, the pharmaceutical composition may optionally comprise one or more other pharmaceutically active ingredients, e.g., another antibody (e.g., an anti-IgE antibody) or drug.

The pharmaceutical composition of the present disclosure is suitable for intravenous, intramuscular, subcutaneous, parenteral, spinal or epidermal administration (e.g., by injection or infusion). Depending on the route of administration, the active ingredient may be encapsulated in a material to protect it from acids and other natural conditions that may inactivate it. The phrase "parenteral administration" used herein refers to modes of administration other than enteral and topical administration that are typically performed by injection, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, the pharmaceutical composition of the present disclosure may be administered through a non-parenteral route, for example, topical, epidermal or mucosal route, such as intranasal, oral, vaginal, rectal, sublingual or topical administration.

The pharmaceutical composition may be in the form of a sterile aqueous solution or a dispersion. They may also be formulated in microemulsions, liposomes or other ordered structures suitable for high concentrations of drugs. The administration regimen is adjusted to provide the best desired response (e.g., therapeutic response). For example, a single large dose may be administered, multiple split doses may be administered over time, or the dose may be reduced or increased in proportion to the criticality of the treatment situation. It is particularly advantageous to formulate parenteral compositions in dosage unit forms for ease of administration and uniformity of dosage. Dosage unit forms used herein refer to physically discrete units of a single dose suitable for a subject to be treated, each unit comprising a predetermined quantity of active ingredient calculated to produce the desired therapeutic effect in association with the required pharmaceutical carrier. Alternatively, the antibody may be administered as a sustained-release formulation, in which case the frequency of administration required is reduced.

In the administration of the antibody, the dose may be in the range of 0.0001 to 100 mg/kg of host body weight. The anti-IL-36R antibody or the antigen-binding fragment thereof of the present disclosure has a variety of *in vitro* and *in vivo* applications relating to the diagnosis, treatment and/or prevention of IL-36/IL-36R-mediated related diseases and conditions. The anti-IL-36R antibody or the antigen-binding fragment thereof, the pharmaceutical composition, the multispecific molecule, the immunoconjugate, the CAR-T cell, the gene vector, or the nucleic acid molecule of the present disclosure may be administered to a human subject to prevent, alleviate, ameliorate or treat the IL-36/IL-36R-mediated related diseases and conditions.

In another aspect, the present disclosure provides a method for inhibiting IL-36/IL-36R signal transduction in a subject, comprising administering to the subject a therapeutically effective amount of the anti-IL-36R antibody or the antigen-binding fragment thereof, the pharmaceutical composition, the multispecific molecule, the immunoconjugate, the CAR-T cell, the gene vector, or the nucleic acid molecule of the present disclosure. In some embodiments, the subject is a human. In some embodiments, the subject has an autoimmune disease, an inflammatory disease, a respiratory disease, a metabolic disorder, or cancer.

In another aspect, the present disclosure provides a method for treating or preventing IL-36/IL-36R-mediated related diseases and conditions in a subject, comprising administering to the subject a therapeutically effective amount of the anti-IL-36R antibody or the antigen-binding fragment thereof, the pharmaceutical composition, the multispecific molecule, the immunoconjugate, the CAR-T cell, the gene vector, or the nucleic acid molecule of the present disclosure. In some embodiments, the subject is a human.

In some specific embodiments, in the uses and methods, the IL-36/IL-36R-mediated related diseases and conditions include autoimmune diseases, inflammatory diseases, respiratory diseases, metabolic disorders, cancer, or the like. In some specific embodiments, in the uses and methods, the IL-36/IL-36R-mediated related diseases and conditions include psoriasis, palmoplantar pustulosis, atopic dermatitis, inflammatory bowel disease, nephritis, hidradenitis suppurativa and/or rheumatoid arthritis, etc. In some specific embodiments, in the uses and methods, the IL-36/IL-36R-mediated related diseases and conditions include cancer, e.g., a solid tumor or a non-solid tumor.

In another aspect, the present disclosure provides a combination therapy in which the anti-IL-36R antibody or the antigen-binding fragment thereof of the present disclosure is co-administered with one or more other antibodies or drugs capable of preventing, alleviating, ameliorating or treating IL-36/IL-36R-mediated related diseases and conditions. In some embodiments, the IL-36/IL-36R-mediated related diseases and conditions include autoimmune diseases, inflammatory diseases, respiratory diseases, metabolic disorders, cancer, or the like.

In some specific embodiments, the IL-36/IL-36R-mediated related diseases and conditions include psoriasis, palmoplantar pustulosis, atopic dermatitis, inflammatory bowel disease, nephritis, hidradenitis suppurativa and/or rheumatoid arthritis, etc. In some specific embodiments, the present disclosure provides a method for treating or preventing psoriasis, palmoplantar pustulosis, atopic dermatitis, inflammatory bowel disease, nephritis, hidradenitis suppurativa and/or rheumatoid arthritis in a subject, comprising administering to the subject the anti-IL-36R antibody or the antigen-binding fragment thereof of the present disclosure and one or more additional antibodies, e.g., an IgE antibody, an anti-IL4 antibody, an anti-IL13 antibody, and/or an anti-IL5 antibody. In some specific embodiments, the present disclosure provides a method for treating or preventing psoriasis, palmoplantar pustulosis, atopic dermatitis, inflammatory bowel disease, nephritis, hidradenitis suppurativa and/or rheumatoid arthritis in a subject, comprising administering to the subject the anti-IL-36R antibody or the antigen-binding fragment thereof of the present disclosure and one or more additional drugs, e.g., an antiallergic drug. Other therapies that can be combined with the anti-IL-36R antibody or the antigen-binding fragment thereof of the present disclosure also include: reducing or avoiding allergen exposure, hormone therapy, surgery, etc. In some embodiments, the subject is a human.

In some specific embodiments, the IL-36/IL-36R-mediated related diseases and conditions include cancer, e.g., a solid tumor or a non-solid tumor. In some specific embodiments, the present disclosure provides a method for treating or preventing cancer in a subject, comprising administering to the subject the anti-IL-36R antibody or the antigen-binding fragment thereof of the present disclosure and one or more additional antibodies, e.g., an anti-PD-1 antibody, an anti-PD-L1 antibody, and/or an anti-CTLA-4 antibody. In some specific embodiments, the present disclosure provides a method for treating or preventing cancer in a subject, comprising administering to the subject the anti-IL-36R antibody or the antigen-binding fragment thereof of the present disclosure and one or more additional drugs, e.g., a small-molecule anti-cancer agent, e.g., a chemotherapeutic agent. Other therapies that can be combined with the anti-IL-36R antibody or the antigen-binding fragment thereof of the present disclosure also include: surgery, radiation therapy, etc. In some embodiments, the subject is a human.

An autoimmune disease refers to any disease, disorder or condition of the body that attacks and damages its tissues caused by excessive activation of the immune system, for example, multiple sclerosis, scleroderma, asthma, hidradenitis suppurativa, arthritis (including rheumatoid arthritis and psoriatic arthritis), ankylosing spondylitis, psoriasis (including psoriasis vulgaris, pustular psoriasis, e.g., local pustular psoriasis, generalized pustular psoriasis (GPP)), palmoplantar pustulosis (PPP), systemic lupus erythematosus (SLE), Crohn's disease, and ulcerative colitis.

An inflammatory disease refers to any disease, disorder or condition of excessive inflammatory symptoms, host tissue damage or loss of function due to excessive or uncontrolled inflammatory responses, including allergic inflammation of the skin, kidney, lung, gastrointestinal tract and respiratory tract, e.g., atopic dermatitis, acne vulgaris, skin rashes, allergic rhinitis, nephritis, inflammatory bowel disease (including Crohn's disease and ulcerative colitis), food allergies and other allergies.

Examples of respiratory diseases that can be treated by the method of the present disclosure include, but are not limited to: asthma and chronic obstructive pulmonary disease (COPD).

Cancer refers to a wide variety of diseases characterized by the uncontrolled growth of abnormal cells in the body. "Cancer" or "cancer tissue" may include a tumor. Cancers that can be treated by the method of the present disclosure include, but are not limited to: carcinomas, sarcomas, lymphomas, blastomas, and leukemias.

The combination of therapeutic agents discussed herein can be administered simultaneously as a single composition in a pharmaceutically acceptable carrier, or administered simultaneously as separate compositions, wherein each agent is in a pharmaceutically acceptable carrier. In another embodiment, the combination of therapeutic agents may be administered sequentially.

Furthermore, if the combination therapy is administered multiple times and the agents are administered sequentially, the sequence in the sequential administration at each time point can be reversed or maintained, and the sequential administration can be combined with simultaneous administration or any combination thereof. Although the foregoing invention has been described in detail by providing illustrations and examples for the purpose of clear understanding, it will be apparent to those of ordinary skill in the art in light of the teachings of the present disclosure that certain changes and modifications can be made to the present disclosure without departing from the spirit or scope of the appended claims. The present disclosure is further explained by the description of the following examples, which are not intended to be limiting. Those skilled in the art will readily identify a variety of noncritical parameters that may be changed or modified to produce substantially similar results.

Unless otherwise indicated, the practice of the present disclosure will use conventional methods in protein chemistry, biochemistry, the recombinant DNA technique, and pharmacology within the art.

**Example**

Example 1. Preparation of Test Protein

hIL36R-ECD mFc recombinant protein (SEQ ID NO: 76) consisted of an hIL-36R extracellular region and a mouse antibody heavy chain Fc, hIL36R-ECD hFc recombinant protein (SEQ ID NO: 77) consisted of an hIL-36R extracellular region and a human antibody heavy chain Fc, CynoIL36R-ECD mFc recombinant protein (SEQ ID NO: 79) consisted of a cynomolgus IL-36R extracellular region and a mouse antibody heavy chain Fc, and CynoIL36R-ECD hFc recombinant protein (SEQ ID NO: 80) consisted of a cynomolgus IL-36R extracellular region and a human antibody heavy chain Fc. cDNAs encoding the recombinant proteins described above were obtained by gene synthesis and subcloned into expression vectors pcDNA3.1 (Invitrogen V-790), respectively, and the expression vectors were transfected into ExpiCHO cells for transient expression. After 7 days of ExpiCHO cell culture, hIL36R-ECD mFc, CynoIL36R-ECD mFc, hIL36R-ECD hFc and CynoIL36R-ECD hFc recombinant proteins in cell culture supernatants were purified by Protein A column (GE healthcare) respectively for later-stage screening detection.

Example 2. Preparation of Hybridoma Anti-IL-36R Monoclonal Antibodies

Human IL-1Rrp2/IL-1R6 protein, His Tag (ACROBiosystems, IL2-H52H6) protein (100 $\mu$g/mouse) was used as an immunogen, which was mixed thoroughly and emulsified with equal volumes of complete Freund's adjuvant (prime immunization) or incomplete Freund's adjuvant (boost immunization), and BALB/c mice were immunized subcutaneously every 2 weeks for 6 weeks. Three days before cell fusion, intraperitoneal injection of adjuvant-free immunogen (50 $\mu$g/mouse) was performed. Mice with high serum antibody titer and gradual increase were selected, and splenic lymphocytes of the mice were isolated in a sterile environment. Splenic lymphocytes ($1 \times 10^8$) of the immunized mice were fused with myeloma cells Sp2/0 ($5 \times 10^7$) by an electric fusion method to obtain hybridoma cells. After the fusion, the hybridoma cells were resuspended with HAT complete medium, and then added to a 96-well plate at $1 \times 10^7$ cells/well and cultured in an incubator containing 5% $CO_2$ at 37 °C. On day 6 to day 7 after the fusion, the medium was removed. 200 $\mu$L of HAT complete medium was added to each well. The culture was performed at 37 °C in an incubator containing 5% $CO_2$ for 1 day, and then the culture supernatant was collected from the 96-well plate. The binding activity of the antibody in the culture supernatant to hIL-36R was measured by ELISA (see Example 5 for the method), the binding activity of the antibody in the culture supernatant to cells overexpressing hIL-36R or CynoIL-36R was measured by FACS (see Example 6 for the method), and the function of the antibody in the culture supernatant to block the binding of IL36 protein to cells overexpressing hIL-36R or CynoIL-36R was measured by FACS (see Example 7 for the method). Antibodies having binding activity and blocking function were screened, and their corresponding clones, e.g., clones 72C1, 84C4, 74B4D6, 58A8D1 and 6G10, were subcloned by limiting dilution.

Example 3. Construction and Expression of Chimeric Anti-hIL-36R Antibodies

The total RNA was isolated as a template from the hybridoma cells screened in Example 2 using a total RNA extraction kit (Takara, Cat: 9767), and first strand cDNA was synthesized using superscript III reverse transcriptase (Thermo, 18080051). Using the first strand cDNA as a template, the variable region sequences of the antibodies were then amplified by PCR reactions using degenerate mouse IgG primers. The PCR mixture was electrophoretically separated in a 1% agarose/Tris-borate gel containing 0.5 $\mu$g/mL ethidium bromide. The position of the amplification product fragment was determined, and the PCR product was recovered and purified by using a PCR product recovery kit (Corning,AP-PCR-250). The purified PCR product was cloned into pMD-19T vector (Takara, 6013) and transformed into DH5$\alpha$ competent *Escherichia coli* cells (Takara, 9057). Positive clones were screened by colony PCR and DNA sequencing was performed on the positive clones to obtain the sequences of the heavy chain variable region and the light chain variable region of the antibody, such as 72C1 (SEQ ID NOs: 7 and 15), 84C4 (SEQ ID NOs: 27 and 39), 74B4D6 (SEQ ID NOs: 51 and 52), 58A8D1 (SEQ ID NOs: 59 and 60) and 6G10 (SEQ ID NOs: 67 and 68).

The VL region gene synthetic fragment of the mouse antibody was linked to a human $\kappa$ light chain constant region to construct a chimeric light chain, and the VH region gene synthetic fragment of the mouse antibody was linked to a human IgG4 heavy chain constant region to construct a chimeric heavy chain. The vector containing the chimeric light chain DNA and the vector containing the chimeric heavy chain DNA were transfected into Expi CHO cells for transient expression, and the chimeric antibody in the cell culture supernatant was purified using a Protein A column (GE healthcare) 7 days after the culture of the Expi CHO cells. The human $\kappa$ light chain constant region had an amino acid sequence set forth in SEQ ID NO: 73 and the human IgG4 heavy chain constant region had an amino acid sequence set forth in SEQ ID NO: 71. "xi" of the present disclosure referred to a chimeric antibody. For example, xi72C1 refers to a chimeric 72C1 antibody.

Example 4. Determination of Affinity of Anti-IL-36R Antibodies

The affinity of anti-IL-36R antibodies to CynoIL36R-ECD mFc and human IL-1Rrp2/IL-1R6 protein, His Tag (ACROBiosystems, IL2-H52H6) was measured by a ForteBio molecular interaction instrument. First, a ProA biosensor (SARTORIUS, 18-5010) was activated, and then the biosensor was immersed in a solution containing 5 $\mu$g/mL of the anti-IL-36R antibody of the present disclosure or a reference antibody (BI655130 of Boehringer Ingelheim, prepared in house, whose amino acid sequences of the heavy and light chains were shown in SEQ ID NOs: 81 and 83) to capture the anti-IL36R antibody. And then the biosensor was used to bind CynoIL36R-ECD mFc or human IL-1Rrp2/IL-1R6 protein, His Tag which was diluted by a gradient (the initial concentration was 200 nM, 2-fold gradient dilution), the response signals were detected in real time using a Fortebio molecular interaction instrument (SARTORIUS, octet red 96e), and association and dissociation curves were obtained. After the completion of dissociation in each cycle, the biosensor was regenerated for the next capture; the procedure was repeated until the affinity of the different anti-IL-36R antibodies for IL-36R was determined. The resulting data were analyzed by Fortebio Data Analysis 11.0 software in a 1:1 (Langmuir) binding model to determine $k_a$ ($k_{on}$) and $k_d$ ($k_{off}$) values, and the dissociation constant $K_D$ was calculated by $K_D = k_d/k_a$. The detection method essentially followed the manufacturer's recommendations, and more information could be found at www.fortebio.com. The affinity of the chimeric anti-IL-36R antibody for the human IL36R antigen is shown in Table 2.1 and Table 2.2.

Table 2.1. Affinity of chimeric anti-IL-36R antibodies for hIL-36R

| hIL-36R | | | |
|---|---|---|---|
| Antibody | $K_{on}$ (1/Ms) | $K_{off}$ (1/s) | $K_D$(M) |
| BI655130 | 6.41E+04 | 1.57E-05 | 2.45E-10 |
| xi72C1 | 5.96E+04 | 4.38E-05 | 7.36E-10 |
| xi74B4D6 | 7.04E+04 | 3.32E-04 | 4.72E-09 |

Table 2.2. Affinity of chimeric anti-IL-36R antibodies for hIL-36R

| hIL-36R | | | |
|---|---|---|---|
| Antibody | $K_{on}$ (1/Ms) | $K_{off}$ (1/s) | $K_D$ (M) |
| BI655130 | 5.86E+04 | 1.29E-05 | 2.20E-10 |
| xi84C4 | 1.06E+05 | 7.00E-05 | 6.60E-10 |
| xi58A8D1 | 3.60E+04 | 9.15E-06 | 2.54E-10 |
| xi6G10 | 5.69E+04 | 5.01E-05 | 8.81E-10 |

Example 5. ELISA-Based Binding Assay of Anti-IL-36R Antibodies

The binding activity of anti-IL-36R antibodies to IL-36R was analyzed by ELISA using human IL-1Rrp2/IL-1R6 protein, His Tag (ACROBiosystems, IL2-H52H6) and CynoIL36R-ECD mFc (for detection of chimeric and humanized antibodies), and hIL36R-ECD hFc and CynoIL36R-ECD hFc protein (for detection of hybridoma antibodies) as antigens. A 96-well plate (Costar, 9018) was coated with 2 $\mu$g/mL antigen at 100 $\mu$L/well and incubated overnight at 4 °C. After the 96-well plate was washed with a washing buffer (PBS containing 0.05% v/v Tween 20), a blocking buffer (PBS containing 2% bovine serum albumin) was added for blocking at 37 °C for 2 h. After the 96-well plate was washed with the washing buffer, 100 $\mu$L of the gradient diluted anti-IL-36R antibody of the present disclosure or the reference antibody BI655130 (the initial concentration of the antibody was 66 nM, 4-fold gradient dilution) was added to each well, and the plate was incubated at room temperature for 1 h. After the 96-well plate was washed with the washing buffer, 100 $\mu$L of horseradish peroxidase-conjugated recombinant protein A/G (TheroScientific, 32490) was added to each well, and the plate was incubated at room temperature for 1 h. After the 96-well plate was washed with the washing buffer, 100 $\mu$L of a TMB solution (Thermo, 00-4201-56) was added to each well, and the plate was incubated at room temperature for 2 min. Finally, 100 $\mu$L of a stop solution (2 N $H_2SO_4$) was added to each well to stop the reaction. Absorbance was read at 450 nm using a microplate reader (PE, Envision), the resulting data was analyzed using GraphPad Prism, and $EC_{50}$ was calculated. Binding $EC_{50}$ of the chimeric anti-IL-36R antibodies to hIL-36R in an ELISA assay is shown in FIG. 1, and binding $EC_{50}$ of the chimeric anti-IL-36R antibodies to CynoIL-36R in an ELISA assay is shown in FIG. 2. The binding of BI655130 is shown as a control, and the binding of IgG4 is shown as a negative control.

Example 6. Cell-Based Binding Assay of Anti-IL-36R Antibodies

The binding activity of anti-IL-36R antibodies to cell lines (293T-hIL36R-NF$\kappa$B) overexpressing human IL-36R (SEQ ID NO: 75) and cell lines (293T-CynoIL36R-NFxB) overexpressing cynomolgus IL-36R (SEQ ID NO: 78) was analyzed using an FACS-based method. Plasmid pGL4.32 [luc2P/NF-$\kappa$B-RE/Hygro] Vector (Promega, E8491) was transfected into HEK293T cells according to the instructions for lipofectamine 3000 transfection reagent (Thermo, L3000015) to give 293T-NFxB stable cell lines. Then, the coding sequences of hIL-36R and CynoIL-36R were inserted into BamHI and XhoI sites of the pcDNA3.1/Zeo (+) vector, respectively, to obtain recombinant plasmids pcDNA3.1/Zeo (+)-hIL36R and pcDNA3.1/Zeo (+)-CynoIL36R. Two recombinant plasmids were transfected into 293T-NFxB stable cell lines respectively according to the instructions of lipofectamine 3000 transfection reagent (Thermo, L3000015), resulting in 293T-hIL36R-NF$\kappa$B cell lines and 293T-CynoIL36R-NFxB cell lines.

293T-hIL36R-NF$\kappa$B cells and 293T-CynoIL36R-NF$\kappa$B cells in logarithmic growth phase and good cell condition were added to a 96-well culture plate at $2 \times 10^5$ cells/well, the supernatant was discarded, and then the gradient diluted anti-IL-36R antibody of the present disclosure or the reference antibody BI655130 (20 $\mu$g/mL for initial antibody concentration in 293T-hIL36R-NFxB binding assay, 3-fold gradient dilution; 40 $\mu$g/mL for initial antibody concentration in 293T-CynoIL36R-NF$\kappa$B binding assay, 2-fold gradient dilution) was added to the 96-well plate and incubated at room temperature for 60 min. After the cells was washed with PBS, 100 $\mu$L of a goat-anti-human IgG secondary antibody (Jackson, 109-116-170) diluted at 1:200 was added to each well and incubated at room temperature for 30 min. After the cells was washed with PBS, the cells were resuspended with 100 $\mu$L of PBS and then the fluorescence signal was detected using a flow cytometer (BD, Accuri C6). The binding ability of the anti-IL-36R antibodies to hIL36R and CynoIL36R on the surface of 293T cells was measured by mean fluorescence intensity (MFI) of staining. The ratio was the ratio of mean fluorescence intensity (MFI) of the experimental group/the background MFI, and the resulting data were analyzed using GraphPad Prism. Binding $EC_{50}$ of the chimeric anti-IL-36R antibodies to hIL-36R on 293T-hIL36R-NF$\kappa$B cells is shown in FIGs. 3A-3B, binding $EC_{50}$ of the chimeric anti-IL-36R antibodies to CynoIL-36R on 293T-CynoIL36R-NF$\kappa$B cells is shown in FIG. 4, the binding of BI655130 is shown as a control, and the binding of IgG4 is shown as a negative control.

Example 7. Analysis of Anti-IL-36R Antibodies Blocking Interaction Between IL36$\kappa$, IL36$\beta$ and IL36$\gamma$, and Cell Lines Overexpressing Human IL-36R and Cell Lines overexpressing Cynomolgus IL-36R

Based on chemiluminescence assays, the function of the anti-IL-36R antibodies blocking the interaction between hIL36$\alpha$ (R&D, 6995-IL-010), hIL36$\beta$ (R&D, 6834-ILB-025) and hIL36$\gamma$ (R&D, 6835-IL-010), and cell lines overexpressing human IL36R (293T-hIL36R-NF$\kappa$B) and cell lines overexpressing cynomolgus IL36R (293T-CynoIL36R-NF$\kappa$B) was analyzed. The preparation method of the 293T-hIL36R-NF$\kappa$B and 293T-CynoIL36R-NF$\kappa$B cell lines is shown in Example 6. When hIL36$\alpha$, hIL36$\beta$ or hIL36$\gamma$ protein bound to IL-36R on the cell surface, natural IL-1RAcP protein on the 293T cell surface was recruited to act together to activate a downstream NF$\kappa$B signal pathway, induce Luciferase expression and react with a detection substrate to generate a fluorescence signal. Thus, when an anti-IL-36R antibody was present in the assay system, it was capable of blocking the binding of hIL36$\alpha$, hIL36$\beta$ and hIL36$\gamma$, and IL-36R, resulting in a decrease in the signal detected.

293T-hIL36R-NFκB cells and 293T-CynoIL36R-NFκB cells in logarithmic growth phase and good cell condition were diluted to the concentration of $2.5 \times 10^5$ cells/mL by culture medium respectively, and inoculated in a 384-well plate at the concentration of 5 μL/well. Then 15 μL of the gradient diluted anti-IL-36R antibody of the present disclosure or the reference antibody BI655130 (in 293T-hIL36R-NFκB assay, the initial concentration of the antibody was 20 μg/mL, 3-fold gradient dilution; in 293T-CynoIL36R-NFκB assay, the initial concentration of the antibody was 40 μg/mL, 2-fold gradient dilution) was added to each well and incubated in an incubator with $CO_2$ at 37 °C for 60 min. After the incubation, 5 μL of hIL36α, hIL36β, hIL36γ or hIL36αβγ was added to each well (in 293T-hIL36R-NFκB assay, the final concentration of hIL36α was 100 ng/mL, the final concentration of hIL36β was 20 ng/mL, the final concentration of hIL36γ was 10 ng/mL, and hIL36αβγ was formed by mixing hIL36α, hIL36β and hIL36γ solutions, wherein the final concentrations of the three were 100 ng/mL, 20 ng/mL and 10 ng/mL, respectively; in 293T-CynoIL36R-NFκB assay, the final concentration of hIL36α was 3.33 ng/mL, the final concentration of hIL36β was 1 ng/mL, the final concentration of hIL36γ was 0.006 ng/mL, and hIL36αβγ was formed by mixing hIL36α, hIL36β and hIL36γ solutions, wherein the final concentrations of the three were 3.33 ng/mL, 1 ng/mL and 0.006 ng/mL, respectively), mixed well at low speed centrifugation, and incubated overnight in an incubator with $CO_2$ at 37 °C. Then, 25 μL of ONE-Glo™ Luciferase Assay (Promega, E6120) was added to each well, and the reaction was carried out for 2-5 min in the dark, and the chemiluminescent signal was read by a microplate reader (PE, Envision). The obtained data were analyzed using GraphPad Prism, and IC50 values and inhibition rate (inhibition rate = (luminescence value of a no antibody added group - luminescence value of an antibody group) / (luminescence value of a no antibody added group - luminescence value of a blank control group) × 100%) were calculated. The results of the chimeric anti-IL-36R antibody blocking the interaction between hIL36α, hIL36β and hIL36γ proteins, and hIL-36R on 293T-hIL36R-NFκB cells are shown in FIGs. 5A-5B, 5C-5D and 5E-5F, respectively, and the results of the chimeric anti-IL-36R antibody blocking the interaction between hIL36α, hIL36β and hIL36γ proteins, and CynoIL-36R on 293T-CynoIL36R-NFκB cells are shown in FIGs. 6A, 6B and 6C, respectively.

Example 8. Analysis of Anti-IL-36R Antibodies Blocking Interaction Between IL36α, IL36β and IL36γ Proteins, and OVCAR8 or NHK cells Naturally Expressing IL36R Protein

Based on ELISA assays, the function of the anti-IL-36R antibodies blocking the interaction between hIL36α (R&D, 6995-IL-010), hIL36β (R&D, 6834-ILB-025) and hIL36γ (R&D, 6835-IL-010), and human ovarian cancer cells OVCAR8 naturally expressing IL-36R (purchased from Nanjing Cobioer) or normal human epidermal keratinocytes (also known as NHK cells, purchased from ATCC) naturally expressing IL-36R was analyzed. When hIL36α, hIL36β or hIL36γ proteins bound to IL-36R on the cell surface, IL-1RAcP protein was recruited to induce downstream signaling pathways to produce the cytokine IL-8. Therefore, when an anti-IL-36R antibody was present in the assay system, it was capable of blocking the binding of hIL36α, hIL36β and hIL36γ to IL-36R to inhibit the production of IL-8.

OVCAR8 cells and NHK cells in logarithmic growth phase and good cell condition were diluted to a concentration of $1 \times 10^5$ cells/mL with culture medium, inoculated in a 96-well plate at 100 μL/well (i.e., 10000 cells each well), and cultured overnight in an incubator with $CO_2$ at 37 °C. The supernatant was discarded and 100 μL of culture medium was added to each well. Then 50 μL of the gradient diluted anti-IL-36R antibody of the present disclosure or the reference antibody BI655130 (the initial concentration of the antibody was 1000 μg/mL, 3-fold gradient dilution) was added to each well and incubated in an incubator with $CO_2$ at 37 °C for 60 min. After the incubation, 50 μL of hIL36α, hIL36β, hIL36γ or hIL36αβγ (the final concentration of hIL36α was 15 ng/mL, the final concentration of hIL36β was 4 ng/mL, the final concentration of hIL36γ was 2 ng/mL, and hIL36αβγ was formed by mixing hIL36α, hIL36β and hIL36γ solutions, wherein the final concentrations of the three were 15 ng/mL, 4 ng/mL and 2 ng/mL, respectively) was added to each well, mixed well at low speed centrifugation, and incubated in an incubator with $CO_2$ at 37 °C for 48 h. The supernatant was collected, and the IL-8 content in the supernatant of the 96-well plate was detected according to the instructions of the IL-8 detection kit (R&D, DY208), and the OD450 value was read by a microplate reader (PE, Envision). The obtained data were analyzed using GraphPad Prism, and IC50 values and inhibition rate (inhibition rate = (luminescence value of a no antibody added group - luminescence value of an antibody group) / (luminescence value of a no antibody added group - luminescence value of a blank control group) × 100%) were calculated. The results of chimeric anti-IL-36R antibodies blocking the interaction between hIL36α, hIL36β and hIL36γ, and OVCAR8 cells are shown in FIGs. 7A-7B, 7C-7D and 7E-7F, respectively, and chimeric anti-IL-36R antibodies blocking the interaction between hIL36α, hIL36β and hIL36γ, and NHK cells are shown in FIGs. 8A-8B, 8C and 8D, respectively.

Example 9. Humanization of Mouse Anti-IL-36R Monoclonal Antibodies

Mouse antibodies 72C1 and 84C4 were selected for humanization design. A human germline antibody framework region that could be used for humanization of the mouse antibody was screened using the CDR graft method. The CDRs of the mouse antibody were inserted into the selected framework regions by screening a human germline antibody or a subtype thereof having the highest sequence identity with the amino acid sequence of the variable region of the mouse antibody. And the residues of the framework region and/or the CDR region were further mutated to obtain more candidate antibodies. The mouse antibody 72C1 could obtain 6 humanized antibodies in total, the mouse antibody 84C4 could obtain 10 humanized antibodies in total, and the amino acid sequences of the heavy chain variable region and the light chain variable region of the humanized antibodies are shown as follows:

hz72C1-1.1 (SEQ ID NOs: 9, 17), hz72C1-1.2 (SEQ ID NOs: 11, 17), hz72C1-1.3 (SEQ ID NOs: 13, 17),
hz72C1-2.1 (SEQ ID NOs: 9, 19), hz72C1-2.2 (SEQ ID NOs: 11, 19), hz72C1-2.3 (SEQ ID NOs: 13, 19);
hz84C4-1.1 (SEQ ID NOs: 29, 41), hz84C4-1.2 (SEQ ID NOs: 31, 41), hz84C4-1.3 (SEQ ID NOs: 33, 41),
hz84C4-1.4 (SEQ ID NOs: 35, 41), hz84C4-1.5 (SEQ ID NOs: 37, 41), hz84C4-2.1 (SEQ ID NOs: 29, 43),
hz84C4-2.2 (SEQ ID NOs: 31, 43), hz84C4-2.3 (SEQ ID NOs: 33, 43), hz84C4-2.4 (SEQ ID NOs: 35, 43), hz84C4-2.5
(SEQ ID NOs: 37, 43).

A VL region gene synthetic fragment of a humanized antibody was ligated to a human κ light chain constant region to construct a humanized light chain, and a VL region gene synthetic fragment of a humanized antibody was ligated to a human IgG4 heavy chain constant region to construct a humanized heavy chain. The vector containing the humanized light chain DNA and the vector containing the humanized heavy chain DNA were transfected into ExpiCHO cells for protein expression, and after 7 days of culture of the ExpiCHO cells, the humanized antibody in the cell culture supernatant was purified using a Protein A column (GE healthcare). The human κ light chain constant region had an amino acid sequence set forth in SEQ ID NO: 73 and the human IgG4 heavy chain constant region had an amino acid sequence set forth in SEQ ID NO: 71. "hz" of the present disclosure referred to humanized antibodies. For example, hz72C1 referred to humanized 72C1 antibodies.

Example 10. Biological Function Assays of Humanized Anti-IL-36R Antibodies

Affinity, binding and blocking assays for humanized antibodies hz72C1-1.1, hz72C1-1.2, hz72C1-1.3, hz72C1-2.1, hz72C1-22 and hz72C1-2.3 as well as hz84C4-1.1, hz84C4-1.2, hz84C4-1.3, hz84C4-1.4, hz84C4-1.5, hz84C4-2.1, hz84C4-2.2, hz84C4-2.3, hz84C4-2.4 and hz84C4-2.5 were performed according to the methods of Example 4, Example 5, Example 6, Example 7 and Example 8. The affinity of the humanized anti-IL-36R antibodies for hIL-36R and CynoIL-36R is shown in Table 3.1 and Table 3.2.

Table 3.1. Affinity of humanized anti-IL-36R antibodies for hIL-36R and CynoIL-36R

| Antibody | hIL-36R | | | CynoIL-36R | | |
|---|---|---|---|---|---|---|
| | $k_{on}$ (1/Ms) | $k_{off}$ (1/s) | $K_D$ (M) | $k_{on}$ (1/Ms) | $k_{off}$ (1/s) | $K_D$ (M) |
| BI655130 | 6.41E+04 | 1.57E-05 | 2.45E-10 | 2.95E+04 | 7.26E-05 | 2.46E-09 |
| xi72C1 | 5.96E+04 | 4.38E-05 | 7.36E-10 | 2.57E+04 | 9.04E-06 | 3.52E-10 |
| hz72C1-1.1 | 1.13E+05 | 1.81E-05 | 1.60E-10 | 2.28E+04 | 1.51E-05 | 6.60E-10 |
| hz72C1-1.2 | 1.11E+05 | 3.91E-06 | 3.51E-11 | / | / | / |
| hz72C1-1.3 | 1.19E+05 | 8.80E-06 | 7.43E-11 | 2.12E+04 | 9.42E-06 | 4.45E-10 |
| hz72C1-2.1 | 7.04E+04 | 7.81E-07 | 1.11E-11 | / | / | / |
| hz72C1-2.2 | 6.24E+04 | <1.0E-07 | <1.0E-12 | / | / | / |
| hz72C1-2.3 | 6.33E+04 | 1.12E-05 | 1.77E-10 | / | / | / |
| Note: "\" indicates no detection | | | | | | |

Table 3.2. Affinity of humanized anti-IL-36R antibodies for hIL-36R

| Antibody | hIL-36R | | |
|---|---|---|---|
| | $k_{on}$ (1/Ms) | $k_{off}$ (1/s) | $K_D$ (M) |
| BI655130 | 8.19E+04 | 5.84E-05 | 7.13E-10 |
| hz84C4-1.1 | 1.07E+05 | 1.02E-04 | 9.58E-10 |

(continued)

| Antibody | hIL-36R | | |
|---|---|---|---|
| | $k_{on}$ (1/Ms) | $k_{off}$ (1/s) | $K_D$ (M) |
| hz84C4-1.2 | 1.00E+05 | 1.61E-04 | 1.61E-09 |
| hz84C4-1.3 | 6.19E+04 | 3.26E-05 | 5.27E-10 |
| hz84C4-1.4 | 1.19E+05 | 2.36E-04 | 1.98E-09 |
| hz84C4-1.5 | 1.00E+05 | 1.34E-04 | 1.34E-09 |
| hz84C4-2.1 | 9.45E+04 | 1.16E-04 | 1.22E-09 |
| hz84C4-2.2 | 9.15E+04 | 1.42E-04 | 1.55E-09 |
| hz84C4-2.3 | 6.16E+04 | 5.12E-05 | 8.31E-10 |
| hz84C4-2.4 | 1.05E+05 | 1.96E-04 | 1.88E-09 |
| hz84C4-2.5 | 9.64E+04 | 1.38E-04 | 1.44E-09 |

Binding $EC_{50}$ of the humanized anti-IL-36R antibodies to hIL-36R in an ELISA assay is shown in FIGs. 9A-9B, and binding $EC_{50}$ of the humanized anti-IL-36R antibodies to CynoIL-36R in an ELISA assay is shown in FIG. 10.

Binding $EC_{50}$ of the humanized anti-IL-36R antibodies to hIL-36R on 293T-hIL36R-NFxB cells is shown in FIGs. 11A-11B, and binding $EC_{50}$ of the humanized anti-IL-36R antibodies to CynoIL-36R on 293T-CynoIL36R-NFκB cells is shown in FIGs. 12A-12B.

The results of the humanized anti-IL-36R antibodies blocking the interaction between hIL36α, hIL36β, hIL36γ and hIL36αβγ proteins, and hIL-36R on 293T-hIL36R-NFκB cells are shown in FIGs. 13A-13B, 13C-13D, 13E-13F and 13G-13H, respectively, and Table 4.1 and Table 4.2.

Table 4.1. Ability of humanized anti-IL-36R antibodies blocking interaction between hIL36α, hIL36β, hIL36γ and hIL36αβγ, and hIL-36R on 293T-hIL36R-NFxB cells

| Antibody | 293T-hIL36R-NFxB cell | | | | | | | |
|---|---|---|---|---|---|---|---|---|
| | hIL36α | | hIL36β | | hIL36γ | | hIL36αβγ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| BI655130 | 6.09E-09 | 84.48 | 2.45E-09 | 86.67 | -1.32E-08 | 79.24 | 2.36E-08 | 81.21 |
| hz72C1-1.1 | 9.56E-09 | 99.57 | 3.07E-09 | 94.59 | 2.82E-08 | 98.14 | 5.27E-08 | 108.8 |
| hz72C1-1.2 | 1.04E-08 | 100.79 | 5.07E-09 | 96.94 | 2.18E-08 | 98.36 | -4.15E-08 | 86.67 |
| hz72C1-1.3 | 6.82E-09 | 98.73 | 3.88E-09 | 96.66 | 1.88E-08 | 101.43 | 4.03E-08 | 90.13 |
| hz72C1-2.1 | 9.83E-09 | 87.89 | 5.08E-09 | 92.29 | 3.72E-08 | 85.36 | 6.62E-08 | 70.52 |
| hz72C1-22 | 1.61E-08 | 92.04 | 4.66E-09 | 94.67 | 3.68E-08 | 87.89 | ~4.44E-08 | 77.86 |
| hz72C1-2.3 | 1.61E-08 | 90.8 | 2.93E-09 | 83.67 | 2.94E-08 | 89.00 | ~1.39E-08 | 66.28 |

Table 4.2. Ability of humanized anti-IL-36R antibodies blocking interaction between hIL36α, hIL36β, hIL36γ and hIL36αβγ, and hIL-36R on 293T-hIL36R-NFxB cells

| Antibody | 293T-hIL36R-NFκB cell | | | | | | | |
|---|---|---|---|---|---|---|---|---|
| | hIL36α | | hIL36β | | hIL36γ | | hIL36αβγ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| BI655130 | 1.75E-09 | 83.63 | 2.21E-09 | 85.24 | 1.10E-09 | 81.02 | 3.27E-09 | 63.28 |
| hz84C4-1.1 | 8.82E-10 | 84.14 | 9.28E-10 | 85.35 | 5.76E-10 | 83.51 | 1.82E-09 | 61.20 |
| hz84C4-2.5 | 9.38E-10 | 85.70 | 7.66E-10 | 83.10 | 3.50E-10 | 84.68 | ~1.55E-09 | 54.39 |

The results of the humanized anti-IL-36R antibodies blocking the interaction between hIL36α, hIL36β and hIL36γ proteins, and 293T-CynoIL36R-NFκB cells are shown in FIGs. 14A, 14B and 14C, respectively, and Table 5.

Table 5. Ability of humanized anti-IL-36R antibodies blocking interaction between hIL36α, hIL36β and hIL36γ, and CynoIL-36R on 293T-CynoIL36R-NFκB cells

| Antibody | 293T-CynoIL36R-NFκB cell | | | | | |
|---|---|---|---|---|---|---|
| | hIL36α | | hIL36β | | hIL36γ | |
| | $IC_{50}$ (M) | Maximum inhibition rate % | $IC_{50}$ (M) | Maximum inhibition rate % | $IC_{50}$ (M) | Maximum inhibition rate % |
| BI655130 | 2.77E-08 | 49.07% | 5.63E-08 | 60.76% | 1.19E-08 | 83.08% |
| hz72C1-1.1 | 3.15E-09 | 100.97% | 3.09E-09 | 100.37% | 1.66E-09 | 103.74% |
| hz72C1-1.3 | 3.13E-09 | 101.22% | 2.70E-09 | 100.62% | 1.65E-09 | 102.87% |

The results of the humanized anti-IL-36R antibodies blocking the interaction between hIL36α, hIL36β, hIL36γ and hIL36αβγ proteins, and OVCAR8 cells are shown in sequence in FIGs. 15A-15B, 15C-15D, 15E-15F and 15G-15H, respectively, and Table 6.1 and Table 6.2.

Table 6.1. Ability of humanized anti-IL-36R antibodies blocking interaction between hIL36α, hIL36β, hIL36γ and hIL36αβγ, and IL-36R on OVCAR8 cells

| Antibody | OVCAR8 cell | | | | | | | |
|---|---|---|---|---|---|---|---|---|
| | hIL36α | | hIL36β | | hIL36γ | | hIL36αβγ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| BI655130 | 4.34E-11 | 99.79 | 1.57E-11 | 99.7 | 1.42E-11 | 99.67 | 4.88E-11 | 99.87 |
| hz72C1-1.1 | 3.02E-11 | 98.95 | 4.11E-11 | 98.36 | 2.59E-11 | 98.16 | 9.17E-11 | 97.5 |
| hz72C1-1.2 | 6.21E-11 | 98.93 | 2.61E-11 | 98.16 | 1.38E-11 | 98.23 | 8.82E-11 | 97.92 |
| hz72C1-1.3 | 4.08E-11 | 99.01 | 1.60E-11 | 98.37 | 2.42E-11 | 98.3 | 8.40E-11 | 97.65 |
| hz72C1-2.1 | 1.43E-10 | 98.57 | 9.90E-11 | 98.35 | 8.27E-11 | 97.81 | 3.97E-10 | 96.38 |

(continued)

| Antibody | OVCAR8 cell | | | | | | | |
|---|---|---|---|---|---|---|---|---|
| | hIL36$\alpha$ | | hIL36$\beta$ | | hIL36$\gamma$ | | hIL36$\alpha\beta\gamma$ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| hz72C1-2.2 | 1.94E-10 | 98.55 | 1.45E-10 | 98.05 | 7.60E-11 | 97.74 | 2.30E-10 | 97.12 |
| hz72C1-2.3 | 2.17E-10 | 98.1 | 1.67E-10 | 97.93 | 5.31E-11 | 97.67 | 3.58E-10 | 94.96 |

Table 6.2. Ability of humanized anti-IL-36R antibodies blocking interaction between hIL36$\alpha$, hIL36$\beta$, hIL36$\gamma$ and hIL36$\alpha\beta\gamma$, and IL-36R on OVCAR8 cells

| Antibody | OVCAR8 cell | | | | | | | |
|---|---|---|---|---|---|---|---|---|
| | hIL36$\alpha$ | | hIL36$\beta$ | | hIL36$\gamma$ | | hIL36$\alpha\beta\gamma$ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| BI655130 | 1.60E-11 | 99.63 | 2.45E-11 | 99.09 | 8.12E-12 | 100.09 | 1.42E-11 | 99.87 |
| hz84C4-1.1 | 6.38E-12 | 99.67 | 7.61E-12 | 99.30 | 4.77E-12 | 99.57 | 6.51E-12 | 99.89 |
| hz84C4-1.2 | 8.50E-12 | 99.13 | 1.60E-12 | 98.94 | 8.99E-12 | 99.49 | 7.75E-12 | 99.23 |
| hz84C4-1.3 | - | 20.62 | - | 21.81 | -6.46E-12 | 59.49 | 5.33E-12 | 44.24 |
| hz84C4-1.4 | 1.42E-11 | 99.50 | 1.46E-11 | 98.51 | 7.75E-12 | 99.40 | 1.12E-11 | 98.89 |
| hz84C4-1.5 | 8.48E-12 | 99.52 | 1.06E-11 | 99.23 | 5.29E-12 | 99.57 | 7.24E-12 | 99.26 |
| hz84C4-2.1 | 1.09E-11 | 99.91 | 5.70E-12 | 99.27 | 6.45E-12 | 99.60 | 3.66E-12 | 99.90 |
| hz84C4-2.2 | 1.19E-11 | 99.18 | 8.59E-12 | 98.52 | 8.61E-12 | 98.73 | 1.31E-11 | 99.21 |
| hz84C4-2.3 | -6.47E-12 | 28.44 | 3.42E-12 | 18.58 | ~0.0062 | 41.51 | -4.34E-12 | 40.59 |
| hz84C4-2.4 | 7.52E-12 | 99.51 | 7.24E-12 | 99.10 | 5.15E-12 | 99.33 | 2.58E-12 | 98.80 |
| hz84C4-2.5 | 6.45E-12 | 100.03 | 7.06E-12 | 99.38 | 6.62E-12 | 99.74 | 4.01E-12 | 99.15 |
| Note: "-" indicates unfitted | | | | | | | | |

The results of the humanized anti-IL-36R antibodies blocking the interaction between hIL36$\alpha$, hIL36$\beta$, hIL36$\gamma$ and hIL36$\alpha\beta\gamma$ proteins, and NHK cells are shown in FIGs. 16A-16B, 16C-16D, 16E-16F and 16G-16H, respectively, and Table 7.1 and Table 7.2.

Table 7.1. Ability of humanized anti-IL-36R antibodies blocking interaction between hIL36α, hIL36β, hIL36γ and hIL36αβγ, and IL-36R on NHK cells

| Antibody | NHK cell | | | | | | | |
|---|---|---|---|---|---|---|---|---|
| | hIL36α | | hIL36β | | hIL36γ | | hIL36αβγ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| BI655130 | 1.26E-10 | 106 | 3.03E-10 | 100.74 | 4.42E-11 | 104.65 | 1.98E-10 | 98.88 |
| hz72C1-1.1 | 2.19E-10 | 99.05 | 3.85E-10 | 86.73 | 1.21E-10 | 102.45 | 3.72E-10 | 93.7 |
| hz72C1-12 | 1.19E-10 | 96.12 | 4.33E-10 | 84.74 | 1.14E-10 | 85.72 | 3.29E-10 | 89.58 |
| hz72C1-1.3 | 7.09E-11 | 97.26 | 2.40E-10 | 90.3 | 1.03E-10 | 93.51 | 3.82E-10 | 90.34 |
| hz72C1-2.1 | 1.13E-09 | 93.52 | 1.41E-09 | 82.06 | 2.41E-10 | 94.1 | 1.78E-9 | 84.56 |
| hz72C1-22 | 2.45E-10 | 97.26 | 1.10E-09 | 82.53 | 1.18E-9 | 89.05 | 1.49E-9 | 85.27 |
| hz72C1-2.3 | 1.80E-10 | 82.02 | 2.67E-09 | 78.29 | 5.60E-10 | 90.85 | 4.50E-9 | 79.95 |

Table 7.2. Ability of humanized anti-IL-36R antibodies blocking interaction between hIL36α, hIL36β, hIL36γ and hIL36αβγ, and IL-36R on NHK cells

| Antibody | NHK cell | | | | | | | |
|---|---|---|---|---|---|---|---|---|
| | hIL36α | | hIL36β | | hIL36γ | | hIL36αβγ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| BI655130 | 6.33E-11 | 101.02 | 3.29E-11 | 97.11 | 2.63E-11 | 106.57 | 7.27E-11 | 90.71 |
| hz84C4-1.1 | 2.62E-11 | 103.39 | 2.27E-11 | 103.48 | 1.44E-11 | 102.52 | 5.25E-11 | 93.68 |
| hz84C4-1.2 | 1.02E-10 | 92.54 | 9.53E-12 | 91.22 | 3.42E-12 | 98.34 | 6.67E-11 | 82.85 |
| hz84C4-1.3 | -2.41E-09 | 16.87 | -1.43E-09 | 19.38 | 2.13E-09 | 57.55 | -6.51E-12 | 40.74 |
| hz84C4-1.4 | 3.71E-11 | 94.42 | 3.48E-11 | 93.51 | 5.01E-12 | 109.19 | 5.76E-11 | 92.46 |
| hz84C4-1.5 | 2.87E-11 | 100.89 | 1.55E-11 | 100.52 | 1.27E-11 | 109.40 | 2.77E-11 | 96.95 |
| hz84C4-2.1 | 5.23E-11 | 99.49 | 3.18E-11 | 96.77 | 1.81E-11 | 97.53 | 4.82E-11 | 107.48 |
| hz84C4-2.2 | 8.45E-11 | 95.14 | 4.64E-11 | 95.83 | 6.21E-11 | 14.96 | 6.63E-11 | 100.35 |

(continued)

| Antibody | NHK cell | | | | | | | |
|---|---|---|---|---|---|---|---|---|
| | hIL36α | | hIL36β | | hIL36γ | | hIL36αβγ | |
| | $IC_{50}$ (M) | Maximum inhibition rate % | $IC_{50}$ (M) | Maximum inhibition rate % | $IC_{50}$ (M) | Maximum inhibition rate % | $IC_{50}$ (M) | Maximum inhibition rate % |
| hz84C4-2.3 | -9.24E-13 | 44.50 | -2.73E-11 | 23.39 | 8.76E-10 | 56.96 | 8.23E-11 | 33.13 |
| hz84C4-2.4 | 4.68E-11 | 94.95 | 6.52E-11 | 92.68 | 1.12E-11 | 101.24 | 1.57E-10 | 95.58 |
| hz84C4-2.5 | 4.81E-11 | 99.66 | 1.24E-11 | 101.80 | 8.40E-12 | 102.94 | 6.61E-11 | 98.52 |

Example 11. Expression Amount of Humanized Anti-IL-36R Antibodies

cDNAs encoding hz72C1-1.1, hz72C1-1.3, hz84C4-1.1, hz84C4-2.5 and reference antibody BI655130 were obtained by gene synthesis and subcloned into expression vector pcDNA™ 3.1 (Invitrogen V-790), respectively, and the expression vectors were transfected into ExpiCHO cells for transient expression. hz72C1-1.1, hz72C1-1.3, hz84C4-1.1, hz84C4-2.5 and BI655130 were then purified using a MabSelect SuRe LX column (GE healthcare), respectively. The concentration of the purified antibody was determined using an ultramicro spectrophotometer (Thermo Scientific, Nanodrop2000); and the purity of the purified antibody was analyzed using Superdex 200 increase 10/300 GL (GE healthcare), in which the mobile phase was phosphate buffered saline (1X, pH 7.4) and the detection wavelength was A280. The results are shown in Table 8.1 and Table 8.2, the transient expression amount of hz72C1-1.1, hz72C1-1.3, hz84C4-1.1 and hz84C4-2.5 were significantly better than that of BI655130.

Table 8.1. Expression Amount and Purities of Humanized Anti-IL-36R Antibodies

| Antibody | Transient transfection expression amount (7 days) | SEC purity |
|---|---|---|
| BI655130 | 27 mg/L | 99.9 |
| hz72C1-1.1 | 103.5 mg/L | 98.59 |
| hz72C1-1.3 | 87 mg/L | 99.21 |

Table 8.2. Expression Amount and Purities of Humanized Anti-IL-36R Antibodies

| Antibody | Transient transfection expression amount (8 days) | SEC purity |
|---|---|---|
| BI655130 | 25 mg/L | 100 |
| hz84C4-1.1 | 240 mg/L | 100 |
| hz84C4-2.5 | 285 mg/L | 99.37 |

Example 12. *In Vivo* Pharmacokinetic Analysis of Humanized Anti-IL36R Antibodies

ICR mice (purchased from Shanghai Sippe-Bk Lab Animal Co., Ltd.) were used as animal models and randomly divided into 3 groups of 6 mice each. On the day of experiment, hz72C1-1.1, hz72C1-1.3 and reference antibody BI655130 were injected into ICR mice via the tail vein respectively, with the administration dose of 10 mg/kg and the administration frequency of 1 time. Blood was collected at time points of 0 min before administration, 30 min, 2 h (± 1 min), 8 h (± 1 min), 24 h (± 2 min), 2 days (± 2 min), 4 days (± 2 min), 7 days (± 2 min), 14 days (± 5 min), 21 days (± 5 min) and 28 days (± 5 min) after administration. Blood samples were collected, and supernatants were separated by centrifugation, and assayed for indicators such as blood concentration in the supernatants by ELISA (see the method in Example 5). The experimental methods for hz84C4-1.1 and hz84C4-2.5 were identical to those described above (i.e., the experimental methods for hz72C1-1.1 and hz72C1-1.3).
The results are shown in Table 9.1 and Table 9.2, wherein the PK parameters for hz72C1-1.1 and hz84C4-2.5 were superior to those of BI655130, and the PK parameters for hz72C1-1.3 and hz84C4-1.1 were comparable to that of BI655130.

Table 9.1. PK parameters of humanized anti-IL-36R antibodies in mice

| PK parameter | Mean±SD | | |
|---|---|---|---|
| | BI655130 | hz72C1-1.1 | hz72C1-1.3 |
| Tmax (d) | 0.0208±0 | 0.0208±0 | 0.0208±0 |
| Cmax (µg/mL) | 177±17.3 | 176±4.04 | 135±11.9 |
| AUC(0-28d) (d*µg/mL) | 1063±96.5 | 1137±89.8 | 846±71.0 |
| t1/2 (d) | 12.2±1.17 | 13.4±1.21 | 13.1±0.660 |
| MRT(0-28d) (d) | 9.87±0.385 | 10.4±0.323 | 10.2±0.181 |
| CL (mL/d/kg) | 7.73±0.697 | 6.94±0.718 | 9.29±0.858 |

Table 9.2. PK parameters of humanized anti-IL-36R antibodies in mice

| PK parameter | Mean±SD | | |
|---|---|---|---|
| | BI655130 | hz84C4-1.1 | hz84C4-2.5 |
| Tmax (d) | 0.0208±0 | 0.0208±0 | 0.0208±0 |
| Cmax (µg/mL) | 154±12.8 | 143±18.0 | 158±21.6 |
| AUC(0-28d) (d*µg/mL) | 754.2±138 | 781±92.8 | 855±200 |
| t1/2 (d) | 11.4±0.759 | 11.4±2.19 | 11.9±1.40 |
| MRT(0-28d) (d) | 8.83±0.547 | 9.31±0.587 | 9.06±1.48 |
| CL (mL/d/kg) | 11.51±2.23 | 10.7±1.83 | 10.3±3.61 |
| Note: Tmax: peak time; Cmax: peak concentration; AUC: area under concentration-time curve; t1/2: half-life; MRT: mean residence time; CL: clearance; mean ± SD: mean ± standard deviation; d: day (s); PK: pharmacokinetics | | | |

The sequence information of the present disclosure is summarized in Table 10 below.

Table 10. Summary of sequence information

| Description of antibodies Sequence/SEQ ID NO. |
|---|
| VH-CDR1 of a mouse, chimeric and humanized 72C1 antibody<br>GASITSGYWN (SEQ ID NO: 1) |
| VH-CDR2 of mouse 72C1, chimeric 72C1, hz72C1-1.1, hz72C1-1.2, hz72C1-2.1 and hz72C1-2.2 antibodies<br>YIX$_1$YSGYTYYNPSLKS (SEQ ID NO: 2, X$_1$=S)<br>YISYSGYTYYNPSLKS |
| VH-CDR2 of hz72C1-1.3 and hz72C1-2.3 antibodies<br>YIX$_1$YSGYTYYNPSLKS (SEQ ID NO: 2, X$_1$=Y)<br>YIYYSGYTYYNPSLKS |
| VH-CDR3 of mouse, chimeric and humanized 72C1 antibodies<br>GGYDYVEDALYY (SEQ ID NO: 3) |
| VL-CDR1 of mouse, chimeric and humanized 72C1 antibodies<br>RASSNVRYMY (SEQ ID NO: 4) |
| VL-CDR2 of mouse, chimeric and humanized 72C1 antibodies<br>LTSNLAS (SEQ ID NO: 5) |

(continued)

| VL-CDR3 of mouse 72C1, chimeric 72C1, hz72C1-1.1, hz72C1-1.2 and hz72C1-1.3 antibodies |
|---|
| QQX$_2$TTSPYT (SEQ ID NO: 6, X$_2$=F)<br><br>QQFTTSPYT |
| VL-CDR3 of hz72C1-2.1, hz72C1-2.2 and hz72C1-2.3 antibodies<br>QQX$_2$TTSPYT (SEQ ID NO: 6, X$_2$=Y)<br><br>QQYTTSPYT |
| VH of mouse and chimeric 72C1 antibodies<br><br>EVQLQQSGPSLVKPSQTLSLTCSVTGASITSGYWNWIRNFPGNKLEYMGYISYSGYTYYNPSLKSRISI<br>RDTSKNQYYLQLISVTTEDTATYYCATGGYDYVEDALYYWGQGTSVTVSS (SEQ ID NO: 7) |
| VH of chimeric 72C1 antibody<br><br>GAGGTGCAGCTGCAGCAGTCCGGCCCCTCTCTGGTGAAGCCTTCTCAGACCCTGAGCCTGACATG<br>CTCCGTGACCGGCGCCAGCATCACATCCGGCTACTGGAACTGGATCCGGAATTTCCCAGGCAACA<br>AGCTGGAGTACATGGGCTATATCTCTTACAGCGGCTATACCTACTATAATCCCTCTCTGAAGAGC(<br>GGATCTCCATCACCAGAGACACATCTAAGAACCAGTACTATCTGCAGCTGATCTCCGTGACCACA<br>GAGGATACCGCCACATACTATTGTGCCACAGGCGGCTACGACTATGTGGAGGATGCCCTGTACTA<br>TTGGGGCCAGGGCACCAGCGTGACAGTGAGCTCC (SEQ ID NO: 8) |
| VH of hz72C1-1.1 and hz72C1-2.1 antibodies<br><br>QVQLQESGPGLVKPSQTLSLTCTVSGASITSGYWNWIRNHPGKGLEYIGYISYSGYTYYNPSLKSRVTI<br>SRDTSKNQFSLKLSSVTAADTAVYYCATGGYDYVEDALYYWGQGTLVTVSS (SEQ ID NO: 9)<br>CAGGTGCAGCTGCAGGAGTCCGGACCAGGACTGGTGAAGCCTTCTCAGACCCTGTCCCTGACCTG<br>CACAGTGTCCGGCGCCAGCATCACATCTGGCTACTGGAACTGGATCAGGAATCACCCAGGCAAG(<br>GCCTGGAGTACATCGGCTATATCAGCTACTCTGGCTATACCTACTATAACCCCTCCCTGAAGAGC/<br>GGGTGACCATCAGCCGGGACACATCTAAGAATCAGTTCTCCCTGAAGCTGTCCAGCGTGACCGC(<br>GCTGATACAGCCGTGTACTATTGTGCTACAGGCGGCTACGACTATGTGGAGGATGCTCTGTACTA`<br>TGGGGCCAGGGCACCCTGGTGACAGTGTCTTCC (SEQ ID NO: 10) |
| VH of hz72C1-1.2 and hz72C1-2.2 antibodies<br><br>QVQLQESGPGLVKPSQTLSLTCTVSGASITSGYWNWIRNHPGKGLEYIGYISYSGYTYYNPSLKSRVTI<br>SRDTSKNQYYLKLSSVTAADTAVYYCATGGYDYVEDALYYWGQGTLVTVSS (SEQ ID NO: 11)<br>CAGGTGCAGCTGCAGGAGTCCGGACCAGGACTGGTGAAGCCTTCTCAGACCCTGTCCCTGACCTG<br>CACAGTGTCCGGCGCCAGCATCACATCTGGCTACTGGAACTGGATCAGGAATCACCCAGGCAAG(<br>GCCTGGAGTACATCGGCTATATCAGCTACTCTGGCTATACCTACTATAACCCCTCCCTGAAGAGC/<br>GGGTGACCATCAGCCGGGACACATCTAAGAATCAGTACTATCTGAAGCTGTCCAGCGTGACCGC(<br>GCTGATACAGCCGTGTACTATTGTGCTACAGGCGGCTACGACTATGTGGAGGATGCTCTGTACTA`<br>TGGGGCCAGGGCACCCTGGTGACAGTGTCTTCC (SEQ ID NO: 12) |
| VH of hz72C1-1.3 and hz72C1-2.3 antibodies<br><br>QVQLQESGPGLVKPSQTLSLTCTVSGASITSGYWNWIRNHPGKGLEYIGYIYYSGYTYYNPSLKSRVTI<br>SRDTSKNQYYLKLSSVTAADTAVYYCATGGYDYVEDALYYWGQGTLVTVSS (SEQ ID NO: 13)<br>CAGGTGCAGCTGCAGGAGAGCGGACCAGGACTGGTGAAGCCTTCCCAGACCCTGAGCCTGACCT`<br>GCACAGTGAGCGGCGCCTCTATCACATCCGGCTACTGGAACTGGATCAGGAATCACCCAGGCAAC<br>GGCCTGGAGTACATCGGCTATATCTACTATTCTGGCTATACCTACTATAACCCCAGCCTGAAGTCT<br>AGGGTGACCATCTCTCGGGACACATCCAAGAATCAGTACTATCTGAAGCTGTCCAGCGTGACCGC<br>CGCTGATACAGCCGTGTACTATTGTGCTACAGGCGGCTACGACTATGTGGAGGATGCTCTGTACT<br>ATTGGGGCCAGGGCACCCTGGTGACAGTGTCTTCC (SEQ ID NO: 14) |
| VL of mouse and chimeric 72C1 antibodies<br><br>EILLTQSPAIMSATLGEKVTMSCRASSNVRYMYWYQLKSGASPKLWIYLTSNLASGVPARFSGSGSGT!<br>YSLTISSVEAEDAATYFCQQFTTSPYTFGGGTKLEIK (SEQ ID NO: 15) |
| VL of chimeric 72C1 antibody |

(continued)

| |
|---|
| GAGATCCTGCTGACCCAGTCCCCAGCCATCATGTCTGCCACCCTGGGCGAGAAGGTGACAATGTC<br>CTGCCGGGCCAGCTCCAACGTGAGATACATGTATTGGTACCAGCTGAAGAGCGGCGCCTCCCCCA<br>AGCTGTGGATCTATCTGACCTCTAATCTGGCAAGCGGAGTGCCTGCACGGTTCTCCGGCTCTGGC<br>GCGGCACCTCCTACTCTCTGACAATCTCTAGCGTGGAGGCAGAGGACGCAGCAACATATTTCTGT<br>CAGCAGTTTACCACAAGCCCCTACACCTTTGGCGGCGGCACAAAGCTGGAGATCAAG (SEQ II<br>NO: 16) |
| VL of hz72C1-1.1, hz72C1-1.2 and hz72C1-1.3 antibodies<br><br>DIQMTQSPSSLSASVGDRVTITCRASSNVRYMYWYQQKPGKAPKLWIYLTSNLASGVPSRFSGSGSGT<br>YTLTISSLQPEDFATYYCQQFTTSPYTFGQGTKLEIK (SEQ ID NO: 17)<br>GACATCCAGATGACCCAGTCCCCTTCCAGCCTGTCCGCCAGCGTGGGCGATAGGGTGACCATCAC<br>ATGCCGGGCTTCTTCCAACGTGAGATACATGTATTGGTACCAGCAGAAGCCCGGCAAGGCCCCTA<br>AGCTGTGGATCTATCTGACATCTAATCTGGCTTCCGGAGTGCCAAGCCGCTTCTCTGGATCCGGA |
| GCGGCACCTCTTACACCCTGACAATCAGCTCTCTGCAGCCAGAGGACTTTGCCACATACTATTGT<br>AGCAGTTCACCACATCCCCCTATACCTTTGGCCAGGGCACAAAGCTGGAGATCAAG (SEQ ID NO<br>18) |
| VL of hz72C1-2.1, hz72C1-2.2 and hz72C1-2.3 antibodies<br><br>DIQMTQSPSSLSASVGDRVTITCRASSNVRYMYWYQQKPGKAPKLWIYLTSNLASGVPSRFSGSGSGT<br>SYTLTISSLQPEDFATYYCQQYTTSPYTFGQGTKLEIK (SEQ ID NO: 19)<br>GACATCCAGATGACCCAGTCCCCTTCCAGCCTGTCCGCCAGCGTGGGCGATAGGGTGACCATCAC<br>ATGCCGGGCTTCTTCCAACGTGAGATACATGTATTGGTACCAGCAGAAGCCCGGCAAGGCCCCTA<br>AGCTGTGGATCTATCTGACATCTAATCTGGCTTCCGGAGTGCCAAGCCGCTTCTCTGGATCCGGA<br>GCGGCACCTCTTACACCCTGACAATCAGCTCTCTGCAGCCAGAGGACTTCGCCACATACTATTGT<br>AGCAGTATACCACATCCCCCTACACCTTTGGCCAGGGCACAAAGCTGGAGATCAAG (SEQ ID NO<br>20) |
| VH-CDR1 of mouse 84C4, chimeric 84C4, hz84C4-1.1, hz84C4-1.2, hz84C4-1.3, hz84C4-1.5, hz84C4-2.1,<br>hz84C4-2.2, hz84C4-2.3 and hz84C4-2.5 antibodies<br>GYX$_3$FTSYWMN (SEQ ID NO: 21, X$_3$=F)<br><br>GYFFTSYWMN |
| VH-CDR1 of hz84C4-1.4 and hz84C4-2.4 antibodies<br>GYX$_3$FTSYWMN (SEQ ID NO: 21, X$_3$=Y)<br><br>GYYFTSYWMN |
| VH-CDR2 of mouse and chimeric 84C4 antibodies<br>X$_4$IX$_5$PYDSETRX$_6$X$_7$QKFX$_8$G (SEQ ID NO: 22, X$_4$=M, X$_5$=H, X$_6$=L, X$_7$=N, X$_8$=K)<br>MIHPYDSETRLNQKFKG |
| VH-CDR2 of hz84C4-1.1 and hz84C4-2.1 antibodies<br>X$_4$IX$_5$PYDSETRX$_6$X$_7$QKFX$_8$G (SEQ ID NO: 22, X$_4$=M, X$_5$=H, X$_6$=Y, X$_7$=A, X$_8$=Q)<br>MIHPYDSETRYAQKFQG |
| VH-CDR2 of hz84C4-1.2 and hz84C4-2.2 antibodies<br>X$_4$IX$_5$PYDSETRX$_6$X$_7$QKFX$_8$G (SEQ ID NO: 22, X$_4$=Y, X$_5$=H, X$_6$=Y, X$_7$=A, X$_8$=Q)<br>YIHPYDSETRYAQKFQG |
| VH-CDR2 of hz84C4-1.3 and hz84C4-2.3 antibodies<br>X$_4$IX$_5$PYDSETRX$_6$X$_7$QKFX$_8$G (SEQ ID NO: 22, X$_4$=M, X$_5$=Y, X$_6$=Y, X$_7$=A, X$_8$=Q)<br>MIYPYDSETRYAQKFQG |
| VH-CDR2 of hz84C4-1.4, hz84C4-1.5, hz84C4-2.4 and hz84C4-2.5 antibodies<br>X$_4$IX$_5$PYDSETRX$_6$X$_7$QKFX$_8$G (SEQ ID NO: 22, X$_4$=M, X$_5$=H, X$_6$=L, X$_7$=A, X$_8$=K)<br>MIHPYDSETRLAQKFKG |

(continued)

| |
|---|
| VH-CDR3 of mouse, chimeric and humanized 84C4 antibodies<br>STTAFAY (SEQ ID NO: 23) |
| VL-CDR1 of mouse, chimeric and humanized 84C4 antibodies<br>ASQDIGNALN (SEQ ID NO: 24) |
| VL-CDR2 of mouse, chimeric and humanized 84C4 antibodies<br>ATSSLDP (SEQ ID NO: 25) |
| VL-CDR3 of mouse, chimeric and humanized 84C4 antibodies<br>LQYASSPYT (SEQ ID NO: 26) |
| VH of mouse and chimeric 84C4 antibodies<br>EVQLQQLGAELVRPGASVMLSCKVSGYFFTSYWMNWVNQRPGQGLEWIGMIHPYDSETRLNQKFKC<br>KATLTVDKSASTAYLQLSSPTSEEYAVYYCASSTTAFAYWGQGTLVTVSA (SEQ ID NO: 27)<br>VH of chimeric 84C4 antibody<br>GAGGTGCAGCTGCAGCAGCTGGGAGCAGAGCTGGTGCGGCCAGGAGCAAGCGTGATGCTGTCCT<br>GCAAGGTGTCTGGCTACTTCTTTACCAGCTATTGGATGAACTGGGTGAATCAGAGGCCAGGACAG<br>GGACTGGAGTGGATCGGCATGATCCACCCTTACGACTCCGAGACAAGACTGAACCAGAAGTTCAA<br>GGGCAAGGCCACCCTGACAGTGGATAAGTCCGCCTCTACCGCCTACCTGCAGCTGAGCTCCCCCA<br>CATCTGAGGAGTATGCCGTGTACTATTGTGCCTCTAGCACCACAGCCTTTGCCTATTGGGGCCAGC<br>GCACCCTGGTGACAGTGAGCGCC (SEQ ID NO: 28) |
| VH of hz84C4-1.1 and hz84C4-2.1 antibodies<br>QVQLVQSGAEVKKPGASVKVSCKASGYFFTSYWMNWVRQAPGQGLEWMGMIHPYDSETRYAQKFC<br>GRVTMTVDKSTSTVYMELSSLRSEDTAVYYCASSTTAFAYWGQGTLVTVSS (SEQ ID NO: 29)<br>CAAGTGCAGCTGGTGCAGAGCGGAGCCGAGGTGAAAAAGCCCGGCGCTTCTGTGAAGGTGTCCT<br>GCAAGGCCAGCGGCTACTTCTTCACCTCCTACTGGATGAACTGGGTGCGGCAGGCCCCTGGCCAG<br>GGCCTGGAATGGATGGGCATGATCCACCCTTACGACAGCGAGACAAGATACGCCCAGAAGTTCCA<br>GGGCAGAGTGACCATGACCGTGGATAAGAGCACCAGCACAGTGTATATGGAACTGAGCAGCCTGA<br>GAAGCGAGGACACCGCCGTGTACTACTGTGCCTCTTCTACAACCGCTTTTGCCTACTGGGGCCAGC<br>GAACCCTGGTTACAGTCAGCAGC (SEQ ID NO: 30) |
| VH of hz84C4-1.2 and hz84C4-2.2 antibodies<br>QVQLVQSGAEVKKPGASVKVSCKASGYFFTSYWMNWVRQAPGQGLEWMGYIHPYDSETRYAQKFC<br>GRVTMTVDKSTSTVYMELSSLRSEDTAVYYCASSTTAFAYWGQGTLVTVSS (SEQ ID NO: 31)<br>CAAGTGCAGCTGGTTCAGAGCGGCGCCGAGGTGAAGAAGCCTGGCGCTAGCGTGAAGGTGTCTTC<br>TAAAGCCTCTGGCTACTTCTTCACCAGCTACTGGATGAACTGGGTCAGACAGGCCCCTGGCCAGGC<br>CCTGGAATGGATGGGATATATCCACCCCTACGACAGCGAGACAAGATACGCCCAGAAGTTCCAGGG<br>CAGAGTGACAATGACCGTGGACAAGTCCACCTCTACAGTGTACATGGAACTGAGCAGCCTGCGGA<br>GCGAGGATACCGCCGTGTACTACTGCGCCAGCAGCACCACCGCTTTTGCCTACTGGGGCCAGGGA<br>CACTGGTGACCGTGTCCAGC (SEQ ID NO: 32) |
| VH of hz84C4-1.3 and hz84C4-2.3 antibodies<br>QVQLVQSGAEVKKPGASVKVSCKASGYFFTSYWMNWVRQAPGQGLEWMGMIYPYDSETRYAQKFC<br>GRVTMTVDKSTSTVYMELSSLRSEDTAVYYCASSTTAFAYWGQGTLVTVSS (SEQ ID NO: 33)<br>CAAGTGCAGCTGGTTCAGAGCGGCGCCGAGGTGAAAAAGCCTGGCGCTTCTGTGAAGGTGTCCTC<br>CAAGGCCTCTGGCTACTTCTTCACCTCCTACTGGATGAACTGGGTGCGGCAGGCCCCTGGACAGGC<br>CCTGGAATGGATGGGCATGATCTACCCCTACGACAGCGAGACAAGATACGCCCAGAAGTTCCAGGC<br>AAGAGTGACAATGACCGTGGATAAGAGCACCAGCACAGTGTATATGGAACTGAGCAGCCTGAGAA<br>GCGAGGACACCGCCGTGTACTACTGTGCCAGCAGCACCACAGCTTTTGCCTACTGGGGCCAGGGC<br>ACCCTGGTGACCGTCAGCTCT (SEQ ID NO: 34) |
| VH of hz84C4-1.4 and hz84C4-2.4 antibodies |

(continued)

| |
|---|
| QVQLVQSGAEVKKPGASVKVSCKASGYYFTSYWMNWVRQAPGQGLEWMGMIHPYDSETRLAQKF KGRVTMTVDKSTSTVYMELSSLRSEDTAVYYCASSTTAFAYWGQGTLVTVSS (SEQ ID NO: 35) CAAGTGCAGCTGGTCCAGAGCGGCGCCGAGGTGAAAAAGCCTGGAGCTTCTGTGAAGGTGTCCTĠ CAAGGCCTCCGGCTACTACTTCACCAGCTACTGGATGAACTGGGTGCGGCAGGCCCCTGGCCAGG GCCTGGAATGGATGGGCATGATCCACCCCTACGACAGCGAGACAAGACTGGCCCAGAAATTCAAG GGCAGAGTGACCATGACCGTGGATAAGAGCACAAGCACCGTGTACATGGAACTGAGCAGCCTGAC AAGCGAGGACACCGCCGTGTACTATTGTGCCTCTAGCACCACCGCTTTTGCCTACTGGGGCCAGGG AACACTGGTGACAGTTTCTAGC (SEQ ID NO: 36) |
| VH of hz84C4-1.5 and hz84C4-2.5 antibodies<br><br>QVQLVQSGAEVKKPGASVKVSCKVSGYFFTSYWMNWVNQAPGQGLEWMGMIHPYDSETRLAQKFI GRVTMTVDKSTSTVYMELSSLRSEDTAVYYCASSTTAFAYWGQGTLVTVSS (SEQ ID NO: 37) CAAGTGCAGCTGGTTCAGAGCGGCGCCGAGGTGAAAAAGCCTGGCGCTTCTGTGAAGGTGTCTTĠ TAAAGTGTCCGGATATTTCTTCACCAGCTACTGGATGAACTGGGTCAACCAGGCCCCTGGACAGGĊ CCTGGAATGGATGGGCATGATCCACCCCTACGACAGCGAGACAAGACTGGCCCAGAAGTTCAAGG GCAGAGTGACCATGACCGTGGATAAGAGCACCTCTACCGTGTACATGGAACTGAGCAGCCTGCGG AGCGAGGACACCGCCGTGTACTACTGCGCCAGCAGCACAACAGCTTTTGCCTACTGGGGCCAGGG CACCCTGGTGACAGTGTCCAGC (SEQ ID NO: 38) |
| VL of mouse and chimeric 84C4 antibodies<br><br>DILMTQSPSSLSASLGERVSLTCRASQDIGNALNWLQQAPDGTIKRLIYATSSLDPGVPKRFSGSRSGSL YSLTISSLASEDFVYYYCLQYASSPYTFGGGTKLEIK (SEQ ID NO: 39)<br>VL of chimeric 84C4 antibody<br>GACATCCTGATGACCCAGAGCCCAAGCTCCCTGTCTGCCAGCCTGGGAGAGAGGGTGTCTCTGAĊ ATGCAGGGCAAGCCAGGACATCGGAAACGCCCTGAATTGGCTGCAGCAGGCCCCCGATGGCACĊ ATCAAGCGGCTGATCTATGCCACATCTAGCCTGGATCCCGGCGTGCCTAAGCGGTTCTCCGGCTĊ AGAAGCGGCTCCGACTACTCCCTGACCATCTCCTCTCTGGCCTCTGAGGATTTCGTGTACTATTAĊ TGTCTGCAGTATGCAAGCTCCCCATACACCTTTGGCGGAGGAACAAAGCTGGAGATCAAG (SEĠ ID NO: 40) |
| VL of hz84C4-1.1, hz84C4-1.2, hz84C4-1.3, hz84C4-1.4 and hz84C4-1.5 antibodies<br><br>DIQMTQSPSSLSASVGDRVTITCRASQDIGNALNWYQQKPGKAPKRLIYATSSLDPGVPSRFSGSRSGT DFTLTISSLQPEDFATYYCLQYASSPYTFGQGTKVEIK (SEQ ID NO: 41) GATATTCAGATGACCCAGAGCCCCTTCCTCCCTGAGCGCTAGCGTGGGCGACAGAGTGACCATCAĊ CTGTAGAGCCTCTCAGGACATCGGCAACGCCCTGAACTGGTACCAGCAGAAACCTGGCAAGGCCĊ CTAAGCGGCTGATCTACGCCACCAGCAGCCTGGACCCCGGAGTCCCCAGCAGATTCAGCGGCAGĊ CGGAGCGGAACAGATTTTACCCTGACCATCAGCTCCCTGCAACCTGAGGACTTCGCCACATACTA<br>CTGCCTGCAGTACGCTTCTTCTCCATATACATTCGGCCAGGGCACCAAGGTGGAAATCAAG (SEĠ ID NO: 42) |
| VL of hz84C4-2.1, hz84C4-2.2, hz84C4-2.3, hz84C4-2.4 and hz84C4-2.5 antibodies<br><br>DIQMTQSPSSLSASVGDRVTITCRASQDIGNALNWLQQKPGKAPKRLIYATSSLDPGVPSRFSGSRSGT DYTLTISSLQPEDFATYYCLQYASSPYTFGQGTKVEIK (SEQ ID NO: 43) GATATTCAGATGACCCAGAGCCCATCTTCCCTGAGCGCCAGCGTGGGCGATAGAGTGACCATCAĊ CTGTAGAGCCTCCCAGGACATCGGCAACGCCCTGAACTGGCTGCAGCAGAAACCTGGCAAGGCCĊ CTAAGCGGCTGATCTACGCTACAAGCTCCCTCGACCCCGGCGTCCCCAGCAGATTCAGCGGATCT CGGAGCGGAACAGACTACACCCTGACCATCAGCAGCCTGCAACCTGAGGACTTCGCCACCTACTA CTGCCTGCAGTACGCTTCTAGCCCTTATACCTTTGGCCAGGGCACAAAGGTGGAAATCAAG (SEĠ ID NO: 44) |
| VH-CDR1 of mouse and chimeric 74B4D6 antibodies<br>GYTFTNYWIH (SEQ ID NO: 45) |
| VH-CDR2 of mouse and chimeric 74B4D6 antibodies<br>TIDPSENYTNYNQKFKG (SEQ ID NO: 46) |

(continued)

| | |
|---|---|
| VH-CDR3 of mouse and chimeric 74B4D6 antibodies<br>SSFSY (SEQ ID NO: 47) | |
| VL-CDR1 of mouse and chimeric 74B4D6 antibodies<br>SASSSVSFIH (SEQ ID NO: 48) | |
| VL-CDR2 of mouse and chimeric 74B4D6 antibodies<br>TTSNLAS (SEQ ID NO: 49) | |
| VL-CDR3 of mouse and chimeric 74B4D6 antibodies<br>QQRSSYPLT (SEQ ID NO: 50) | |
| VH of mouse and chimeric 74B4D6 antibodies<br>EVQLQQPGAELGKPGASVKMACKASGYTFTNYWIHWVKQRPGQGLEWIGTIDPSENYTNYNQKFKG<br>KATLTVDTSSSTAYMQLSSLTSEDKAVYFCTGSSFSYWGQGTLVTVSA (SEQ ID NO: 51) | |
| VL of mouse and chimeric 74B4D6 antibodies<br>QNVLTQSPAIMSVSPGEKVTMTCSASSSVSFIHWFQQKPGTSPKLWIYTTSNLASGVPARFSGSGSGT<br>YSLTISRMEAEDAATYYCQQRSSYPLTFGAGTKLELK (SEQ ID NO: 52) | |
| VH-CDR1 of mouse and chimeric 58A8D1 antibodies<br>GYSFTSSWMH (SEQ ID NO: 53) | |
| VH-CDR2 of mouse and chimeric 58A8D1 antibodies<br>EIHPNSGNTNYNEKFKG (SEQ ID NO: 54) | |
| VH-CDR3 of mouse and chimeric 58A8D1 antibodies<br>SPYGYGGSMDY (SEQ ID NO: 55) | |
| VL-CDR1 of mouse and chimeric 58A8D1 antibodies<br>RASQDIHNYLN (SEQ ID NO: 56) | |
| VL-CDR2 of mouse and chimeric 58A8D1 antibodies<br>YTSRLYS (SEQ ID NO: 57) | |
| VL-CDR3 of mouse and chimeric 58A8D1 antibodies<br>QQGNTLPVM (SEQ ID NO: 58) | |
| VH of mouse and chimeric 58A8D1 antibodies<br>EVQLQQLGSVLVRPGISVKLSCKASGYSFTSSWMHWTKQRPGQGLEWIGEIHPNSGNTNYNEKFKGK<br>ATLTVDTSSRTAYVDLSSLTSEDSAVYYCARSPYGYGGSMDYWGQGTSVTVSS (SEQ ID NO: 59) | |
| VL of mouse and chimeric 58A8D1 antibodies<br>DIQMTQTTSSLSASLGDRVTISCRASQDIHNYLNWYQQKPDGTVKLLIYYTSRLYSGVPSRFSGSGSGT<br>DYSLTISNLEQEDFATYFCQQGNTLPVMFGSGTKLELK (SEQ ID NO: 60) | |
| VH-CDR1 of mouse and chimeric 6G10 antibodies<br>GITFSRFAMS (SEQ ID NO: 61) | |
| VH-CDR2 of mouse and chimeric 6G10 antibodies<br>TISSGDMYTYYPDSVKG (SEQ ID NO: 62) | |
| VH-CDR3 of mouse and chimeric 6G10 antibodies<br>MITTGEHAY (SEQ ID NO: 63) | |
| VL-CDR1 of mouse and chimeric 6G10 antibodies | |
| RASQDISVWLT (SEQ ID NO: 64) | |
| VL-CDR2 of mouse and chimeric 6G10 antibodies<br>KASNLHT (SEQ ID NO: 65) | |
| VL-CDR3 of mouse and chimeric 6G10 antibodies | |

(continued)

| LQSQSYPWT (SEQ ID NO: 66) |
| --- |

| VH of mouse and chimeric 6G10 antibodies<br><br>EVMLVESGGGLVKPGGSLKLSCAASGITFSRFAMSWIRQTPEKRLEWVATISSGDMYTYYPDSVKGR<br>TISRDNANNTLYLQMRSLRSEDTAMYYCAIMITTGEHAYWGQGTLVTVSA (SEQ ID NO: 67) |
| --- |

| EIKMTQSPSSLSASLGDTITITCRASQDISVWLTWYQQKPGNIPKLLIYKASNLHTGVPPRFSGSGSGTI<br>FTFTISSLQPEDITTYYCLQSQSYPWTFGGGTKLEIK (SEQ ID NO: 68)<br><br>VL of mouse and chimeric 6G10 antibodies |
| --- |

| Human IgG1 heavy chain constant region<br><br>ASTKGPSVFPLAPSSKSTSGGTAALGCLVKDYFPEPVTVSWNSGALTSGVHTFPAVLQSSGLYSLSSVV<br>TVPSSSLGTQTYICNVNHKPSNTKVDKRVEPKSCDKTHTCPPCPAPEAAGGPSVFLFPPKPKDTLMISR<br>TPEVTCVVVDVSHEDPEVKFNWYVDGVEVHNAKTKPREEQYNSTYRVVSVLTVLHQDWLNGKEYK<br>CKVSNKALPAPIEKTISKAKGQPREPQVYTLPPSREEMTKNQVSLTCLVKGFYPSDIAVEWESNGQPEN<br>NYKTTPPVLDSDGSFFLYSKLTVDKSRWQQGNVFSCSVMHEALHNHYTQKSLSLSPGK (SEQ ID NO:<br>69)<br>GCCTCTACCAAGGGCCCCTCCGTGTTCCCTCTGGCTCCAAGCTCTAAGTCCACCAGCGGAGGAAC<br>AGCCGCTCTGGGCTGTCTGGTGAAGGATTATTTCCCAGAGCCCGTGACCGTGTCCTGGAATAGCG<br>GCGCCCTGACCTCCGGAGTGCATACATTTCCCGCTGTGCTGCAGTCCAGCGGCCTGTACAGCCTGT<br>CTTCCGTGGTGACCGTGCCTAGCTCTTCCCTGGGCACCCAGACATATATCTGCAATGTGAACCACA<br>AGCCTTCCAACACAAAGGTGGACAAGAGGGTGGAGCCAAAGAGCTGTGATAAGACCCATACATG<br>CCCCCCTTGTCCTGCTCCAGAGGCTGCTGGAGGACCATCCGTGTTCCTGTTTCCACCCAAGCCCAA<br>GGACACCCTGATGATCTCTAGGACCCCTGAGGTGACATGCGTGGTGGTGGACGTGTCCCACGAGG<br>ATCCAGAGGTGAAGTTTAATTGGTACGTGGATGGCGTGGAGGTGCATAACGCTAAGACCAAGCCT<br>AGGGAGGAGCAGTACAACAGCACCTATCGGGTGGTGTCTGTGCTGACAGTGCTGCACCAGGACTG<br>GCTGAATGGCAAGGAGTATAAGTGCAAGGTGAGCAACAAGGCCCTGCCCGCTCCTATCGAGAAG<br>ACCATCTCTAAGGCCAAGGGCCAGCCCAGAGAGCCTCAGGTGTACACACTGCCTCCAAGCCGCGA<br>GGAGATGACCAAGAATCAGGTGTCTCTGACATGTCTGGTGAAGGGCTTCTATCCATCTGACATCG<br>CTGTGGAGTGGGAGTCCAACGGCCAGCCCGAGAACAATTACAAGACCACACCCCCTGTGCTGGAC<br>TCTGATGGCTCCTTCTTTCTGTATTCCAAGCTGACCGTGGATAAGAGCCGGTGGCAGCAGGGCAA<br>CGTGTTCTCCTGTTCCGTGATGCATGAGGCCCTGCACAACCATTACACACAGAAGAGCCTGTCTCT<br>GTCCCCAGGCAAG (SEQ ID NO: 70) |
| --- |

| Human IgG4 heavy chain constant region<br><br>ASTKGPSVFPLAPCSRSTSESTAALGCLVKDYFPEPVTVSWNSGALTSGVHTFPAVLQSSGLYSLSSVV<br>TVPSSSLGTKTYTCNVDHKPSNTKVDKRVESKYGPPCPPCPAPEFLGGPSVFLFPPKPKDTLMISRTPEV<br>TCVVVDVSQEDPEVQFNWYVDGVEVHNAKTKPREEQFNSTYRVVSVLTVLHQDWLNGKEYKCKVS<br>NKGLPSSIEKTISKAKGQPREPQVYTLPPSQEEMTKNQVSLTCLVKGFYPSDIAVEWESNGQPENNYKT<br>TPPVLDSDGSFFLYSRLTVDKSRWQEGNVFSCSVMHEALHNHYTQKSLSLSLGK (SEQ ID NO: 71)<br>GCCAGCACAAAGGGACCATCCGTGTTCCCACTGGCTCCATGCTCCCGGAGCACCTCTGAGTCCAC<br>AGCCGCTCTGGGCTGTCTGGTGAAGGACTACTTCCCTGAGCCAGTGACCGTGTCCTGGAATAGCG<br>GCGCCCTGACCAGCGGAGTGCACACATTTCCTGCTGTGCTGCAGAGCTCTGGCCTGTACTCTCTGT<br>CCAGCGTGGTGACCGTGCCATCTTCCAGCCTGGGCACAAAGACCTATACATGCAACGTGGACCAT<br>AAGCCCTCCAATACAAAGGTGGATAAGAGAGTGGAGAGCAAGTACGGACCACCTTGCCCACCAT<br>GTCCAGCTCCTGAGTTTCTGGGAGGACCATCCGTGTTCCTGTTTCCTCCAAAGCCTAAGGACACCC<br>TGATGATCTCCAGGACCCCAGAGGTGACATGCGTGGTGGTGGACGTGAGCCAGGAGGATCCTGA<br>GGTGCAGTTCAACTGGTACGTGGATGGCGTGGAGGTGCACAATGCTAAGACCAAGCCTAGAGAG<br>GAGCAGTTTAACTCCACCTACCGCGTGGTGAGCGTGCTGACAGTGCTGCATCAGGATTGGCTGAA<br>CGGCAAGGAGTATAAGTGCAAGGTGTCTAATAAGGGCCTGCCATCTTCCATCGAGAAGACCATCT<br>CCAAGGCCAAGGGCCAGCCTAGGGAGCCACAGGTGTACACACTGCCCCCTTCTCAGGAGGAGAT<br>GACCAAGAACCAGGTGTCCCTGACATGTCTGGTGAAGGGCTTCTATCCTAGCGACATCGCTGTGG<br>AGTGGGAGTCTAATGGCCAGCCAGAGAACAATTACAAGACCACACCACCCGTGCTGGACTCTGAT<br>GGCTCCTTCTTTCTGTATTCTAGGCTGACCGTGGATAAGAGTCCCGGTGGCAGGAGGGCAACGTGTTT<br>AGCTGCTCTGTGATGCATGAGGCTCTGCACAATCATTACACACAGAAGTCCCTGAGCCTGTCTCTG<br>GGCAAG (SEQ ID NO: 72) |
| --- |

(continued)

| Human κ light chain constant region |
| --- |
| RTVAAPSVFIFPPSDEQLKSGTASVVCLLNNFYPREAKVQWKVDNALQSGNSQESVTEQDSKDSTYSL |

| STLTLSKADYEKHKVYACEVTHQGLSSPVTKSFNRGEC (SEQ ID NO: 73) CGCACAGTGGCCGCTCCAAGCGTGTTCATCTTTCCCCCTTCTGACGAGCAGCTGAAGTCTGGCACC GCTTCCGTGGTGTGCCTGCTGAACAATTTCTACCCCCGCGAGGCCAAGGTGCAGTGGAAGGTGGA AACGCTCTGCAGTCCGGCAATAGCCAGGAGTCTGTGACAGAGCAGGACTCCAAGGATAGCACCTA TTCTCTGTCCAGCACACTGACCCTGTCTAAGGCCGATTACGAGAAGCACAAGGTGTATGCTTGCGA GGTGACACATCAGGGCCTGTCTTCCCCAGTGACCAAGTCCTTTAACAGGGGCGAGTGT (SEQ II NO: 74) |

| Human IL-36R (hIL-36R) |
| --- |
| MWSLLLCGLSIALPLSVTADGCKDIFMKNEILSASQPFAFNCTFPPITSGEVSVTWYKNSSKIPVSKIIQS RIHQDETWILFLPMEWGDSGVYQCVIKGRDSCHRIHVNLTVFEKHWCDTSIGGLPNLSDEYKQILHLG KDDSLTCHLHFPKSCVLGPIKWYKDCNEIKGERFTVLETRLLVSNVSAEDRGNYACQAILTHSGKQYE VLNGITVSITERAGYGGSVPKIIYPKNHSIEVQLGTTLIVDCNVTDTKDNTNLRCWRVNNTLVDDYYDI SKRIREGVETHVSFREHNLYTVNITFLEVKMEDYGLPFMCHAGVSTAYIILQLPAPDFRAYLIGGLIALV/ VAVSVVYIYNIFKIDIVLWYRSAFHSTETIVDGKLYDAYVLYPKPHKESQRHAVDALVLNILPEVLERQC GYKLFIFGRDEFPGQAVANVIDENVKLCRRLIVIVVPESLGFGLLKNLSEEQIAVYSALIQDGMKVILIEL EKIEDYTVMPESIQYIKQKHGAIRWHGDFTEQSQCMKTKFWKTVRYHMPPRRCRPFPPVQLLQHTPCY RTAGPELGSRRKKCTLTTG (SEQ ID NO: 75) |

| Extracellular region of human IL36R protein with mouse antibody heavy chain Fc (hIL36R-ECD mFc) |
| --- |
| DGCKDIFMKNEILSASQPFAFNCTFPPITSGEVSVTWYKNSSKIPVSKIIQSRIHQDETWILFLPMEWGDS GVYQCVIKGRDSCHRIHVNLTVFEKHWCDTSIGGLPNLSDEYKQILHLGKDDSLTCHLHFPKSCVLGPI KWYKDCNEIKGERFTVLETRLLVSNVSAEDRGNYACQAILTHSGKQYEVLNGITVSITERAGYGGSVP KIIYPKNHSIEVQLGTTLIVDCNVTDTKDNTNLRCWRVNNTLVDDYYDESKRIREGVETHVSFREHNLY TVNITFLEVKMEDYGLPFMCHAGVSTAYIILQLPAPDFRIEGRMDEPRGPTIKPCPPCKCPAPNLLGGPS VFIFPPKIKDVLMISLSPIVTCVVVDVSEDDPDVQISWFVNNVEVHTAQTQTHREDYNSTLRVVSALPIQ HQDWMSGKEFKCKVNNKDLPAPIERTISKPKGSVRAPQVYVLPPPEEEMTKKQVTLTCMVTDFMPED YVEWTNNGKTELNYKNTEPVLDSDGSYFMYSKLRVEKKNWVERNSYSCSVVHEGLHNHHTTKSFSR TPGK (SEQ ID NO: 76) |

| Extracellular region of human IL36R protein with human antibody heavy chain Fc (hIL36R-ECD hFc) |
| --- |
| DGCKDIFMKNEILSASQPFAFNCTFPPITSGEVSVTWYKNSSKIPVSKIIQSRIHQDETWILFLPMEWGDS GVYQCVIKGRDSCHRIHVNLTVFEKHWCDTSIGGLPNLSDEYKQILHLGKDDSLTCHLHFPKSCVLGPI KWYKDCNEIKGERFTVLETRLLVSNVSAEDRGNYACQAILTHSGKQYEVLNGITVSITERAGYGGSVP KIIYPKNHSIEVQLGTTLIVDCNVTDTKDNTNLRCWRVNNTLVDDYYDESKRIREGVETHVSFREHNLY TVNITFLEVKMEDYGLPFMCHAGVSTAYIILQLPAPDFRIEGRMDPKSCDKTHTCPPCPAPELLGGPSVF LFPPKPKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHNHYTQKSLSLS PGK (SEQ ID NO: 77) |

| Cynomolgus monkey IL-36R (CynoIL-36R) |
| --- |
| MWSLLLCGLSIALPLSVTADGCNDIFMKNEMLSASQPFAFNCTFPPITSGEVSVTWYKNSSKIPVSKIIQS RIHQDETWILFLPMEWGDSGVYQCVIKGRDSCHRIHVNLTVFEKQWCDTSVKGLPNLSDEYKQILHLC KDDSLTCHLHFPKSCILGPIKWYKDCNEITGERFTVLETRLLVSNVSAEDRGNYACQVILTHSGKQYEV LNGITVSITERAGYGGSVPKIIYPKNNSIEVQLGTTLIVDCNITDTKDNTNLRCWRVNNTLVDDYYNES KRIREGVETHASFREYNLYTVNITFLEVKMEDYGLPFMCHAGVSAAYIMLQLPAPDFRAYLIGGLIAFL AVAVSVVYIYNIFKIDIVLWYRSTFQSTETLEDGKLYDAYVLYPKPRRESQRHAVDTLVLKILPEVLERQ CGYKLFIFGRDEFPGQAVANVIDENVKLCRRLIVIVVPESLGFGLLKNLSEEQIAVYNALIQDGMKVILIE LEKIEDYTAMPESIQYIRQKHGVIRWHGDFTEQSQCVKTKFWKTVRYHMPPRRCRPSPPVQLPQHTPC YRTTGERVGGHKVRHALMEGLSLRGGSQLEEDTFHRGRPL (SEQ ID NO: 78) |

(continued)

| |
|---|
| Extracellular region of cynomolgus monkey IL36R protein with mouse antibody heavy chain Fc (CynoIL36R-ECD mFc) |
| DGCNDIFMKNEMLSASQPFAFNCTFPPITSGEVSVTWYKNSSKIPVSKIIQSRIHQDETWILFLPMEWGD SGVYQCVIKGRDSCHRIHVNLTVFEKQWCDTSVKGLPNLSDEYKQILHLGKDDSLTCHLHFPKSCILG IKWYKDCNEITGERFTVLETRLLVSNVSAEDRGNYACQVILTHSGKQYEVLNGITVSITERAGYGGSVF KIIYPKNNSIEVQLGTTLIVDCNITDTKDNTNLRCWRVNNTLVDDYYNESKRIREGVETHASFREYNLY TVNITFLEVKMEDYGLPFMCHAGVSAAYIMLQLPAPDFRIEGRMDEPRGPTIKPCPPCKPCAPNLLGGP SVFIFPPKIKDVLMISLSPIVTCVVVDVSEDDPDVQISWFVNNVEVHTAQTQTHREDYNSTLRVVSALP QHQDWMSGKEFKCKVNNKDLPAPIERTISKPKGSVRAPQVYVLPPPEEEMTKKQVTLTCMVTDFMPE DIYVEWTNNGKTELNYKNTEPVLDSDGSYFMYSKLRVEKKNWVERNSYSCSVVHEGLHNHHTTKSF SRTPGK (SEQ ID NO: 79) |
| Extracellular region of cynomolgus monkey IL36R protein with human antibody heavy chain Fc (CynoIL36R-ECD hFc) |
| DGCNDIFMKNEMLSASQPFAFNCTFPPITSGEVSVTWYKNSSKIPVSKIIQSRIHQDETWILFLPMEWGD SGVYQCVIKGRDSCHRIHVNLTVFEKQWCDTSVKGLPNLSDEYKQILHLGKDDSLTCHLHFPKSCILGF IKWYKDCNEITGERFTVLETRLLVSNVSAEDRGNYACQVILTHSGKQYEVLNGITVSITERAGYGGSVP KIIYPKNNSIEVQLGTTLIVDCNITDTKDNTNLRCWRVNNTLVDDYYNESKRIREGVETHASFREYNLY TVNITFLEVKMEDYGLPFMCHAGVSAAYIMLQLPAPDFRIEGRMDPKSCDKTHTCPPCPAPELLGGPSV FLFPPKPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVMHEALHNHYTQKSLSI SPGK (SEQ ID NO: 80) |
| Heavy chain of BI655130 |
| QVQLVQSGAEVKKPGASVKVSCKASGYSFTSSWIHWVKQAPGQGLEWMGEINPGNVRTNYNENFRN KVTMTVDTSISTAYMELSRLRSDDTAVYYCTVVFYGEPYFPYWGQGTLVTVSSASTKGPSVFPLAPSSK STSGGTAALGCLVKDYFPEPVTVSWNSGALTSGVHTFPAVLQSSGLYSLSSVVTVPSSSLGTQTYICNVN HKPSNTKVDKRVEPKSCDKTHTCPPCPAPEAAGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO: 81) CAGGTGCAGCTGGTGCAGTCTGGCGCCGAGGTGAAGAAGCCAGGCGCTAGCGTGAAGGTGTCTTG CAAGGCCAGCGGCTACTCTTTCACCTCCAGCTGGATCCACTGGGTGAAGCAGGCTCCAGGACAGG GACTGGAGTGGATGGGCGAGATCAATCCTGGCAACGTGAGAACAAACTACAATGAGAACTTTCGC AATAAGGTGACCATGACAGTGGACACCAGCATCTCTACAGCCTATATGGAGCTGTCCAGGCTGCGG AGCGACGATACCGCCGTGTACTATTGCACAGTGGTGTTCTACGGCGAGCCATACTTTCCCTATTGGG GCCAGGGCACCCTGGTGACAGTGTCTTCCGCCTCTACCAAGGGCCCCTCCGTGTTCCCTCTGGCTC CAAGCTCTAAGTCCACCAGCGGAGGAACAGCCGCTCTGGGCTGTCTGGTGAAGGATTATTTCCCAG AGCCCGTGACCGTGTCCTGGAATAGCGGCGCCCTGACCTCCGGAGTGCATACATTTCCCGCTGTGC TGCAGTCCAGCGGCCTGTACAGCCTGTCTTCCGTGGTGACCGTGCCTAGCTCTTCCCTGGGCACCC AGACATATATCTGCAATGTGAACCACAAGCCTTCCAACACAAAGGTGGACAAGAGGGTGGAGCCA AAGAGCTGTGATAAGACCCATACATGCCCCCCTTGTCCTGCTCCAGAGGCTGCTGGAGGACCATCC GTGTTCCTGTTTCCACCCAAGCCCAAGGACACCCTGATGATCTCTAGGACCCCTGAGGTGACATGC GTGGTGGTGGACGTGTCCCACGAGGATCCAGAGGTGAAGTTTAATTGGTACGTGGATGGCGTGGA GGTGCATAACGCTAAGACCAAGCCTAGGGAGGAGCAGTACAACAGCACCTATCGGGTGGTGTCTG TGCTGACAGTGCTGCACCAGGACTGGCTGAATGGCAAGGAGTATAAGTGCAAGGTGAGCAACAAG GCCCTGCCCGCTCCTATCGAGAAGACCATCTCTAAGGCCAAGGGCCAGCCCAGAGAGCCTCAGGT GTACACACTGCCTCCAAGCCGCGAGGAGATGACCAAGAATCAGGTGTCTCTGACATGTCTGGTGA AGGGCTTCTATCCATCTGACATCGCTGTGGAGTGGGAGTCCAACGGCCAGCCCGAGAACAATTACA AGACCACACCCCCTGTGCTGGACTCTGATGGCTCCTTCTTTCTGTATTCCAAGCTGACCGTGGATAA GAGCCGGTGGCAGCAGGGCAACGTGTTCTCCTGTTCCGTGATGCATGAGGCCCTGCACAACCATTA CACACAGAAGAGCCTGTCTCTGTCCCCAGGCAAG (SEQ ID NO: 82) |
| Light chain of BI655130 |

(continued)

| |
|---|
| QIVLTQSPGTLSLSPGERATMTCTASSSVSSSYFHWYQQKPGQAPRLWIYRTSRLASGVPDRFSGSGSGT DFTLTISRLEPEDAATYYCHQFHRSPLTFGAGTKLEIKRTVAAPSVFIFPPSDEQLKSGTASVVCLLNNFY PREAKVQWKVDNALQSGNSQESVTEQDSKDSTYSLSSTLTLSKADYEKHKVYACEVTHQGLSSPVTK SFNRGEC (SEQ ID NO: 83) CAGATCGTGCTGACACAGAGCCCTGGCACCCTGAGCCTGTCTCCAGGAGAGAGGGCCACCATGAC ATGCACCGCTTCCAGCTCCGTGTCCAGCTCTTACTTCCACTGGTATCAGCAGAAGCCAGGACAGGC TCCAAGGCTGTGGATCTACAGGACCTCCAGGCTGGCTAGCGGAGTGCCTGACCGGTTCTCCGGAA GCGGATCTGGCACAGACTTCACCCTGACCATCTCCAGACTGGAGCCCGAGGACGCCGCTACCTAC ATTGCCACCAGTTCCATAGAAGCCCTCTGACATTTGGCGCCGGCACCAAGCTGGAGATCAAGCGCA CAGTGGCCGCTCCAAGCGTGTTCATCTTTCCCCCTTCTGACGAGCAGCTGAAGTCTGGCACCGCTT CCGTGGTGTGCCTGCTGAACAATTTCTACCCCCGCGAGGCCAAGGTGCAGTGGAAGGTGGATAAC GCTCTGCAGTCCGGCAATAGCCAGGAGTCTGTGACAGAGCAGGACTCCAAGGATAGCACCTATTC CTGTCCAGCACACTGACCCTGTCTAAGGCCGATTACGAGAAGCACAAGGTGTATGCTTGCGAGGTC ACACATCAGGGCCTGTCTTCCCCAGTGACCAAGTCCTTTAACAGGGGCGAGTGT (SEQ ID NO: 84) |
| Kabat-defined VH-CDR1 of mouse, chimeric and humanized 72C1 antibodies SGYWN (SEQ ID NO: 85) |
| Kabat-defined VH-CDR1 of mouse, chimeric and humanized 84C4 antibodies |
| SYWMN (SEQ ID NO: 86) |
| Kabat-defined VL-CDR1 of mouse, chimeric and humanized 84C4 antibodies RASQDIGNALN (SEQ ID NO: 87) |
| Kabat-defined VH-CDR1 of mouse and chimeric 74B4D6 antibodies NYWIH (SEQ ID NO: 88) |
| Kabat-defined VH-CDR1 of mouse and chimeric 58A8D1 antibodies SSWMH (SEQ ID NO: 89) |
| Kabat-defined VH-CDR1 of mouse and chimeric 6G10 antibodies RFAMS (SEQ ID NO: 90) |

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

**Claims**

1. An isolated anti-IL-36R antibody or an antigen-binding fragment thereof, wherein the antibody or the antigen-binding fragment thereof comprises:

   (i) heavy chain CDR1,heavy chain CDR2 and heavy chain CDR3, wherein

   (1) heavy chain CDR1 comprises an amino acid sequence set forth in SEQ ID NO: 1 or 85 or an amino acid sequence having at least 80% identity to SEQ ID NO: 1 or 85, heavy chain CDR2 comprises an amino acid sequence set forth in SEQ ID NO: 2 or an amino acid sequence having at least 80% identity to SEQ ID NO: 2, and heavy chain CDR3 comprises an amino acid sequence set forth in SEQ ID NO: 3 or an amino acid sequence having at least 80% identity to SEQ ID NO: 3;
   (2) heavy chain CDR1 comprises an amino acid sequence set forth in SEQ ID NO: 21 or 86 or an amino acid sequence having at least 80% identity to SEQ ID NO: 21 or 86, heavy chain CDR2 comprises an amino acid sequence set forth in SEQ ID NO: 22 or an amino acid sequence having at least 80% identity to SEQ ID NO: 22, and heavy chain CDR3 comprises an amino acid sequence set forth in SEQ ID NO: 23 or an amino acid sequence having at least 80% identity to SEQ ID NO: 23;
   (3) heavy chain CDR1 comprises an amino acid sequence set forth in SEQ ID NO: 45 or 88 or an amino acid sequence having at least 80% identity to SEQ ID NO: 45 or 88, heavy chain CDR2 comprises an amino acid sequence set forth in SEQ ID NO: 46 or an amino acid sequence having at least 80% identity to SEQ ID NO: 46, and heavy chain CDR3 comprises an amino acid sequence set forth in SEQ ID NO: 47 or an amino acid sequence having at least 80% identity to SEQ ID NO: 47;

(4) heavy chain CDR1 comprises an amino acid sequence set forth in SEQ ID NO: 53 or 89 or an amino acid sequence having at least 80% identity to SEQ ID NO: 53 or 89, heavy chain CDR2 comprises an amino acid sequence set forth in SEQ ID NO: 54 or an amino acid sequence having at least 80% identity to SEQ ID NO: 54, and heavy chain CDR3 comprises an amino acid sequence set forth in SEQ ID NO: 55 or an amino acid sequence having at least 80% identity to SEQ ID NO: 55; or

(5) heavy chain CDR1 comprises an amino acid sequence set forth in SEQ ID NO: 61 or 90 or an amino acid sequence having at least 80% identity to SEQ ID NO: 61 or 90, heavy chain CDR2 comprises an amino acid sequence set forth in SEQ ID NO: 62 or an amino acid sequence having at least 80% identity to SEQ ID NO: 62, and heavy chain CDR3 comprises an amino acid sequence set forth in SEQ ID NO: 63 or an amino acid sequence having at least 80% identity to SEQ ID NO: 63;

(ii) light chain CDR1, light chain CDR2 and light chain CDR3, wherein

(1) light chain CDR1 comprises an amino acid sequence set forth in SEQ ID NO: 4 or an amino acid sequence having at least 80% identity to SEQ ID NO: 4, light chain CDR2 comprises an amino acid sequence set forth in SEQ ID NO: 5 or an amino acid sequence having at least 80% identity to SEQ ID NO: 5, and light chain CDR3 comprises an amino acid sequence set forth in SEQ ID NO: 6 or an amino acid sequence having at least 80% identity to SEQ ID NO: 6;

(2) light chain CDR1 comprises an amino acid sequence set forth in SEQ ID NO: 24 or 87 or an amino acid sequence having at least 80% identity to SEQ ID NO: 24 or 87, light chain CDR2 comprises an amino acid sequence set forth in SEQ ID NO: 25 or an amino acid sequence having at least 80% identity to SEQ ID NO: 25, and light chain CDR3 comprises an amino acid sequence set forth in SEQ ID NO: 26 or an amino acid sequence having at least 80% identity to SEQ ID NO: 26;

(3) light chain CDR1 comprises an amino acid sequence set forth in SEQ ID NO: 48 or an amino acid sequence having at least 80% identity to SEQ ID NO: 48, light chain CDR2 comprises an amino acid sequence set forth in SEQ ID NO: 49 or an amino acid sequence having at least 80% identity to SEQ ID NO: 49, and light chain CDR3 comprises an amino acid sequence set forth in SEQ ID NO: 50 or an amino acid sequence having at least 80% identity to SEQ ID NO: 50;

(4) light chain CDR1 comprises an amino acid sequence set forth in SEQ ID NO: 56 or an amino acid sequence having at least 80% identity to SEQ ID NO: 56, light chain CDR2 comprises an amino acid sequence set forth in SEQ ID NO: 57 or an amino acid sequence having at least 80% identity to SEQ ID NO: 57, and light chain CDR3 comprises an amino acid sequence set forth in SEQ ID NO: 58 or an amino acid sequence having at least 80% identity to SEQ ID NO: 58; or

(5) light chain CDR1 comprises an amino acid sequence set forth in SEQ ID NO: 64 or an amino acid sequence having at least 80% identity to SEQ ID NO: 64, light chain CDR2 comprises an amino acid sequence set forth in SEQ ID NO: 65 or an amino acid sequence having at least 80% identity to SEQ ID NO: 65, and light chain CDR3 comprises an amino acid sequence set forth in SEQ ID NO: 66 or an amino acid sequence having at least 80% identity to SEQ ID NO: 66; or

(iii) heavy chain CDR1, heavy chain CDR2 and heavy chain CDR3 set forth in (i), and light chain CDR1, light chain CDR2 and light chain CDR3 set forth in (ii),
wherein

the amino acid sequence of SEQ ID NO: 2 is $YIX_1YSGYTYYNPSLKS$, the amino acid sequence of SEQ ID NO: 6 is $QQX_2TTSPYT$, wherein $X_1$ = S or Y, and $X_2$ = F or Y;
and the amino acid sequence of SEQ ID NO: 21 is $GYX_3FTSYWMN$, the amino acid sequence of SEQ ID NO: 22 is $X_4IX_5PYDSETRX_6X_7QKFX_8G$, wherein $X_3$ = F or Y, $X_4$ = M or Y, $X_5$ = H or Y, $X_6$ = L or Y, $X_7$ = N or A, and $X_8$ = K or Q;
preferably, the amino acid sequence of SEQ ID NO: 2 is $YIX_1YSGYTYYNPSLKS$, the amino acid sequence of SEQ ID NO: 6 is $QQX_2TTSPYT$, wherein i) $X_1$ = S or Y, $X_2$ = F, or ii) $X_1$ = S or Y, $X_2$ = Y;
and the amino acid sequence of SEQ ID NO: 21 is $GYX_3FTSYWMN$, the amino acid sequence of SEQ ID NO: 22 is $X_4IX_5PYDSETRX_6X_7QKFX_8G$, wherein i) $X_3$ = F or Y, $X_4$ = M or Y, $X_5$ = H or Y, $X_6$ = L, $X_7$ = N or A, and $X_8$ = K, or ii) $X_3$ = F or Y, $X_4$ = M or Y, $X_5$ = H or Y, $X_6$ = Y, $X_7$ = N or A, and $X_8$ = Q.

2. The antibody or the antigen-binding fragment thereof according to claim 1, wherein the antibody or the antigen-binding fragment thereof comprises heavy chain CDR1, heavy chain CDR2, heavy chain CDR3, light chain CDR1, light chain CDR2, and light chain CDR3, wherein

(1) heavy chain CDR1 comprises an amino acid sequence set forth in SEQ ID NO: 1 or 85 or an amino acid sequence having at least 80% identity to SEQ ID NO: 1 or 85, heavy chain CDR2 comprises an amino acid sequence set forth in SEQ ID NO: 2 or an amino acid sequence having at least 80% identity to SEQ ID NO: 2, heavy chain CDR3 comprises an amino acid sequence set forth in SEQ ID NO: 3 or an amino acid sequence having at least 80% identity to SEQ ID NO: 3, light chain CDR1 comprises an amino acid sequence set forth in SEQ ID NO: 4 or an amino acid sequence having at least 80% identity to SEQ ID NO: 4, light chain CDR2 comprises an amino acid sequence set forth in SEQ ID NO: 5 or an amino acid sequence having at least 80% identity to SEQ ID NO: 5, and light chain CDR3 comprises an amino acid sequence set forth in SEQ ID NO: 6 or an amino acid sequence having at least 80% identity to SEQ ID NO: 6;
(2) heavy chain CDR1 comprises an amino acid sequence set forth in SEQ ID NO: 21 or 86 or an amino acid sequence having at least 80% identity to SEQ ID NO: 21 or 86, heavy chain CDR2 comprises an amino acid sequence set forth in SEQ ID NO: 22 or an amino acid sequence having at least 80% identity to SEQ ID NO: 22, heavy chain CDR3 comprises an amino acid sequence set forth in SEQ ID NO: 23 or an amino acid sequence having at least 80% identity to SEQ ID NO: 23, light chain CDR1 comprises an amino acid sequence set forth in SEQ ID NO: 24 or 87 or an amino acid sequence having at least 80% identity to SEQ ID NO: 24 or 87, light chain CDR2 comprises an amino acid sequence set forth in SEQ ID NO: 25 or an amino acid sequence having at least 80% identity to SEQ ID NO: 25, and light chain CDR3 comprises an amino acid sequence set forth in SEQ ID NO: 26 or an amino acid sequence having at least 80% identity to SEQ ID NO: 26;
(3) heavy chain CDR1 comprises an amino acid sequence set forth in SEQ ID NO: 45 or 88 or an amino acid sequence having at least 80% identity to SEQ ID NO: 45 or 88, heavy chain CDR2 comprises an amino acid sequence set forth in SEQ ID NO: 46 or an amino acid sequence having at least 80% identity to SEQ ID NO: 46, heavy chain CDR3 comprises an amino acid sequence set forth in SEQ ID NO: 47 or an amino acid sequence having at least 80% identity to SEQ ID NO: 47, light chain CDR1 comprises an amino acid sequence set forth in SEQ ID NO: 48 or an amino acid sequence having at least 80% identity to SEQ ID NO: 48, light chain CDR2 comprises an amino acid sequence set forth in SEQ ID NO: 49 or an amino acid sequence having at least 80% identity to SEQ ID NO: 49, and light chain CDR3 comprises an amino acid sequence set forth in SEQ ID NO: 50 or an amino acid sequence having at least 80% identity to SEQ ID NO: 50;
(4) heavy chain CDR1 comprises an amino acid sequence set forth in SEQ ID NO: 53 or 89 or an amino acid sequence having at least 80% identity to SEQ ID NO: 53 or 89, heavy chain CDR2 comprises an amino acid sequence set forth in SEQ ID NO: 54 or an amino acid sequence having at least 80% identity to SEQ ID NO: 54, heavy chain CDR3 comprises an amino acid sequence set forth in SEQ ID NO: 55 or an amino acid sequence having at least 80% identity to SEQ ID NO: 55, light chain CDR1 comprises an amino acid sequence set forth in SEQ ID NO: 56 or an amino acid sequence having at least 80% identity to SEQ ID NO: 56, light chain CDR2 comprises an amino acid sequence set forth in SEQ ID NO: 57 or an amino acid sequence having at least 80% identity to SEQ ID NO: 57, and light chain CDR3 comprises an amino acid sequence set forth in SEQ ID NO: 58 or an amino acid sequence having at least 80% identity to SEQ ID NO: 58; or
(5) heavy chain CDR1 comprises an amino acid sequence set forth in SEQ ID NO: 61 or 90 or an amino acid sequence having at least 80% identity to SEQ ID NO: 61 or 90, heavy chain CDR2 comprises an amino acid sequence set forth in SEQ ID NO: 62 or an amino acid sequence having at least 80% identity to SEQ ID NO: 62, heavy chain CDR3 comprises an amino acid sequence set forth in SEQ ID NO: 63 or an amino acid sequence having at least 80% identity to SEQ ID NO: 63, light chain CDR1 comprises an amino acid sequence set forth in SEQ ID NO: 64 or an amino acid sequence having at least 80% identity to SEQ ID NO: 64, light chain CDR2 comprises an amino acid sequence set forth in SEQ ID NO: 65 or an amino acid sequence having at least 80% identity to SEQ ID NO: 65, and light chain CDR3 comprises an amino acid sequence set forth in SEQ ID NO: 66 or an amino acid sequence having at least 80% identity to SEQ ID NO: 66;
wherein

the amino acid sequence of SEQ ID NO: 2 is $YIX_1YSGYTYYNPSLKS$, the amino acid sequence of SEQ ID NO: 6 is $QQX_2TTSPYT$, wherein

i)

$$X_1 = S \text{ or } Y, X_2 = F,$$

or

ii)

$$X_1 = S \text{ or } Y, X_2 = Y;$$

and the amino acid sequence of SEQ ID NO: 21 is GYX$_3$FTSYWMN, the amino acid sequence of SEQ ID NO: 22 is X$_4$IX$_5$PYDSETRX$_6$X$_7$QKFX$_8$G, wherein

i)

$$X_3 = F \text{ or } Y, X_4 = M \text{ or } Y, X_5 = H \text{ or } Y, X_6 = L, X_7 = N \text{ or } A, \text{ and } X_8 = K,$$

or

ii)

$$X_3 = F \text{ or } Y, X_4 = M \text{ or } Y, X_5 = H \text{ or } Y, X_6 = Y, X_7 = N \text{ or } A, \text{ and } X_8 = Q.$$

3. An isolated anti-IL-36R antibody or an antigen-binding fragment thereof, wherein the antibody or the antigen-binding fragment thereof comprises:

(1) heavy chain CDR1, heavy chain CDR2 and heavy chain CDR3 in a heavy chain variable region set forth in SEQ ID NO: 7, and light chain CDR1, light chain CDR2 and light chain CDR3 in a light chain variable region set forth in SEQ ID NO: 15;
(2) heavy chain CDR1, heavy chain CDR2 and heavy chain CDR3 in a heavy chain variable region set forth in SEQ ID NO: 9, and light chain CDR1, light chain CDR2 and light chain CDR3 in a light chain variable region set forth in SEQ ID NO: 17;
(3) heavy chain CDR1, heavy chain CDR2 and heavy chain CDR3 in a heavy chain variable region set forth in SEQ ID NO: 11, and light chain CDR1, light chain CDR2 and light chain CDR3 in a light chain variable region set forth in SEQ ID NO: 17;
(4) heavy chain CDR1, heavy chain CDR2 and heavy chain CDR3 in a heavy chain variable region set forth in SEQ ID NO: 13, and light chain CDR1, light chain CDR2 and light chain CDR3 in a light chain variable region set forth in SEQ ID NO: 17;
(5) heavy chain CDR1, heavy chain CDR2 and heavy chain CDR3 in a heavy chain variable region set forth in SEQ ID NO: 9, and light chain CDR1, light chain CDR2 and light chain CDR3 in a light chain variable region set forth in SEQ ID NO: 19;
(6) heavy chain CDR1, heavy chain CDR2 and heavy chain CDR3 in a heavy chain variable region set forth in SEQ ID NO: 11, and light chain CDR1, light chain CDR2 and light chain CDR3 in a light chain variable region set forth in SEQ ID NO: 19;
(7) heavy chain CDR1, heavy chain CDR2 and heavy chain CDR3 in a heavy chain variable region set forth in SEQ ID NO: 13, and light chain CDR1, light chain CDR2 and light chain CDR3 in a light chain variable region set forth in SEQ ID NO: 19;
(8) heavy chain CDR1, heavy chain CDR2 and heavy chain CDR3 in a heavy chain variable region set forth in SEQ ID NO: 27, and light chain CDR1, light chain CDR2 and light chain CDR3 in a light chain variable region set forth in SEQ ID NO: 39;
(9) heavy chain CDR1, heavy chain CDR2 and heavy chain CDR3 in a heavy chain variable region set forth in SEQ ID NO: 29, and light chain CDR1, light chain CDR2 and light chain CDR3 in a light chain variable region set forth in SEQ ID NO: 41;
(10) heavy chain CDR1, heavy chain CDR2 and heavy chain CDR3 in a heavy chain variable region set forth in SEQ ID NO: 31, and light chain CDR1, light chain CDR2 and light chain CDR3 in a light chain variable region set forth in SEQ ID NO: 41;
(11) heavy chain CDR1, heavy chain CDR2 and heavy chain CDR3 in a heavy chain variable region set forth in SEQ ID NO: 33, and light chain CDR1, light chain CDR2 and light chain CDR3 in a light chain variable region set forth in SEQ ID NO: 41;
(12) heavy chain CDR1, heavy chain CDR2 and heavy chain CDR3 in a heavy chain variable region set forth in SEQ ID NO: 35, and light chain CDR1, light chain CDR2 and light chain CDR3 in a light chain variable region set forth in SEQ ID NO: 41;

(13) heavy chain CDR1, heavy chain CDR2 and heavy chain CDR3 in a heavy chain variable region set forth in SEQ ID NO: 37, and light chain CDR1, light chain CDR2 and light chain CDR3 in a light chain variable region set forth in SEQ ID NO: 41;

(14) heavy chain CDR1, heavy chain CDR2 and heavy chain CDR3 in a heavy chain variable region set forth in SEQ ID NO: 29, and light chain CDR1, light chain CDR2 and light chain CDR3 in a light chain variable region set forth in SEQ ID NO: 43;

(15) heavy chain CDR1, heavy chain CDR2 and heavy chain CDR3 in a heavy chain variable region set forth in SEQ ID NO: 31, and light chain CDR1, light chain CDR2 and light chain CDR3 in a light chain variable region set forth in SEQ ID NO: 43;

(16) heavy chain CDR1, heavy chain CDR2 and heavy chain CDR3 in a heavy chain variable region set forth in SEQ ID NO: 33, and light chain CDR1, light chain CDR2 and light chain CDR3 in a light chain variable region set forth in SEQ ID NO: 43;

(17) heavy chain CDR1, heavy chain CDR2 and heavy chain CDR3 in a heavy chain variable region set forth in SEQ ID NO: 35, and light chain CDR1, light chain CDR2 and light chain CDR3 in a light chain variable region set forth in SEQ ID NO: 43;

(18) heavy chain CDR1, heavy chain CDR2 and heavy chain CDR3 in a heavy chain variable region set forth in SEQ ID NO: 37, and light chain CDR1, light chain CDR2 and light chain CDR3 in a light chain variable region set forth in SEQ ID NO: 43;

(19) heavy chain CDR1, heavy chain CDR2 and heavy chain CDR3 in a heavy chain variable region set forth in SEQ ID NO: 51, and light chain CDR1, light chain CDR2 and light chain CDR3 in a light chain variable region set forth in SEQ ID NO: 52;

(20) heavy chain CDR1, heavy chain CDR2 and heavy chain CDR3 in a heavy chain variable region set forth in SEQ ID NO: 59, and light chain CDR1, light chain CDR2 and light chain CDR3 in a light chain variable region set forth in SEQ ID NO: 60; or

(21) heavy chain CDR1, heavy chain CDR2 and heavy chain CDR3 in a heavy chain variable region set forth in SEQ ID NO: 67, and light chain CDR1, light chain CDR2 and light chain CDR3 in a light chain variable region set forth in SEQ ID NO: 68.

4. The antibody or the antigen-binding fragment thereof according to any one of claims 1 to 3, wherein the antibody or the antigen-binding fragment thereof comprises:

(1) a heavy chain variable region comprising an amino acid sequence set forth in SEQ ID NO: 7, 9, 11, 13, 27, 29, 31, 33, 35, 37, 51, 59 or 67, or an amino acid sequence having at least 80% identity to the amino acid sequence set forth in SEQ ID NO: 7, 9, 11, 13, 27, 29, 31, 33, 35, 37, 51, 59 or 67;
(2) a light chain variable region comprising an amino acid sequence set forth in SEQ ID NO: 15, 17, 19, 39, 41, 43, 52, 60 or 68, or an amino acid sequence having at least 80% identity to the amino acid sequence set forth in SEQ ID NO: 15, 17, 19, 39, 41, 43, 52, 60 or 68; or
(3) a heavy chain variable region set forth in (1) and a light chain variable region set forth in (2).

5. The antibody or the antigen-binding fragment thereof according to any one of claims 1 to 4, wherein the antibody or the antigen-binding fragment thereof comprises a heavy chain variable region and a light chain variable region, wherein

(1) the heavy chain variable region comprises an amino acid sequence set forth in SEQ ID NO: 7 or an amino acid sequence having at least 80% identity to SEQ ID NO: 7, and the light chain variable region comprises an amino acid sequence set forth in SEQ ID NO: 15 or an amino acid sequence having at least 80% identity to SEQ ID NO: 15;
(2) the heavy chain variable region comprises an amino acid sequence set forth in SEQ ID NO: 9 or an amino acid sequence having at least 80% identity to SEQ ID NO: 9, and the light chain variable region comprises an amino acid sequence set forth in SEQ ID NO: 17 or an amino acid sequence having at least 80% identity to SEQ ID NO: 17;
(3) the heavy chain variable region comprises an amino acid sequence set forth in SEQ ID NO: 11 or an amino acid sequence having at least 80% identity to SEQ ID NO: 11, and the light chain variable region comprises an amino acid sequence set forth in SEQ ID NO: 17 or an amino acid sequence having at least 80% identity to SEQ ID NO: 17;
(4) the heavy chain variable region comprises an amino acid sequence set forth in SEQ ID NO: 13 or an amino acid sequence having at least 80% identity to SEQ ID NO: 13, and the light chain variable region comprises an amino acid sequence set forth in SEQ ID NO: 17 or an amino acid sequence having at least 80% identity to SEQ ID NO: 17;

(5) the heavy chain variable region comprises an amino acid sequence set forth in SEQ ID NO: 9 or an amino acid sequence having at least 80% identity to SEQ ID NO: 9, and the light chain variable region comprises an amino acid sequence set forth in SEQ ID NO: 19 or an amino acid sequence having at least 80% identity to SEQ ID NO: 19;

(6) the heavy chain variable region comprises an amino acid sequence set forth in SEQ ID NO: 11 or an amino acid sequence having at least 80% identity to SEQ ID NO: 11, and the light chain variable region comprises an amino acid sequence set forth in SEQ ID NO: 19 or an amino acid sequence having at least 80% identity to SEQ ID NO: 19;

(7) the heavy chain variable region comprises an amino acid sequence set forth in SEQ ID NO: 13 or an amino acid sequence having at least 80% identity to SEQ ID NO: 13, and the light chain variable region comprises an amino acid sequence set forth in SEQ ID NO: 19 or an amino acid sequence having at least 80% identity to SEQ ID NO: 19;

(8) the heavy chain variable region comprises an amino acid sequence set forth in SEQ ID NO: 27 or an amino acid sequence having at least 80% identity to SEQ ID NO: 27, and the light chain variable region comprises an amino acid sequence set forth in SEQ ID NO: 39 or an amino acid sequence having at least 80% identity to SEQ ID NO: 39;

(9) the heavy chain variable region comprises an amino acid sequence set forth in SEQ ID NO: 29 or an amino acid sequence having at least 80% identity to SEQ ID NO: 29, and the light chain variable region comprises an amino acid sequence set forth in SEQ ID NO: 41 or an amino acid sequence having at least 80% identity to SEQ ID NO: 41;

(10) the heavy chain variable region comprises an amino acid sequence set forth in SEQ ID NO: 31 or an amino acid sequence having at least 80% identity to SEQ ID NO: 31, and the light chain variable region comprises an amino acid sequence set forth in SEQ ID NO: 41 or an amino acid sequence having at least 80% identity to SEQ ID NO: 41;

(11) the heavy chain variable region comprises an amino acid sequence set forth in SEQ ID NO: 33 or an amino acid sequence having at least 80% identity to SEQ ID NO: 33, and the light chain variable region comprises an amino acid sequence set forth in SEQ ID NO: 41 or an amino acid sequence having at least 80% identity to SEQ ID NO: 41;

(12) the heavy chain variable region comprises an amino acid sequence set forth in SEQ ID NO: 35 or an amino acid sequence having at least 80% identity to SEQ ID NO: 35, and the light chain variable region comprises an amino acid sequence set forth in SEQ ID NO: 41 or an amino acid sequence having at least 80% identity to SEQ ID NO: 41;

(13) the heavy chain variable region comprises an amino acid sequence set forth in SEQ ID NO: 37 or an amino acid sequence having at least 80% identity to SEQ ID NO: 37, and the light chain variable region comprises an amino acid sequence set forth in SEQ ID NO: 41 or an amino acid sequence having at least 80% identity to SEQ ID NO: 41;

(14) the heavy chain variable region comprises an amino acid sequence set forth in SEQ ID NO: 29 or an amino acid sequence having at least 80% identity to SEQ ID NO: 29, and the light chain variable region comprises an amino acid sequence set forth in SEQ ID NO: 43 or an amino acid sequence having at least 80% identity to SEQ ID NO: 43;

(15) the heavy chain variable region comprises an amino acid sequence set forth in SEQ ID NO: 31 or an amino acid sequence having at least 80% identity to SEQ ID NO: 31, and the light chain variable region comprises an amino acid sequence set forth in SEQ ID NO: 43 or an amino acid sequence having at least 80% identity to SEQ ID NO: 43;

(16) the heavy chain variable region comprises an amino acid sequence set forth in SEQ ID NO: 33 or an amino acid sequence having at least 80% identity to SEQ ID NO: 33, and the light chain variable region comprises an amino acid sequence set forth in SEQ ID NO: 43 or an amino acid sequence having at least 80% identity to SEQ ID NO: 43;

(17) the heavy chain variable region comprises an amino acid sequence set forth in SEQ ID NO: 35 or an amino acid sequence having at least 80% identity to SEQ ID NO: 35, and the light chain variable region comprises an amino acid sequence set forth in SEQ ID NO: 43 or an amino acid sequence having at least 80% identity to SEQ ID NO: 43;

(18) the heavy chain variable region comprises an amino acid sequence set forth in SEQ ID NO: 37 or an amino acid sequence having at least 80% identity to SEQ ID NO: 37, and the light chain variable region comprises an amino acid sequence set forth in SEQ ID NO: 43 or an amino acid sequence having at least 80% identity to SEQ ID NO: 43;

(19) the heavy chain variable region comprises an amino acid sequence set forth in SEQ ID NO: 51 or an amino acid sequence having at least 80% identity to SEQ ID NO: 51, and the light chain variable region comprises an amino acid sequence set forth in SEQ ID NO: 52 or an amino acid sequence having at least 80% identity to SEQ ID NO: 52;

(20) the heavy chain variable region comprises an amino acid sequence set forth in SEQ ID NO: 59 or an amino acid sequence having at least 80% identity to SEQ ID NO: 59, and the light chain variable region comprises an amino acid sequence set forth in SEQ ID NO: 60 or an amino acid sequence having at least 80% identity to SEQ ID NO: 60; or

(21) the heavy chain variable region comprises an amino acid sequence set forth in SEQ ID NO: 67 or an amino acid sequence having at least 80% identity to SEQ ID NO: 67, and the light chain variable region comprises an amino acid sequence set forth in SEQ ID NO: 68 or an amino acid sequence having at least 80% identity to SEQ ID NO: 68.

6. The antibody or the antigen-binding fragment thereof according to any one of claims 1 to 5, wherein the antibody or the antigen-binding fragment thereof is chimeric or humanized;
optionally, the antibody or the antigen-binding fragment thereof is of the IgG1, IgG2 or IgG4 isotype.

7. The antibody or the antigen-binding fragment thereof according to any one of claims 1 to 6, wherein the antibody or the antigen-binding fragment thereof further comprises a heavy chain constant region and a light chain constant region, wherein the heavy chain constant region comprises an amino acid sequence set forth in SEQ ID NO: 69 or 71 or an amino acid sequence comprising 1, 2, 3, 4 or 5 amino acid substitutions, deletions and additions compared to the amino acid sequence set forth in SEQ ID NO: 69 or 71, and the light chain constant region comprises an amino acid sequence set forth in SEQ ID NO: 73 or an amino acid sequence comprising 1, 2, 3, 4 or 5 amino acid substitutions, deletions and additions compared to the amino acid sequence set forth in SEQ ID NO: 73.

8. The antibody or the antigen-binding fragment thereof according to any one of claims 1 to 7, wherein the antibody or the antigen-binding fragment thereof is selected from a monoclonal antibody, a fusion protein, a recombinant polypeptide, a multispecific antibody, an Fab fragment, an F(ab')$_2$ fragment, an Fd fragment, an Fv fragment, a dAb fragment, an isolated CDR region, a single-chain Fv molecule, or combinations thereof.

9. An isolated antibody or an antigen-binding fragment thereof, wherein the antibody or the antigen-binding fragment thereof binds to the same epitope as the antibody or the antigen-binding fragment thereof according to any one of claims 1 to 8, or competes with the antibody or the antigen-binding fragment thereof according to any one of claims 1 to 8 for binding to IL-36R.

10. An isolated nucleic acid molecule encoding the antibody or the antigen-binding fragment thereof according to any one of claims 1 to 8.

11. An immunoconjugate or a multispecific molecule comprising the antibody or the antigen-binding fragment thereof according to any one of claims 1 to 8.

12. A pharmaceutical composition comprising the antibody or the antigen-binding fragment thereof according to any one of claims 1 to 8, and one or more pharmaceutically acceptable excipients, diluents or carriers.

13. A method for inhibiting IL-36/IL-36R signal transduction in a subject in need, wherein the method comprises administering to the subject a therapeutically effective amount of the antibody or the antigen-binding fragment thereof according to any one of claims 1 to 8, the immunoconjugate or the multispecific molecule according to claim 11, or the pharmaceutical composition according to claim 12.

14. A method for treating IL-36/IL-36R-mediated related diseases and conditions in a subject in need, wherein the method comprises administering to the subject a therapeutically effective amount of the antibody or the antigen-binding fragment thereof according to any one of claims 1 to 8, the immunoconjugate or the multispecific molecule according to claim 11, or the pharmaceutical composition according to claim 12.

15. The method according to claim 14, wherein the IL-36/IL-36R-mediated related diseases and conditions include autoimmune diseases, inflammatory diseases, respiratory diseases, metabolic disorders, or cancer.

| | BI 655130 | xi72C1 | xi84C4 |
|---|---|---|---|
| EC50 | 7.611e-010 | 2.624e-009 | 9.698e-010 |

FIG. 1

| | BI 655130 | xi72C1 | xi84C4 |
|---|---|---|---|
| EC50 | 1.889e-009 | 3.021e-009 | 2.194e-008 |

FIG. 2

| | BI 655130 | xi72C1 | xi84C4 |
|---|---|---|---|
| EC50 | 1.326e-009 | 2.525e-009 | 8.864e-010 |

FIG. 3A

| | BI 655130 | xi72C1 | xi74B4D6 |
|---|---|---|---|
| EC50 | 7.023e-009 | 6.475e-009 | 1.552e-008 |

FIG. 3B

| | xi6G10 | xi58A8D1 | xi84C4 | xi72C1 | BI 655130 |
|---|---|---|---|---|---|
| EC50 | 3.491e-009 | 2.078e-008 | 3.658e-008 | 8.957e-009 | 9.638e-009 |

FIG. 4

| | xi72C1 | xi74B4D6 | BI655130 |
|---|---|---|---|
| IC50 | 7.996e-009 | 1.293e-008 | 3.620e-009 |

FIG. 5A

| | xi6G10 | xi58A8D1 | xi84C4 | BI 655130 |
|------|-----------|-----------|-----------|-----------|
| IC50 | 2.769e-009 | 1.635e-008 | 3.246e-009 | 6.090e-009 |

FIG. 5B

| | xi72C1 | xi74B4D6 | BI 655130 |
|------|-----------|-----------|-----------|
| IC50 | 7.511e-009 | 2.154e-008 | 5.022e-009 |

FIG. 5C

| | xi6G10 | xi58A8D1 | xi84C4 | BI 655130 |
|---|---|---|---|---|
| IC50 | 1.802e-009 | 9.771e-009 | 2.790e-009 | 2.452e-009 |

FIG. 5D

| | xi72C1 | xi74B4D6 | BI 655130 |
|---|---|---|---|
| IC50 | 1.047e-008 | 2.540e-008 | 4.737e-009 |

FIG. 5E

| | xi6G10 | xi58A8D1 | xi84C4 | BI 655130 |
|---|---|---|---|---|
| IC50 | ~ 4.022e-008 | ~ 4.082e-008 | ~ 1.324e-008 | ~ 1.318e-008 |

FIG. 5F

| | BI 655130 | xi72C1 |
|---|---|---|
| IC50 | 2.770e-008 | 3.323e-009 |

FIG. 6A

FIG. 6B

FIG. 6C

| | xi72C1 | xi74B4D6 | BI655130 |
|---|---|---|---|
| IC50 | 1.132e-010 | 7.300e-010 | 7.989e-011 |

FIG. 7A

| | BI 655130 | xi58A8D1 | xi84C4 | xi6G10 |
|---|---|---|---|---|
| IC50 | 9.267e-011 | 2.533e-010 | 1.811e-011 | 7.044e-011 |

FIG. 7B

| | xi72C1 | xi74B4D6 | BI655130 |
|---|---|---|---|
| IC50 | 1.156e-010 | 9.721e-010 | 7.549e-011 |

FIG. 7C

| | BI 655130 | xi58A8D1 | xi84C4 | xi6G10 |
|---|---|---|---|---|
| IC50 | 8.610e-011 | 4.152e-010 | 1.131e-010 | 1.824e-010 |

FIG. 7D

| | xi72C1 | xi74B4D6 | BI655130 |
|---|---|---|---|
| IC50 | 5.551e-011 | 1.049e-009 | 4.055e-011 |

FIG. 7E

| | BI 655130 | xi58A8D1 | xi84C4 | xi6G10 |
|---|---|---|---|---|
| IC50 | 5.924e-011 | 2.126e-010 | 1.811e-011 | 7.270e-011 |

FIG. 7F

| | BI655130 | xi6G10 | xi58A8D1 | xi84C4 |
|---|---|---|---|---|
| IC50 | 2.139e-010 | 5.553e-010 | 6.931e-011 | 1.016e-009 |

FIG. 8A

| | BI 655130 | xi72C1 |
|---|---|---|
| IC50 | 7.411e-011 | 1.592e-010 |

FIG. 8B

| | BI655130 | xi72C1 | xi6G10 | xi58A8D1 | xi84C4 |
|---|---|---|---|---|---|
| IC50 | 2.481e-010 | 4.745e-010 | 1.272e-009 | 7.906e-011 | 8.478e-010 |

FIG. 8C

| | BI655130 | xi72C1 | xi6G10 | xi58A8D1 | xi84C4 |
|---|---|---|---|---|---|
| IC50 | 3.868e-011 | 4.858e-011 | 1.296e-010 | 1.356e-011 | 1.246e-010 |

FIG. 8D

| | BI655130 | hz72C1-1.1 | hz72C1-1.2 | hz72C1-1.3 |
|---|---|---|---|---|
| EC50 | 7.638e-010 | 2.936e-009 | 5.021e-009 | 2.861e-009 |
| | | hz72C1-2.1 | hz72C1-2.2 | hz72C1-2.3 |
| EC50 | | 9.353e-009 | 4.878e-009 | 8.904e-009 |

FIG. 9A

| | BI655130 | hz84C4-1.1 | hz84C4-1.2 | hz84C4-1.3 | hz84C4-1.4 | hz84C4-1.5 |
|---|---|---|---|---|---|---|
| EC50 | 3.476e-010 | 5.093e-010 | 5.508e-010 | 3.408e-009 | 6.838e-010 | 6.243e-010 |
| | | hz84C4-2.1 | hz84C4-2.2 | hz84C4-2.3 | hz84C4-2.4 | hz84C4-2.5 |
| EC50 | | 5.577e-010 | 5.331e-010 | 2.246e-009 | 4.527e-010 | 4.966e-010 |

FIG. 9B

| | BI655130 | hz72C1-1.1 | hz72C1-1.2 | hz72C1-1.3 |
|---|---|---|---|---|
| EC50 | 1.966e-009 | 2.735e-009 | 4.264e-009 | 2.863e-009 |
| | | hz72C1-2.1 | hz72C1-2.2 | hz72C1-2.3 |
| EC50 | | 1.049e-008 | 4.866e-009 | 6.378e-009 |

FIG. 10

| | BI655130 | hz72C1-1.1 | hz72C1-1.2 | hz72C1-1.3 |
|---|---|---|---|---|
| EC50 | 5.133e-009 | 1.164e-008 | 1.190e-008 | 1.194e-008 |
| | | hz72C1-2.1 | hz72C1-2.2 | hz72C1-2.3 |
| EC50 | | 1.929e-008 | 1.759e-008 | 2.021e-008 |

FIG. 11A

| | EC50 |
|---|---|
| hz84C4-1.1 | ~ 5.282e-009 |
| hz84C4-1.2 | 6.001e-009 |
| hz84C4-1.3 | 1.821e-008 |
| hz84C4-1.4 | 5.750e-009 |
| hz84C4-1.5 | ~ 5.101e-009 |
| hz84C4-2.1 | ~ 5.223e-009 |
| hz84C4-2.2 | ~ 5.227e-009 |
| hz84C4-2.3 | 1.453e-008 |
| hz84C4-2.4 | ~ 5.258e-009 |
| hz84C4-2.5 | ~ 5.044e-009 |
| BI 655130 | 9.818e-009 |

FIG. 11B

| | BI655130 | hz72C1-1.1 | hz72C1-1.2 | hz72C1-1.3 |
|---|---|---|---|---|
| EC50 | 5.638e-009 | 5.206e-009 | ~4.936e-009 | 5.761e-009 |
| | | hz72C1-2.1 | hz72C1-2.2 | hz72C1-2.3 |
| EC50 | | 5.585e-009 | 7.245e-009 | 4.841e-009 |

FIG. 12A

| | EC50 |
|---|---|
| hz84C4-1.1 | 1.801e-008 |
| hz84C4-1.2 | 3.733e-008 |
| hz84C4-1.3 | 9.218e-009 |
| hz84C4-1.4 | 2.442e-008 |
| hz84C4-1.5 | 1.051e-008 |
| hz84C4-2.1 | 5.751e-009 |
| hz84C4-2.2 | 1.715e-008 |
| hz84C4-2.3 | 1.760e-007 |
| hz84C4-2.4 | 4.816e-008 |
| hz84C4-2.5 | 1.187e-008 |
| BI 655130 | 7.641e-009 |

FIG. 12B

FIG. 13A

FIG. 13B

FIG. 13C

FIG. 13D

FIG. 13E

FIG. 13F

FIG. 13G

FIG. 13H

FIG. 14A

FIG. 14B

FIG. 14C

FIG. 15A

FIG. 15B

FIG. 15C

FIG. 15D

FIG. 15E

FIG. 15F

FIG. 15G

FIG. 15H

FIG. 16A

FIG. 16B

FIG. 16C

FIG. 16D

FIG. 16E

FIG. 16F

FIG. 16G

FIG. 16H

<div align="center">INTERNATIONAL SEARCH REPORT</div>

| International application No. |
|---|
| **PCT/CN2022/099294** |

### A. CLASSIFICATION OF SUBJECT MATTER

C07K 16/24(2006.01)i; C07K 16/28(2006.01)i; G01N 33/564(2006.01)i; A61P 17/06(2006.01)i; A61P 37/06(2006.01)i

According to International Patent Classification (IPC) or to both national classification and IPC

### B. FIELDS SEARCHED

Minimum documentation searched (classification system followed by classification symbols)

C07K; G01N; A61P

Documentation searched other than minimum documentation to the extent that such documents are included in the fields searched

Electronic data base consulted during the international search (name of data base and, where practicable, search terms used)

CNTXT, CNABS, VEN, WOTXT, EPTXT, USTXT, PUBMED, BAIDU, ISI Web of knowledge, CNKI, GENBANK, EBI, UNIPROT: IL 36R, IL-36R, IL-1RL2, IL36R, CDR, 抗体, 单抗, antibody, 抗原结合片段, 免疫球蛋白, 抑制剂, 拮抗剂, vh, 重链可变区, vhh, 抑制, 结合蛋白, 结合分子, antibodies, 多抗, 配体, 自免, 自身免疫, 呼吸, 代谢, 癌, 肿, 瘤, tumor, cancer, SEQ ID NO: 1-90.

### C. DOCUMENTS CONSIDERED TO BE RELEVANT

| Category* | Citation of document, with indication, where appropriate, of the relevant passages | Relevant to claim No. |
|---|---|---|
| X | REDDY, S. T. et al. "Accession number: ADM43399" *GENBANK*, 25 July 2016 (2016-07-25), Origin | 1, 9 |
| X | REDDY, S. T. et al. "Accession number: ABC86106" *GENBANK*, 26 July 2016 (2016-07-26), Origin | 1, 9 |
| X | US 2013/0236471 A1 (BOEHRINGER INGELHEIM GMBH) 12 September 2013 (2013-09-12) abstract | 9 |
| A | US 2013/0236471 A1 (BOEHRINGER INGELHEIM GMBH) 12 September 2013 (2013-09-12) entire document | 1-8, 10-15 |
| X | CN 107362351 A (CHILDREN'S HOSPITAL OF SHANGHAI) 21 November 2017 (2017-11-21) abstract | 9 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 September 2022** | **15 September 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/099294** |

**C. DOCUMENTS CONSIDERED TO BE RELEVANT**

| Category* | Citation of document, with indication, where appropriate, of the relevant passages | Relevant to claim No. |
|---|---|---|
| A | CN 107362351 A (CHILDREN'S HOSPITAL OF SHANGHAI) 21 November 2017 (2017-11-21) entire document | 1-8, 10-15 |
| X | CN 110461877 A (BOEHRINGER INGELHEIM INTERNATIONAL GMBH) 15 November 2019 (2019-11-15) abstract | 9 |
| A | CN 110461877 A (BOEHRINGER INGELHEIM INTERNATIONAL GMBH) 15 November 2019 (2019-11-15) entire document | 1-8, 10-15 |
| X | US 2020/0017592 A1 (REGENERON PHARMACEUTICALS INC.) 16 January 2020 (2020-01-16) abstract | 9 |
| A | US 2020/0017592 A1 (REGENERON PHARMACEUTICALS INC.) 16 January 2020 (2020-01-16) entire document | 1-8, 10-15 |
| X | CONNER, K. P. et al. "Preclinical Characterization of the ADME Properties of a Surrogate Anti-IL-36R Monoclonal Antibody Antagonist in Mouse Serum and Tissues" *MAbs*, Vol. 12, No. 1, 31 December 2020 (2020-12-31), Article e1746520, pp. 1-16 | 9 |
| A | CONNER, K. P. et al. "Preclinical Characterization of the ADME Properties of a Surrogate Anti-IL-36R Monoclonal Antibody Antagonist in Mouse Serum and Tissues" *MAbs*, Vol. 12, No. 1, 31 December 2020 (2020-12-31), Article e1746520, pp. 1-16 | 1-8, 10-15 |

Form PCT/ISA/210 (second sheet) (January 2015)

**INTERNATIONAL SEARCH REPORT**

International application No.

**PCT/CN2022/099294**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  [✓] forming part of the international application as filed:

        [✓] in the form of an Annex C/ST.25 text file.

        [ ] on paper or in the form of an image file.

    b.  [ ] furnished together with the international application under PCT Rule 13*ter*.1(a) for the purposes of international search only in the form of an Annex C/ST.25 text file.

    c.  [ ] furnished subsequent to the international filing date for the purposes of international search only:

        [ ] in the form of an Annex C/ST.25 text file (Rule 13*ter*.1(a)).

        [ ] on paper or in the form of an image file (Rule 13*ter*.1(b) and Administrative Instructions, Section 713).

2.  [ ]  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

| International application No. |
| --- |
| **PCT/CN2022/099294** |

| Box No. II     Observations where certain claims were found unsearchable (Continuation of item 2 of first sheet) |
| --- |

This international search report has not been established in respect of certain claims under Article 17(2)(a) for the following reasons:

1. ☑   Claims Nos.: **14-15**
because they relate to subject matter not required to be searched by this Authority, namely:

    [1]   Claims 14-15 relate to a method for treating a disease. Therefore, said claims do not comply with PCT Rule 39.1(iv). The present search was conducted on the basis of a use for drug preparation.

2. ☐   Claims Nos.:
because they relate to parts of the international application that do not comply with the prescribed requirements to such an extent that no meaningful international search can be carried out, specifically:

3. ☐   Claims Nos.:
because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

| International application No. |
|---|
| **PCT/CN2022/099294** |

| Patent document cited in search report | | | Publication date (day/month/year) | Patent family member(s) | | | Publication date (day/month/year) |
|---|---|---|---|---|---|---|---|
| US | 2013/0236471 | A1 | 12 September 2013 | ES | 2755732 | T3 | 23 April 2020 |
| | | | | CA | 2852994 | A1 | 23 May 2013 |
| | | | | EP | 2780373 | A1 | 24 September 2014 |
| | | | | CN | 112812183 | A | 18 May 2021 |
| | | | | AP | 2014007571 | A0 | 30 April 2014 |
| | | | | CN | 107007833 | A | 04 August 2017 |
| | | | | AR | 089178 | A1 | 06 August 2014 |
| | | | | JP | 2020156506 | A | 01 October 2020 |
| | | | | SG | 11201402283 P | A | 27 June 2014 |
| | | | | US | 2015203584 | A1 | 23 July 2015 |
| | | | | AU | 2012339734 | A1 | 24 April 2014 |
| | | | | CN | 104080808 | A | 01 October 2014 |
| | | | | TW | 201333037 | A | 16 August 2013 |
| | | | | PT | 2780373 | T | 27 November 2019 |
| | | | | DK | 2780373 | T3 | 04 November 2019 |
| | | | | EA | 201400579 | A1 | 30 October 2014 |
| | | | | JP | 2018093885 | A | 21 June 2018 |
| | | | | NZ | 623425 | A | 24 December 2015 |
| | | | | PH | 12014501108 | B1 | 04 August 2014 |
| | | | | US | 2020231684 | A1 | 23 July 2020 |
| | | | | ZA | 201402491 | B | 29 September 2021 |
| | | | | IL | 232100 | D0 | 28 May 2014 |
| | | | | CL | 2014001123 | A1 | 23 January 2015 |
| | | | | BR | 112014011594 | A2 | 04 July 2017 |
| | | | | KR | 20140101738 | A | 20 August 2014 |
| | | | | RS | 59248 | B1 | 31 October 2019 |
| | | | | UA | 115540 | C2 | 27 November 2017 |
| | | | | KR | 20200110476 | A | 23 September 2020 |
| | | | | EC | SP14004976 | A | 30 November 2015 |
| | | | | PE | 20142041 | A1 | 18 December 2014 |
| | | | | SI | 2780373 | T1 | 31 December 2019 |
| | | | | HR | P20191835 | T1 | 27 December 2019 |
| | | | | JP | 2015500633 | A | 08 January 2015 |
| | | | | LT | 2780373 | T | 25 September 2019 |
| | | | | WO | 2013074569 | A1 | 23 May 2013 |
| | | | | MX | 2014005759 | A | 08 September 2014 |
| | | | | CO | 7020871 | A2 | 11 August 2014 |
| | | | | EP | 3536710 | A1 | 11 September 2019 |
| | | | | UY | 34456 | A | 31 May 2013 |
| | | | | HU | E047437 | T2 | 28 April 2020 |
| | | | | PL | 2780373 | T3 | 31 March 2020 |
| | | | | IL | 272035 | A | 27 February 2020 |
| | | | | KR | 20190122877 | A | 30 October 2019 |
| US | 2013/0236471 | A1 | 12 September 2013 | ES | 2755732 | T3 | 23 April 2020 |
| | | | | CA | 2852994 | A1 | 23 May 2013 |
| | | | | EP | 2780373 | A1 | 24 September 2014 |
| | | | | CN | 112812183 | A | 18 May 2021 |
| | | | | AP | 2014007571 | A0 | 30 April 2014 |
| | | | | CN | 107007833 | A | 04 August 2017 |
| | | | | AR | 089178 | A1 | 06 August 2014 |

Form PCT/ISA/210 (patent family annex) (January 2015)

# EP 4 357 361 A1

**INTERNATIONAL SEARCH REPORT**
Information on patent family members

International application No.

**PCT/CN2022/099294**

| Patent document cited in search report | | | Publication date (day/month/year) | Patent family member(s) | | | Publication date (day/month/year) |
|---|---|---|---|---|---|---|---|
| | | | | JP | 2020156506 | A | 01 October 2020 |
| | | | | SG | 11201402283 P | A | 27 June 2014 |
| | | | | US | 2015203584 | A1 | 23 July 2015 |
| | | | | AU | 2012339734 | A1 | 24 April 2014 |
| | | | | CN | 104080808 | A | 01 October 2014 |
| | | | | TW | 201333037 | A | 16 August 2013 |
| | | | | PT | 2780373 | T | 27 November 2019 |
| | | | | DK | 2780373 | T3 | 04 November 2019 |
| | | | | EA | 201400579 | A1 | 30 October 2014 |
| | | | | JP | 2018093885 | A | 21 June 2018 |
| | | | | NZ | 623425 | A | 24 December 2015 |
| | | | | PH | 12014501108 | B1 | 04 August 2014 |
| | | | | US | 2020231684 | A1 | 23 July 2020 |
| | | | | ZA | 201402491 | B | 29 September 2021 |
| | | | | IL | 232100 | D0 | 28 May 2014 |
| | | | | CL | 2014001123 | A1 | 23 January 2015 |
| | | | | BR | 112014011594 | A2 | 04 July 2017 |
| | | | | KR | 20140101738 | A | 20 August 2014 |
| | | | | RS | 59248 | B1 | 31 October 2019 |
| | | | | UA | 115540 | C2 | 27 November 2017 |
| | | | | KR | 20200110476 | A | 23 September 2020 |
| | | | | EC | SP14004976 | A | 30 November 2015 |
| | | | | PE | 20142041 | A1 | 18 December 2014 |
| | | | | SI | 2780373 | T1 | 31 December 2019 |
| | | | | HR | P20191835 | T1 | 27 December 2019 |
| | | | | JP | 2015500633 | A | 08 January 2015 |
| | | | | LT | 2780373 | T | 25 September 2019 |
| | | | | WO | 2013074569 | A1 | 23 May 2013 |
| | | | | MX | 2014005759 | A | 08 September 2014 |
| | | | | CO | 7020871 | A2 | 11 August 2014 |
| | | | | EP | 3536710 | A1 | 11 September 2019 |
| | | | | UY | 34456 | A | 31 May 2013 |
| | | | | HU | E047437 | T2 | 28 April 2020 |
| | | | | PL | 2780373 | T3 | 31 March 2020 |
| | | | | IL | 272035 | A | 27 February 2020 |
| | | | | KR | 20190122877 | A | 30 October 2019 |
| CN | 107362351 | A | 21 November 2017 | None | | | |
| CN | 110461877 | A | 15 November 2019 | JP | 2020512344 | A | 23 April 2020 |
| | | | | US | 2018273627 | A1 | 27 September 2018 |
| | | | | EP | 3601350 | A1 | 05 February 2020 |
| | | | | WO | 2018183173 | A1 | 04 October 2018 |
| US | 2020/0017592 | A1 | 16 January 2020 | AU | 2019306217 | A1 | 14 January 2021 |
| | | | | SG | 11202012360 Y | A | 28 January 2021 |
| | | | | EP | 3823989 | A2 | 26 May 2021 |
| | | | | PH | 12020552178 | A1 | 28 June 2021 |
| | | | | CN | 112513091 | A | 16 March 2021 |
| | | | | JP | 2021530229 | A | 11 November 2021 |
| | | | | BR | 112020027015 | A2 | 06 April 2021 |
| | | | | KR | 20210032401 | A | 24 March 2021 |
| | | | | CA | 3103531 | A1 | 23 January 2020 |

Form PCT/ISA/210 (patent family annex) (January 2015)

**INTERNATIONAL SEARCH REPORT**
Information on patent family members

International application No.

**PCT/CN2022/099294**

| Patent document cited in search report | | | Publication date (day/month/year) | Patent family member(s) | | | Publication date (day/month/year) |
|---|---|---|---|---|---|---|---|
| | | | | WO | 2020018503 | A2 | 23 January 2020 |
| | | | | IL | 280013 | A | 01 March 2021 |
| US | 2020/0017592 | A1 | 16 January 2020 | AU | 2019306217 | A1 | 14 January 2021 |
| | | | | SG | 11202012360 Y | A | 28 January 2021 |
| | | | | EP | 3823989 | A2 | 26 May 2021 |
| | | | | PH | 12020552178 | A1 | 28 June 2021 |
| | | | | CN | 112513091 | A | 16 March 2021 |
| | | | | JP | 2021530229 | A | 11 November 2021 |
| | | | | BR | 112020027015 | A2 | 06 April 2021 |
| | | | | KR | 20210032401 | A | 24 March 2021 |
| | | | | CA | 3103531 | A1 | 23 January 2020 |
| | | | | WO | 2020018503 | A2 | 23 January 2020 |
| | | | | IL | 280013 | A | 01 March 2021 |

Form PCT/ISA/210 (patent family annex) (January 2015)

**EP 4 357 361 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110675925
- US 5225539 A
- US 5530101 A
- US 5585089 A
- US 5693762 A
- US 6180370 B
- US 20030153043 A
- US 5677425 A
- US 6165745 A
- US 5714350 A
- US 6350861 B
- EP 0154316 A
- EP 0401384 A
- US 4816567 A
- WO 8704462 A
- WO 8901036 A
- EP 338841 A

**Non-patent literature cited in the description**

- **WANG et al.** *Int J Med Sci,* 2017, vol. 14, 1002-1007
- **TORTOLA et al.** *J Clin Invest,* 2012, vol. 122, 3965-3976
- **NISHIDA et al.** *Inflamm Bowel Dis,* 2016, vol. 22, 303-314
- **BACHELEZ HERVÉ et al.** *NEJM,* 2019, vol. 380, 981-983
- **WARD et al.** *Nature,* 1989, vol. 341, 544-546
- **BIRD et al.** *Science,* 1988, vol. 242, 423-426
- **HUSTON et al.** *Proc. Natl. Acad. Sci.,* 1988, vol. 85, 5879-5883
- **KABAT et al.** Sequences of Proteins of Immunological Interest. National Institutes of Health, 1991
- *Jmol Biol,* 1997, vol. 273, 927-48
- **LEFRANC M.P.** *Immunologist,* 1999, vol. 7, 132-136
- **LEFRANC, M.P. et al.** *Dev. Comp. Immunol.,* 2003, vol. 27, 55-77
- **RIECHMANN et al.** *Nature,* 1998, vol. 332, 323-327
- **JONES et al.** *Nature,* 1986, vol. 321, 522-525
- **QUEEN et al.** *Proc. Natl. Acad. Also see U.S.A.,* 1989, vol. 86, 10029-10033
- **TOMLINSON et al.** *J. Mol. Biol.,* 1992, vol. 227, 776-798
- **COX et al.** *Eur. J. Immunol.,* 1994, vol. 24, 827-836
- **HUSTON et al.** *Proc. Natl. Acad. Sci. USA,* 1988, vol. 85, 5879-5883
- **MCCAFFERTY et al.** *Nature,* 1990, vol. 348, 552-554
- **KOHLER ; MILSTEIN.** *Nature,* 1975, vol. 256, 495
- **MORRISON, S.** *Science,* 1985, vol. 229, 1202
- **GOEDDEL.** Gene Expression Technology. Methods in Enzymology. Academic Press, 1990, 185
- **TAKEBE et al.** *Mol. Cell. Biol.,* 1988, vol. 8, 466-472
- **URLAUB ; CHASIN.** *Proc. Natl. Acad. Sci. USA,* 1980, vol. 77, 4216-4220
- **R. J. KAUFMAN ; P. A. SHARP.** *J. Mol. Biol.,* 1982, vol. 159, 601-621